# EUROPEAN PATENT APPLICATION

(11) **EP 4 617 273 A1**
(43) Date of publication of application: **17.09.2025**
(21) Application number: 23888101.5
(22) Date of filing: 10.11.2023
(51) Int. Cl.: C07D 487/04, C07D 401/14, C07D 471/04, A61K 31/519, A61K 31/444, A61P 35/00

(54) **FUSED TRICYCLIC PARP1 INHIBITOR, PREPARATION METHOD THEREFOR, AND USE THEREOF**

(30) Priority: 10.11.2022 CN 202211409077; 18.01.2023 CN 202310057447; 28.06.2023 CN 202310771365
(71) Applicant: Haihe Biopharma Co., Ltd., Shanghai 201203 (CN)
(72) Inventor: GAO, Shanyun, Shanghai 201203 (CN); HOU, Yingjie, Shanghai 201203 (CN); LI, Jingjing, Shanghai 201203 (CN); ZHANG, Chaobo, Shanghai 201203 (CN); XU, Yanxiao, Shanghai 201203 (CN); TU, Wangyang, Shanghai 201203 (CN); YU, Bing, Shanghai 201203 (CN); ZHANG, Yixiang, Shanghai 201203 (CN); LI, Leping, Shanghai 201203 (CN)
(74) Representative: Petraz, Gilberto Luigi
(86) International application number: PCT/CN2023/130888
(87) International publication number: WO 2024/099416

(57) **Abstract**

The present invention relates to a fused tricyclic PARP1 inhibitor, a preparation method therefor, and a use thereof. In particular, the present invention relates to a compound capable of inhibiting poly ADP-ribose polymerase activity, and a pharmaceutical composition and use thereof. The present invention particularly relates to compounds of formula (I), and also to pharmaceutical compositions comprising these compounds and to the use of these compounds in the preparation of a medicament for the preventing and/or treatment of a disease, in particular, disease that can be ameliorated by inhibiting PARP1.

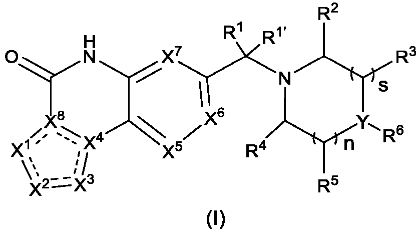

## Description

### TECHNICAL FIELD

The present invention involves a class of compounds having poly ADP-ribose polymerase 1 (PARP1) inhibitory activity and uses thereof, and specifically to a class of quinolone compounds, pharmaceutical compositions comprising the compounds, and uses in drugs for diseases, particularly tumor diseases, ameliorated by PARP1 inhibition.

### BACKGROUND

Poly (ADP-ribose) polymerases (PARPs) are an emerging family of enzymes that catalyze the transfer of ADP-ribose to target proteins (poly ADP-ribosylation). At least 18 PARP family members are encoded by different genes and share homology in the conserved catalytic domain (Morales et al, Critical Reviews™ in Eukaryotic Gene Expression 24.1,2014). PARP1 as an abundant nuclear protein can catalyze the transfer of ADP-ribose residues from NAD+ to target substrate proteins or nucleic acids, constructing a poly (ADP-ribose) (PAR) chain to be added to the downstream target proteins, a post-translational modification known as PARylation (Murai et al, Cancer research 72, 21,2012). PARPs are important in several cellular processes including cell proliferation and cell death. The primary function of PARPs is to participate in DNA damage repair, with DNA single-strand breaks (SSBs) being the most common type of damage, which can be converted into potentially cleavage-causing and lethal DNA double-strand breaks (DSBs). PARP1 binds to damaged DNA at single-stranded DNA breaks (SSBs) and other DNA damages, an event that leads to a series of conformational changes in the structure of PARP1, which activates its catalytic function (Lord et al, Science 355, 6330, 2017). BRCA1 and BRCA2 proteins are essential for the repair of double-stranded DNA breaks (DSBs) by a process known as homologous recombination repair (HRR), a form of DNA repair that utilizes homologous DNA sequences to direct repair at the DSB. HRR is usually a 'conserved' mechanism because it restores the original DNA sequence at the site of DNA damage. Non-conserved forms of DNA repair, such as non-homologous end joining (NHEJ), predominate when cells are HRR-deficient, whether driven by defects in BRCA1, BRCA2 or other pathway components.

PARP inhibitors play an anticancer role by blocking DNA damage repair in highly mutated cancer cells, resulting in "toxic damage" that causes cell death in cells lacking homologous recombination repair (HRR). Multiple signaling pathways for DNA repair exist in healthy cells, so inhibiting PARP alone will not be too toxic to them. However, for some tumor cells, the DNA repair pathway will be PARP1 dependent and therefore will be particularly sensitive to PARP inhibitors, as mutations in specific genes such as BRCA disrupt other DNA repair pathways. This is why ovarian and breast cancer patients carrying BRCA mutations are more likely to benefit from PARP inhibitors. PARP2 has a low intracellular content, accounting for only 5% ~ 10% of total PARP activity. Knockdown of PARP1, compared to knockdown of PARP2 (<10%) significantly reduced PARP activity (Yélamos et al, The EMBO journal 25.18, 2006). Knockdown of PARP1 blocked the PARP inhibitory activity of Olaparib and also eliminated the cell proliferation inhibitory effects of Olaparib (Murai et al, Cancer research, 2012). These data suggest that the key determinant of the antitumor effect of PARP inhibitors is the inhibition of PARP1. It has been reported that mice require intact PARP2 in the bone marrow for survival. PARP2 deficiency results in reduced numbers of RBCs, WBCs and BM cells (Farrés et al, Blood, The Journal of the American Society of Hematology 122, 1, 2013). Knockdown of PARP2 reduced the number of T cells and RBCs, whereas knockdown of PARP1 did not significantly affect the number of T cells and RBCs (Yélamos et al, Blood, The EMBO journal 25, 18, 2006; Farrés et al, Cell Death & Differentiation 22, 7, 2015). Thus, PARP1 inhibition is the main cause of efficacy and PARP2 inhibition is the main cause of toxicity. The development of highly selective PARP1 inhibitors may significantly reduce the toxicity produced by PARP2 without significantly sacrificing the efficacy.

In summary, there is an urgent need in this field to develop PARP inhibitors with high efficacy and safety, especially inhibitors with high selectivity for PARP1.

### SUMMARY OF THE INVENTION

The present invention provides a compound of formula (I), or a stereoisomer, a geometric isomer, a tautomer, a pharmaceutically acceptable salt, a crystal form, a solvate, a hydrate or a prodrug thereof, 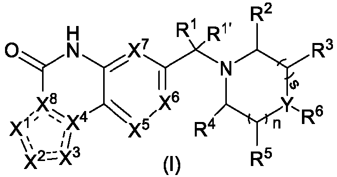 wherein,
X¹ is each independently selected from -N-, -NR¹⁴-, -CR⁷-, -CR⁷R^{7'}, -CH₂CR⁷R^{7'}-, - CR⁷R^{7'}-CH₂-, O and S;
X² is each independently selected from -N-, -NR¹⁵-, -CR⁸-, -CR⁸R^{8'}-, O and S;
X³ is each independently selected from -N-, -NR¹⁶-, -CR⁹-, O and S;
X⁴ and X⁸ are each independently selected from -N- and -C-;
-̅ -̅ -̅ -̅ -̅ -̅ is a single or double bond; and X¹, X², X³, X⁴ together with X⁸ form five-membered heteroaryl, or partially saturated five-membered or six-membered heterocyclyl; wherein the heteroaryl or the heterocyclyl each independently contains 1, 2 or 3 heteroatoms independently selected from N, O and S;
R⁷, R^{7'}, R⁸, R^{8'}, R⁹ are each independently selected from hydrogen, halogen, hydroxyl, cyano, C₁-C₃ alkoxy, unsubstituted or substituted C₃-C₆ cycloalkyl and unsubstituted or substituted C₁-C₆ alkyl; or, R⁷ together with R^{7'}, or R⁸ together with R^{8'}, form C₃-C₆ cycloalkyl; R⁷, R^{7'}, R⁸, R^{8'}, R⁹ are each independently preferably hydrogen, halogen or C₁-C₄ alkyl; R⁷, R^{7'}, R⁸, R^{8'}, R⁹ are each independently more preferably hydrogen, F or methyl; or preferably, R⁷ together with R^{7'}, or R⁸ together with R^{8'}, form C₃-C₄ cycloalkyl, such as cyclopropyl or cyclopentyl;
R¹⁴, R¹⁵, R¹⁶ are each independently selected from hydrogen, unsubstituted or substituted C₃-C₆ cycloalkyl, unsubstituted or substituted C₁-C₆ alkyl; R¹⁴, R¹⁵, R¹⁶ are each independently preferably hydrogen or C₁-C₃ alkyl; R¹⁴, R¹⁵, R¹⁶ are each independently more preferably methyl;
X⁵, X⁶ are each independently selected from -N- and -CR¹⁰-; R¹⁰ is selected from hydrogen, halogen, cyano, unsubstituted or substituted C₁-C₆ alkoxy, unsubstituted or substituted C₁-C₆ alkyl; R¹⁰ is preferably hydrogen, halogen, cyano or C₁-C₄ alkyl; R¹⁰ is more preferably hydrogen, fluorine, chlorine or methyl;
X⁷ is -N- or -CR¹⁷-; R¹⁷ is selected from hydrogen, halogen, cyano, unsubstituted or substituted C₁-C₆ alkoxy, unsubstituted or substituted C₁-C₆ alkyl; R¹⁷ is preferably hydrogen, halogen, cyano or C₁-C₄ alkyl; R¹⁷ is more preferably hydrogen, fluorine, chlorine or methyl;
R¹, R^{1'}, R², R³, R⁴, R⁵ are each independently selected from hydrogen, unsubstituted or substituted C₁-C₆ alkyl; or R⁴, R⁵ together with the carbon atoms bound thereto form C₃-C₆ cycloalkyl;
s, n are independently selected from 0, 1 and 2;
Y is N or CH;
R⁶ is selected from: 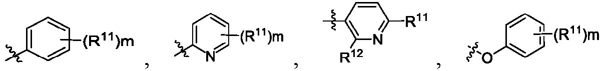 and 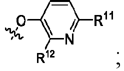
each R¹¹ is independently selected from halogen, cyano, C₁-C₃ alkoxy, carbonyl, - CONHR¹³, amino, preferably selected from halogen, -CONHR¹³ and cyano; more preferably selected from -CONHR¹³;
m is 0, 1, 2 or 3;
R¹² is selected from hydrogen, halogen, cyano, unsubstituted or substituted C₁-C₄ alkyl;
R¹³ is hydrogen, unsubstituted or substituted C₁-C₆ alkyl, unsubstituted or substituted C₃-C₈ cycloalkyl, unsubstituted or substituted C₁-C₆ alkoxy, or unsubstituted or substituted 3-8 membered heterocycloalkyl; preferably, R¹³ is hydrogen, unsubstituted or halogen substituted C₁-C₄ alkyl, C₃-C₆ cycloalkyl or C₁-C₆ alkoxy; the heterocycloalkyl refers to heterocycloalkyl containing 1-3 heteroatoms selected from N, O, and S; preferably, R¹³ is methyl, ethyl, C₂-C₃ alkoxy, cyclopropyl, epoxypropyl, oxetylbutyl, or oxetylpentyl;
wherein, the substitution in R¹, R^{1'}, R², R³, R⁴, R⁵, R⁷, R^{7'}, R⁸, R^{8'}, R⁹, R¹⁰, R¹², R¹³, R¹⁴, R¹⁵, R¹⁶, and R¹⁷ refers to being substituted by one or more selected from C₁-C₄ alkyl, halogen, hydroxyl, cyano, amino, carboxyl, and C₃-C₆ cycloalkyl;
provided that:
   when X⁵ and X⁸ are both -N-, at least one of X¹, X², X³, and X⁴ is -N-;
   when X⁴ and X⁵ are both -N-, X³ is also -N-;
   when X³ is oxygen and X¹, X², X⁴, X⁸ are -C- and X⁷ is -CR¹⁷- , R¹, R^{1'}, and R¹⁷ are not hydrogen at the same time;
   preferably, provided that at least one of X¹, X², X³ is -N- when X⁸ is -N-.
   preferably, provided that X⁵, X⁶ are each independently selected from -CR¹⁰- and X⁷ is - CR¹⁷-.
   more preferably, provided that at least one of X¹, X², X³ is -N- when X⁸is -N-;
   when X⁴ and X⁵ are both -N-, X³ is also -N-;
   when X³ is oxygen and X¹, X², X⁴, X⁸ are -C- and X⁷ is -CR¹⁷-, R¹, R^{1'}, and R¹⁷ are not hydrogen at the same time.

In a preferred embodiment of the present invention, a compound as shown in formula (II), or a stereoisomer, a geometric isomer, a tautomer, a pharmaceutically acceptable salt, a crystal form, a solvate, a hydrate, or a prodrug thereof, is provided. 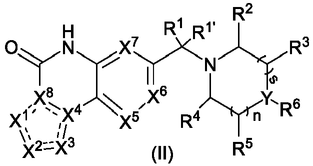 wherein,
X¹ is each independently selected from -N-, -NR¹⁴-, -CR⁷-, O and S;
X² is each independently selected from -N-, -NR¹⁵-, -CR⁸-, O and S;
X³ is each independently selected from -N-, -NR¹⁶-, -CR⁹-, O and S;
X⁴ and X⁸ are each independently selected from -N- and -C-;
-̅ -̅ -̅ -̅ -̅ -̅ is a single or double bond; and X¹, X², X³, X⁴ together with X⁸ form five-membered heteroaryl or a partially saturated five-membered heterocyclyl;
R⁷, R⁸, R⁹ are each independently selected from hydrogen, halogen, unsubstituted or substituted C₃-C₆ cycloalkyl or unsubstituted or substituted C₁-C₆ alkyl; the substitution in R⁷, R⁸, R⁹ refers to being substituted by one or more of a group selected from C₁-C₄ alkyl, halogen, hydroxy, cyano, amino, carboxy, C₃-C₆ cycloalkyl; R⁷, R⁸, R⁹ are preferably hydrogen, halogen or C₁-C₄ alkyl; R⁷, R⁸, R⁹ are more preferably hydrogen, F or methyl;
X⁵, X⁶, X⁷, R¹, R^{1'}, R², R³, R⁴, R⁵, R⁶, R¹⁰, R¹¹, R¹², R¹³, R¹⁴, R¹⁵, R¹⁶, R¹⁷, s, n, m and Y are as described in formula (I).

In a preferred embodiment of the present invention, the compounds shown in formulae (I), (II), X¹, X², X³, X⁴ together with X⁸ formpartially saturated five-membered heterocyclyl or partially saturated six-membered heterocyclyl. Preferably, when X⁴ is C, X³ is N or -NR¹⁶. More preferably, when X⁴ is C, X³ is N.

In a preferred embodiment of the present invention, X¹, X², X³, X⁴ together with X⁸ form five-membered heteroaryl in the compounds shown in formulae (I), (II).

In a preferred embodiment of the present invention, X¹, X², X³, X⁴ together with X⁸ form partially saturated five-membered heterocyclyl in the compounds shown in formula (I), (II).

In a preferred embodiment of the present invention, X¹, X², X³, X⁴ together with X⁸ form partially saturated six-membered heterocyclyl in the compounds shown in formula (I), (II).

In a preferred embodiment of the present invention, in the compounds shown in formula (I), (II), at least one of X¹, X², X³, X⁴, X⁸ is N, and X¹, X², X³, X⁴ together with X⁸ form partially saturated five-membered or six-membered heterocyclyl.

In a preferred embodiment of the present invention, in the compounds shown in formula (I), (II),
X¹ is independently selected from -N-, -NR¹⁴-, -CR⁷-, -CR⁷R^{7'}, -CH₂CR⁷R^{7'}- and - CR⁷R^{7'}-CH₂-;
X² is independently selected from -N-, -NR¹⁵-, -CR⁸- and -CR⁸R^{8'}-;
X⁴ and X⁸ are each independently selected from -N- and -C-;
X³ is independently selected from -N-, -NR¹⁶- and -CR⁹-; and
at least one of X¹, X², X³, X⁴, X⁸ is N, and X¹, X², X³, X⁴ together with X⁸ form partially saturated five-membered or six-membered heterocyclyl.

In a preferred embodiment of the present invention, in the compounds shown in formula (I), (II),
X¹ is independently selected from -CR⁷-, -CR⁷R^{7'}, -CH₂CR⁷R⁷'- and -CR⁷R^{7'}-CH₂-;
X² is independently selected from -CR⁸- and -CR⁸R^{8'}-;
X⁴ is -C-;
X⁸ is -N-;
X³ is selected from -N- and -NR¹⁶-;
and X¹, X², X³, X⁴ together with X⁸ form partially saturated five-membered or six-membered heterocyclyl.

In the above embodiments, the partially saturated five-membered or six-membered heterocyclyl refers to unsaturated and non-aromatic heterocyclyl.

Preferably, 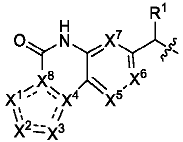 in formula (I) or (II) is selected from the following structures: 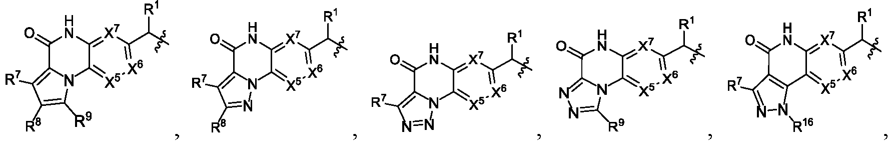 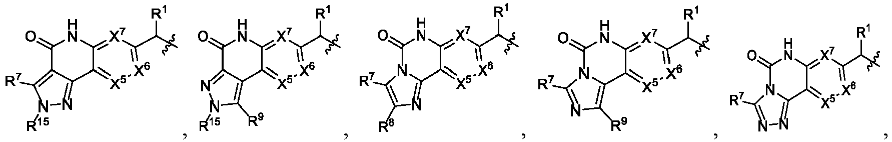 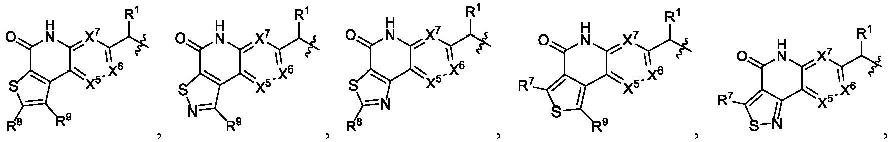 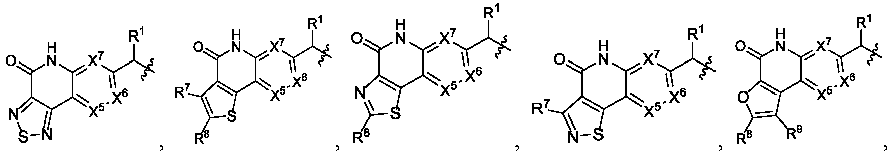 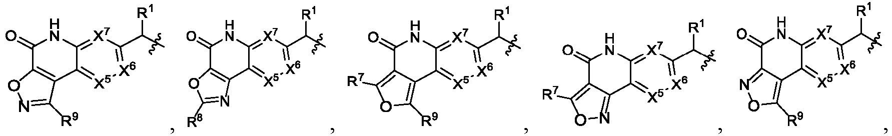 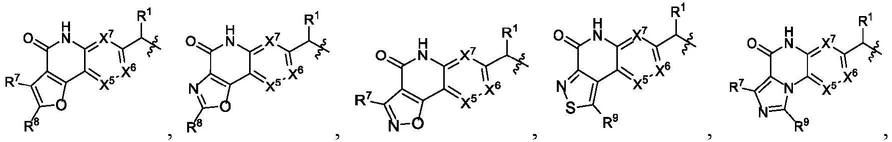 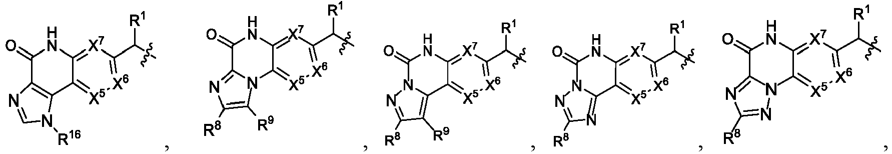 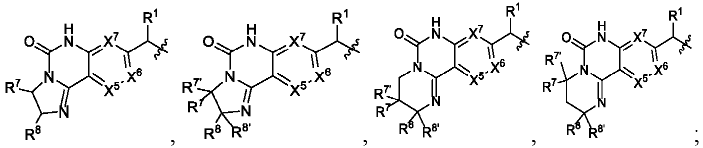 more preferably selected from the following structure:

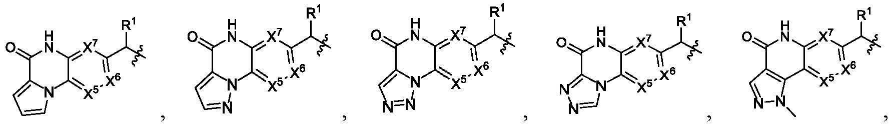

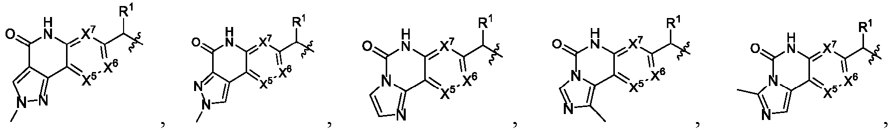

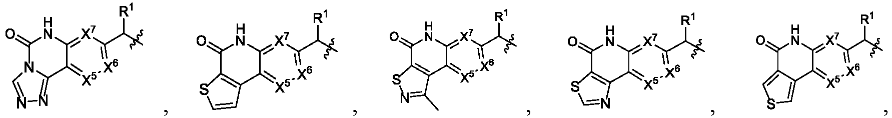

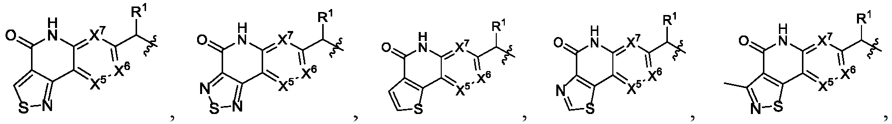

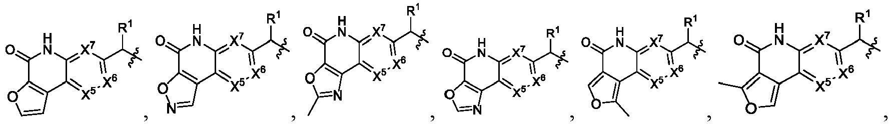

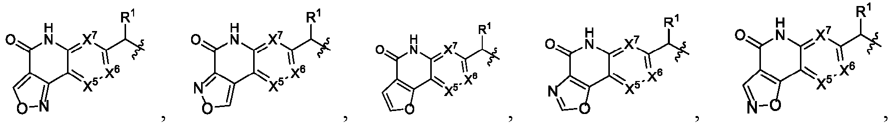

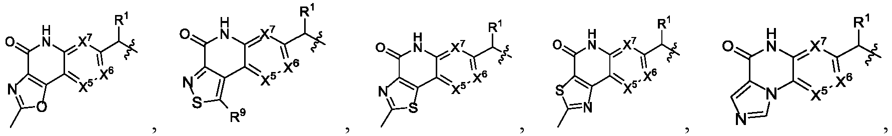

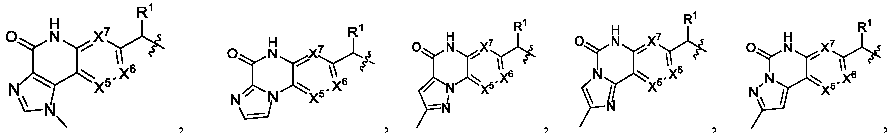

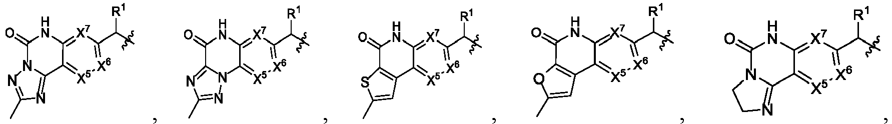

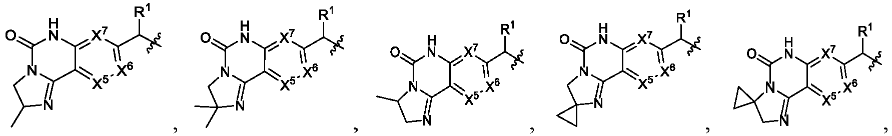

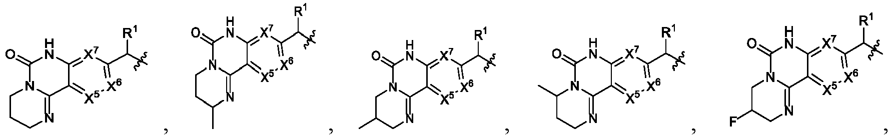

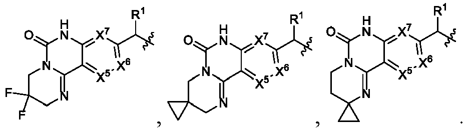

Wherein, 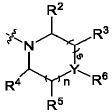 in formula (I) or (II) is preferably selected from the following structures: 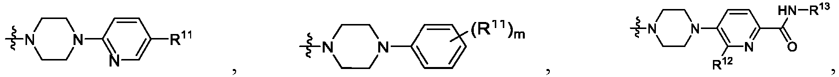 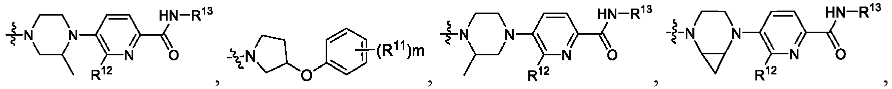 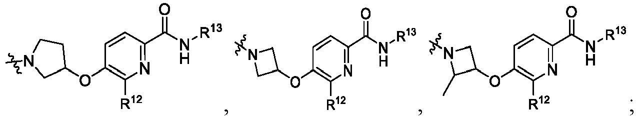 more preferably, selected from the following structures: 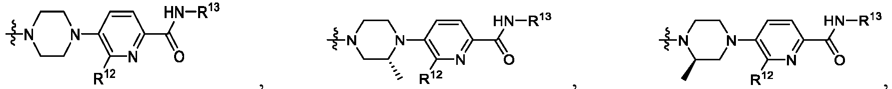 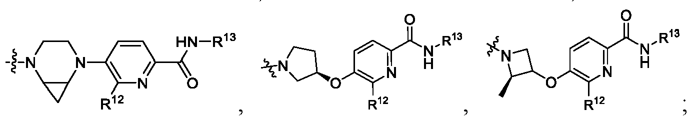 further preferably, selected from the following structures: 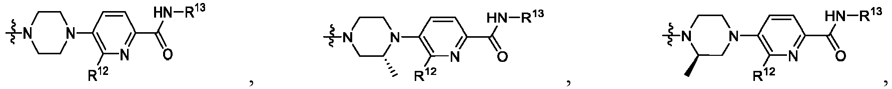 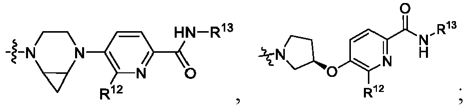 wherein, R², R³, R⁴, R⁵, R¹¹, R¹², R¹³ and m are defined as previously described.

More preferably, the compound described in formula (I) is selected from the following specific compounds:

| No. | Structure | No. | Structure |
|---|---|---|---|
| 5 | 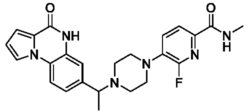 | 4 | 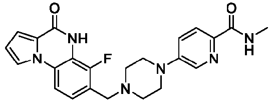 |
| 7 | 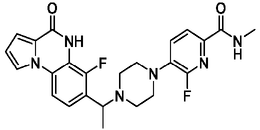 | 6 | 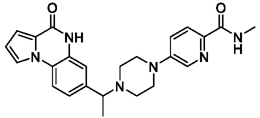 |
| 9 | 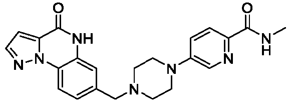 | 8 | 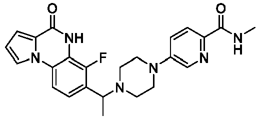 |
| 13 | 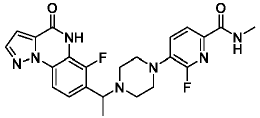 | 12 | 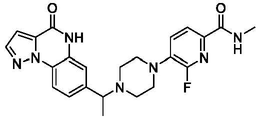 |
| 15 | 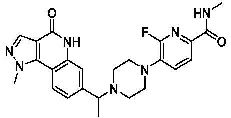 | 16 | 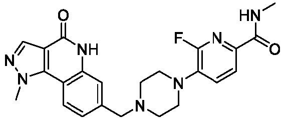 |
| 17 | 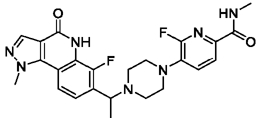 | 19 | 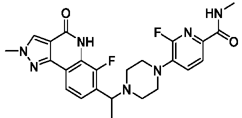 |
| 21 | 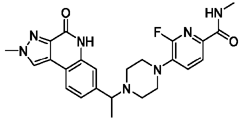 | 22 | 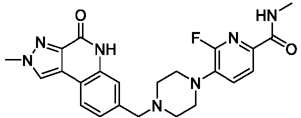 |
| 26 | 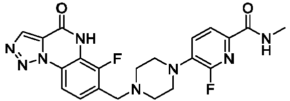 | 34 | 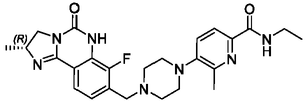 |
| 75 | 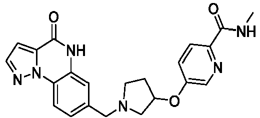 | 77 | 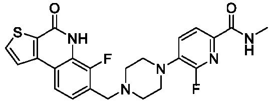 |
| 80 | 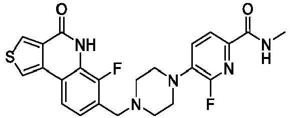 | 98 | 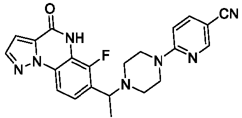 |
| 99 | 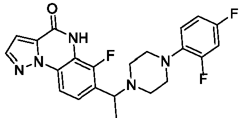 | 102 | 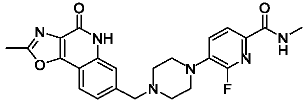 |
| 101 | 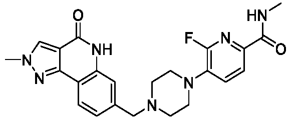 | 106 | 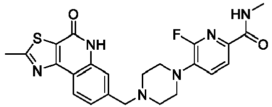 |
| 103 | 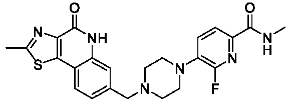 | 112 | 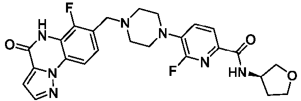 |
| 113 | 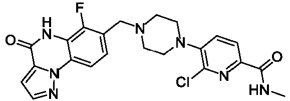 | 117 | 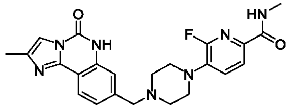 |
| 123 | 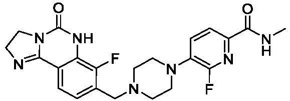 | 124 | 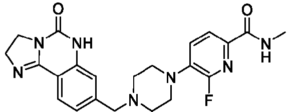 |
| 125 | 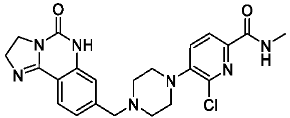 | 126 | 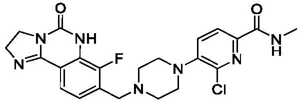 |
| 127 | 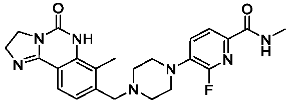 | 128 | 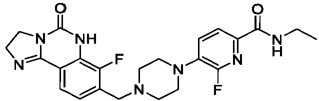 |
| 129 | 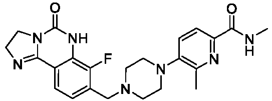 | 130 | 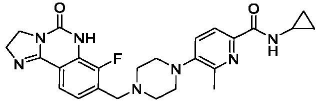 |
| 131 | 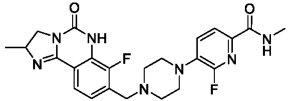 | 132 | 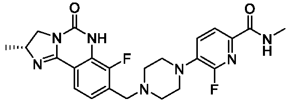 |
| 133 | 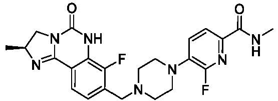 | 134 | 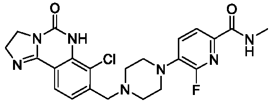 |
| 135 | 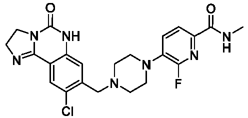 | 136 | 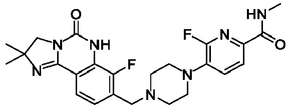 |
| 137 | 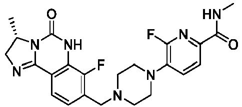 | 138 | 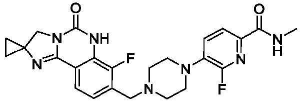 |
| 139 | 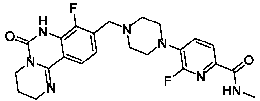 | 140 | 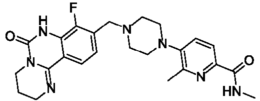 |
| 141 | 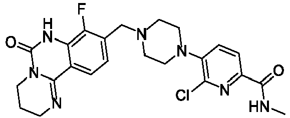 | 142 | 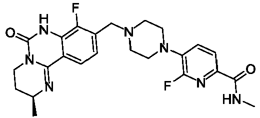 |
| 143 | 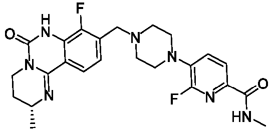 | 144 | 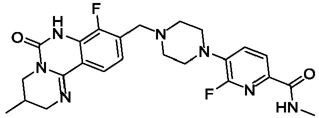 |
| 145 | 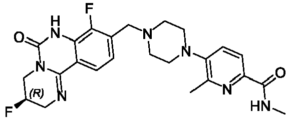 | 146 | 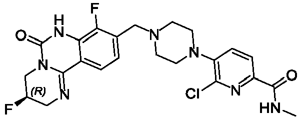 |
| 147 | 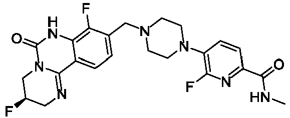 | 148 | 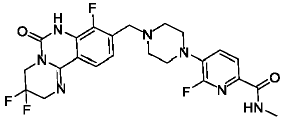 |
| 149 | 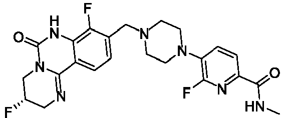 | 150 | 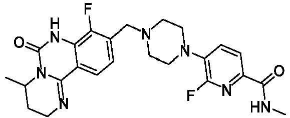 |
| 151 | 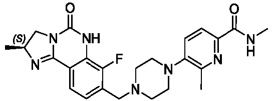 | 152 | 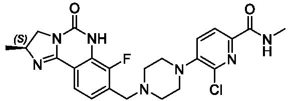 |
| 153 | 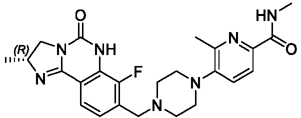 | 154 | 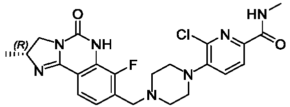 |
| 155 | 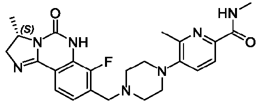 | 156 | 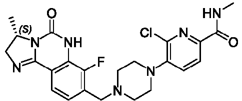 |
| 157 | 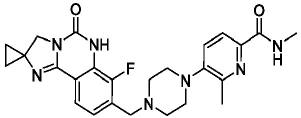 | 158 | 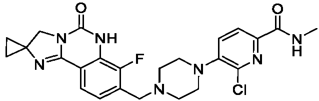 |
| 159 | 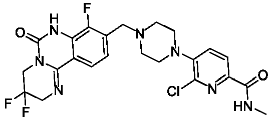 | 160 | 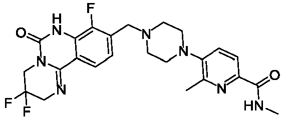 |
| 161 | 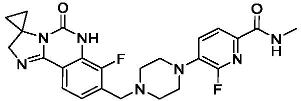 | 162 | 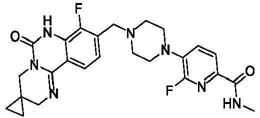 |
| 163 | 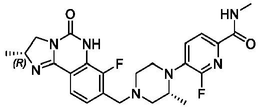 | 164 | 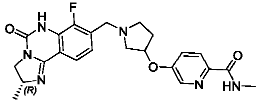 |
| 165 | 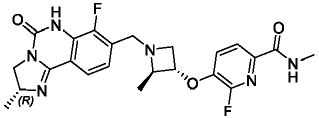 | 166 | 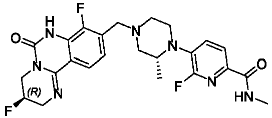 |
| 167 | 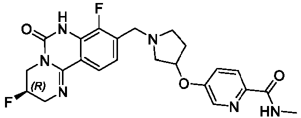 | 168 | 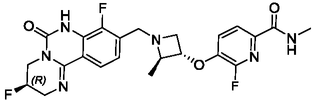 |
| 169 | 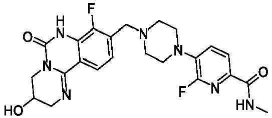 | 170 | 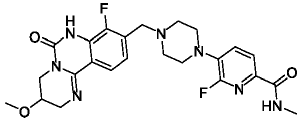 |
| 171 | 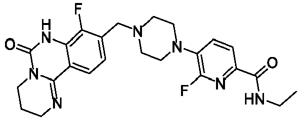 | 172 | 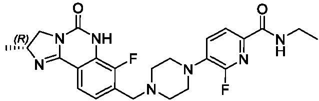 |
| 173 | 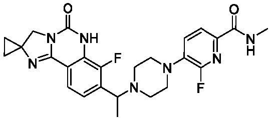 | 174 | 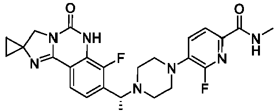 |
| 175 | 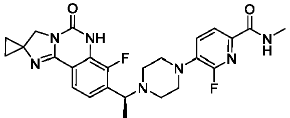 | 176 | 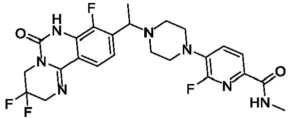 |
| 177 | 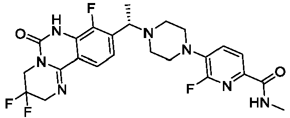 | 178 | 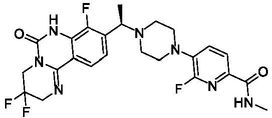 |
| 179 | 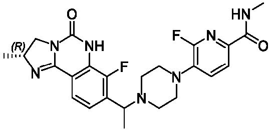 | 180 | 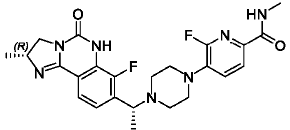 |
| 181 | 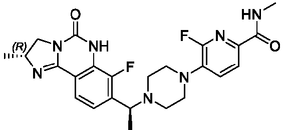 | 182 | 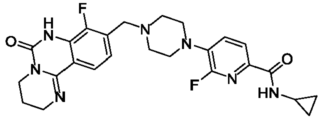 |
| 183 | 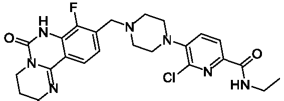 | 184 | 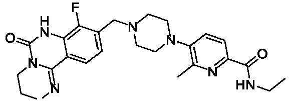 |
| 185 | 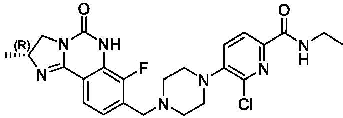 | 186 | 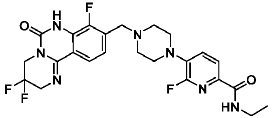 |
| 187 | 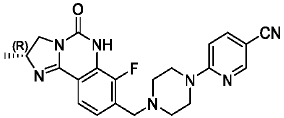 | 188 | 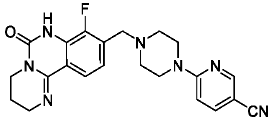 |
| 189 | 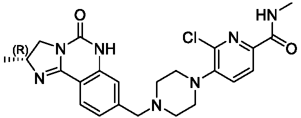 | 190 | 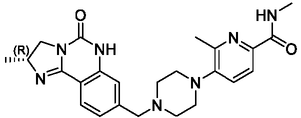 |
| 191 | 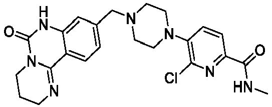 | 192 | 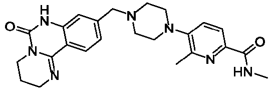 |
| 193 | 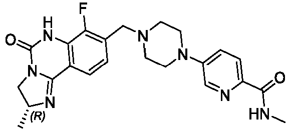 | 194 | 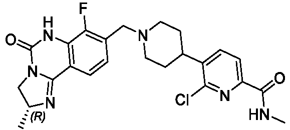 |
| 195 | 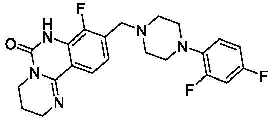 | 196 | 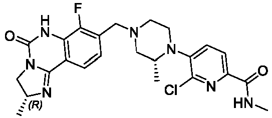 |
| 197 | 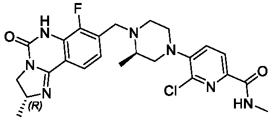 | 198 | 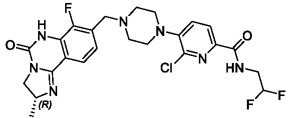 |
| 199 | 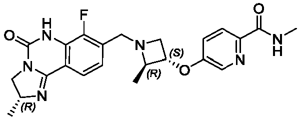 | 200 | 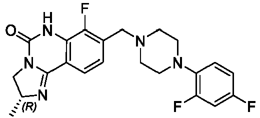 |
| 201 | 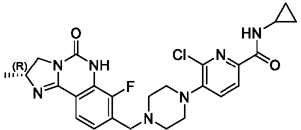 | 202 | 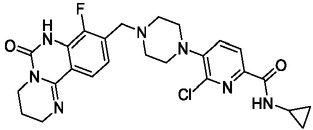 |
| 203 | 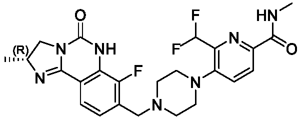 | 204 | 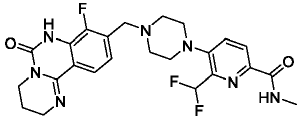 |

On the other hand, the present invention provides a pharmaceutical composition comprising a therapeutically effective amount of the compound of formula (I), or the stereoisomer, the geometric isomer, the tautomer, the pharmaceutically acceptable salt, the crystal form, the solvate, the hydrate or the prodrug thereof, and a pharmaceutically acceptable carrier.

In certain embodiments of the pharmaceutical composition, the pharmaceutical composition is formulated for intravenous administration, intramuscular administration, oral administration, rectal administration, inhalation administration, nasal administration, topical administration, ocular administration or ear administration. In other embodiments of the pharmaceutical composition, the pharmaceutical composition is a tablet, a pill, a capsule, a liquid, an inhalant, a nasal spray solution, a suppository, a solution, an emulsion, an ointment, an eye drop or an ear drop. In other embodiments of the pharmaceutical composition, it further comprises one or more additional therapeutic agents.

On the other hand, the present invention provides use of the compound of formula (I), or the stereoisomer, the geometric isomer, the tautomer, the pharmaceutically acceptable salt, the crystal form, the solvate, the hydrate or the prodrug thereof, or the pharmaceutical composition in the preparation of a drug for preventing, treating or improving a disease by inhibiting PARP1.

On the other hand, the present invention provides a method for preventing, treating or improving a disease by inhibiting PARP1, the method comprising administering an effective amount of the compound of formula (I), or the stereoisomer, the geometric isomer, the tautomer, the pharmaceutically acceptable salt, the crystal form, the solvate, the hydrate or the prodrug thereof, or the pharmaceutical composition to an individual in need of such treatment.

In some embodiments of the present invention, the disease includes but is not limited to cancer.

In some embodiments of the present invention, the cancer includes but is not limited to malignant tumors, such as anyone of ovarian cancer, breast cancer, fallopian tube cancer, endometrial cancer, peritoneal cancer, gastric cancer, colon cancer, bladder cancer, pancreatic cancer, biliary cancer, osteosarcoma, cervical cancer, head and neck tumors, germ cell and embryonic cancer, esophageal cancer, malignant glioma, Ewing sarcoma, pancreatic cancer, melanoma, bile duct cancer, prostate cancer, small cell lung cancer, non-small cell lung cancer, lymphoma and blood cancer.

In some embodiments of the present invention, the genome of the cancer is a type of homologous recombination repair deficiency.

In some embodiments of the present invention, the cancer relies on a pathway where DNA double-strand damage is deficient in homologous recombination repair.

In some embodiments of the present invention, the cancer comprises one or more cancer cells that lack the ability to repair DNA double-strand breaks by homologous recombination relative to normal cells.

In some embodiments of the present invention, the cancer comprises one or more cancer cells that lack BRCA1 or BRCA2, or have a BRCA1 or BRCA2 mutation.

### Terms

In the present invention, unless otherwise explicitly stated, the terms used in the present invention have the meanings defined below. Terms not explicitly defined in the present invention have the general meanings generally understood by those skilled in the art.

As used herein, the term "halogen" or "halo" refers to fluorine, chlorine, bromine and iodine.

As used herein, "heteroaryl" refers to a monocyclic ring system having 5-6 (5-6 members) or 6 (6 members) ring atoms, which contains 1, 2, 3 or 4 heteroatoms independently selected from N, O and S, and the remaining ring atoms are aromatic rings of carbon atoms. For example, it contains 1 N heteroatom and optionally further contains 1, 2 or 3 heteroatoms independently selected from N, O and S. When the total number of S and O atoms in the heteroaryl exceeds 1, these S and O heteroatoms are not adjacent to each other. For example, the heteroaryl includes but is not limited to: 5-6 membered monocyclic heteroaryl, that is, a monocyclic aromatic hydrocarbon group having 5 or 6 ring atoms, which contains 1, 2 or 3 heteroatoms independently selected from N, O and S, and the remaining ring atoms are carbon atoms; examples of the heteroaryl include but are not limited to: pyrrolyl, pyrazolyl, imidazolyl, furanyl, thienyl, thiazolyl, isothiazolyl, pyrazinyl, pyridazinyl, pyridinyl or pyrimidinyl.

As used herein, "heterocyclyl" refers to a fully saturated or partially saturated, non-aromatic monocyclic, bicyclic or tricyclic cyclic having 3-15 ring atoms (e.g., 4-12 ring atoms, 3-10 ring atoms, 5-10 ring atoms, 4-7 ring atoms, 5-6 ring atoms), for example, a 4- to 7-membered monocyclic, 5- to 6-membered monocyclic ring system, containing 1, 2, 3 or 4 heteroatoms independently selected from N, O and S, and the remaining ring atoms are carbon atoms. Wherein nitrogen heteroatoms and sulfur heteroatoms can also be optionally oxidized, for example, sulfur heteroatoms can form -S(O)- or -S(O)₂-structures. The heterocyclyl can be a monocyclic group, a bicyclic group, a condensed ring group, a spirocyclic group and a bridged ring group. "5-6 membered heterocyclyl" means a heterocyclyl having 5 or 6 ring atoms, which contains 1, 2, 3 or 4 heteroatoms independently selected from N, O and S, for example, contains 1, 2 or 3 N heteroatoms, which is a monocyclic ring.

As used herein, "heterocycloalkyl" is a fully saturated heterocyclyl as defined herein. For example, "3-8 membered heterocycloalkyl" is a saturated heterocyclic ring having 3-8 ring atoms, which contains 1, 2, 3 or 4 heteroatoms independently selected from N, O and S, for example, contains 1 N or O heteroatom.

The terms "optional", "optional" or "optionally" as used herein mean that the substitution pattern, event or situation described subsequently may or may not occur, and the description includes situations where the substitution pattern occurs as well as situations where the substitution pattern does not occur. For example, "optionally substituted alkyl" includes "unsubstituted alkyl" and "substituted alkyl" as defined herein. It should be understood by those skilled in the art that for any group containing one or more substituents, the group does not include any substitution pattern that is sterically impractical, chemically incorrect, synthetically unfeasible and/or inherently unstable.

As used herein, the term "pharmaceutically acceptable salt" refers to a salt that retains the biological effects and properties of the compounds of the present invention, and the salt is not biologically or otherwise undesirable. Non-limiting examples of the salt include non-toxic, inorganic or organic base or acid addition salts of the compounds of the present invention. In many cases, the compounds of the present invention are able to form acid salts and/or base salts due to the presence of amino and/or carboxyl groups or groups similar thereto. Pharmaceutically acceptable acid addition salts can be formed with inorganic and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; ammonium, potassium, sodium, calcium, and magnesium salts are particularly preferred. Organic bases from which salts can be derived include, for example, primary, secondary, and tertiary amines, substituted amines (including naturally occurring substituted amines), cyclic amines, basic ion exchange resins, and the like, particularly such as isopropylamine, trimethylamine, diethylamine, triethylamine, tripropylamine, and ethanolamine. Pharmaceutically acceptable salts of the present invention can be synthesized from the parent compound (alkaline or acidic part) by conventional chemical methods. Generally speaking, the salt can be prepared as follows: the free acid form of the compound is reacted with a stoichiometric amount of a suitable base (e.g., hydroxide, carbonate, bicarbonate, etc. of Na, Ca, Mg or K) or the free base form of the compound is reacted with a stoichiometric amount of a suitable acid. Such reactions are usually carried out in water or an organic solvent or a mixed solvent of the two. Generally speaking, when feasible, non-aqueous media such as ether, ethyl acetate, ethanol, isopropanol or acetonitrile are preferred. Other suitable salts can be found in Remington's Pharmaceutical Sciences, 20th edition, Mack Publishing Company, Easton, Pa., (1985), which is incorporated herein by reference.

As used herein, the term "pharmaceutically acceptable excipient" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, binders, excipients, disintegrants, lubricants, sweeteners, flavoring agents, dyes, similar substances and combinations thereof, which are well known to those of ordinary skill in the art (see, e.g., Remington's Pharmaceutical Sciences, 18th edition, Mack Printing Company, 1990, pp. 1289-1329, incorporated herein by reference). Unless any conventional carrier is incompatible with the active ingredient, it may be considered for use in a therapeutic or pharmaceutical composition.

As used herein, the term "solvate" means a stoichiometric or non-stoichiometric solvent addition form. If the solvent is water, the solvate formed is a hydrate, and when the solvent is ethanol, the solvate formed is an ethanolate. Hydrates are formed by one or more molecules of water and one molecule of the substance, wherein the water retains its molecular state of H₂O, and such a combination can form one or more hydrates, such as hemihydrates, monohydrates and dihydrates.

As used herein, "prodrug" refers to a chemically modified active or inactive compound, which, after administration to an individual, undergoes physiological effects in the body (such as hydrolysis, neogenesis, etc.) to become a compound of the present invention. The adaptability and technology of making and using prodrugs are well known to those skilled in the art.

The term "therapeutically effective amount" of a compound of the present invention refers to an amount of a compound of the present invention that can cause a biological or medical response in an individual or improve symptoms, slow down or delay the progression of a disease, or prevent a disease, etc.

As used herein, the term "subject" refers to an animal. Preferably, the animal is a mammal. The subject also refers to, for example, primates (such as humans), cattle, sheep, goats, horses, dogs, cats, rabbits, rats, mice, fish, birds, etc. In a preferred embodiment, the subject is a human.

As used herein, the term "inhibit" refers to the alleviation or suppression of a particular disease, symptom or condition or disease, or a significant reduction in the baseline activity of a biological activity or process.

As used herein, in one embodiment, the term "treating" any disease or condition refers to ameliorating the disease or condition (i.e., preventing or slowing the development of the disease or at least one clinical symptom thereof). In another embodiment, "treating" refers to improving at least one physical parameter, which may not be perceived by the patient. In another embodiment, "treating" refers to regulating the disease or condition physically (e.g., stabilizing perceptible symptoms) or physiologically (e.g., stabilizing physical parameters) or both.

### Beneficial Effects

The main advantage of the present invention is that the compounds of the present invention have high selectivity for PARP1, fewer side effects than olaparib (AZD-2281), and have high clinical application value.

### EMBODIMENTS

Unless otherwise specified, the experimental materials and reagents used in the following examples can be obtained from commercial channels. Raw materials can usually be obtained from commercial sources or easily prepared using methods known to those skilled in the art.

In each example, the experimental instruments or materials used are as follows:
¹H NMR was recorded by Varian Mercury-300 or Varian Mercury-400 nuclear magnetic resonance instrument, ¹³C NMR was recorded by Varian Mercury-400 or Varian Mercury-500 or Varian Mercury-600 nuclear magnetic resonance instrument, chemical shift was represented by δ (ppm); the mass spectrometry was recorded by Finnigan/MAT-95 (EI) and Finnigan LCQ/DECA and Micromass Ultra Q-TOF (ESI) mass spectrometers; reversed phase preparation of silica gel for HPLC separation was 200-300 meshes).

### Abbreviations

| DMAP | 4-dimethylaminopyridin e | PE | Petroleum ether |
|---|---|---|---|
| EA | Ethyl acetate | DCM | Dichloromethane |
| DMF | Dimethylformamide | DIEA | N,N-diisopropylethylamine |
| THF | Tetrahydrofuran | PdCl₂(PhP₃) 2 | Bis(triphenylphosphine)palladium(I I) chloride |
| DMA C | N,N-Dimethylacetamide | T₃P | Propylphosphonic anhydride |
| HATU | 2-(7-azobenzotriazole)-N,N,N',N'-tetramethylurea hexafluorophosphate | | |
| XPhos Pd G2 | Chloro(2-dicyclohexylphosphino-2',4',6'-triisopropyl-1,1'-biphenyl)[2-(2'-amino-1,1'-biphenyl)]palladium(II) | | |
| Rupho s Pd G3 | Methanesulfonato(2-dicyclohexylphosphino-2',6'-di-i-propoxy-1,1'-biphenyl)(2'-amino-1,1'-biphenyl-2-yl)palladium(II) | | |

### Synthesis of Key Intermediates

Intermediate **1a:** (R)-6-fluoro-N-methyl-5-(2-methylpiperazin-1-yl)picolinamide

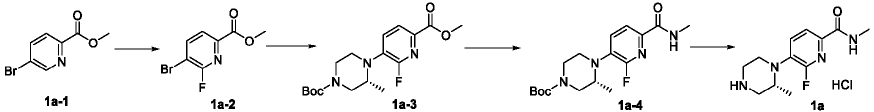

### Step 1: synthesis of methyl 5-bromo-6-fluoropicolinate

Compound **1a-1** (1 g, 4.6 mmol), acetonitrile (30 mL) and silver difluoride (1.76 g, 13.9 mmol) were added sequentially in a 50 mL single-neck flask and the mixture was stirred overnight. The reaction solution was filtered and the filtrate was concentrated and purified by silica gel column (PE:EA = 5:1) to obtain **la-2** (450 mg, white solid), yield: 42%. LCMS (ESI): m/z 233.9[M+H]⁺; RT= 1.51 min (3.00 min).

Step 2: synthesis of tert-butyl (R)-4-(2-fluoro-6-(methoxycarbonyl)pyridin-3-yl)-3-methylpiperazine-1-carboxylate

**1a-2** (450 mg, 1.9 mmol), (R)-4-Boc-2-methylpiperazine (577 mg, 2.9 mmol), Ruphos Pd G3 (159 mg, 0.19 mmol), cesium carbonate (1.5 g, 4.7 mmol), and dioxane (6 mL) were sequentially added to a 50 mL single-neck flask. The mixture was heated at 80°C under nitrogen overnight. The reaction solution was concentrated and purified by silica gel column (PE:EA= 2:1) to obtain the la-3 (300 mg, yellow solid), yield: 44%. LCMS (ESI): m/z 354.1 [M+H]⁺; RT= 1.80 min (3.00 min).

### Step 3: synthesis of tert-Butyl (R)-4-(2-fluoro-6-(methylcarbamoyl)pyridin-3-yl)-3-methylpiperazine-1-carboxylate

To a 50 mL single-necked vial was added **1a-3** (100 mg, 0.28 mmol), methylamine ethanol solution (2 mL) and the solution was stirred overnight. The mixture was concentrated and gave **la-4** (80 mg, yellow solid), yield: 80%. LCMS (ESI): m/z 297.1 [M-56+H]⁺; RT= 1.70 min (3.00 min).

### Step 4: synthesis of (R)-6-fluoro-N-methyl-5-(2-methylpiperazin-1-yl)picolinamide

A20 mL single-necked flask was charged with **la-4** (80 mg, 0.23 mmol), EA (2 mL), 4M HCl/dioxane solution (2 mL). The mixture was stirred at rt for 2 h. After concentrated, **1a** (65 mg, yellow oil, crude) was obtained, yield: 100%. LCMS (ESI): m/z 253.2 [M+H]⁺; RT= 0.99 min (3.00 min).

Intermediate **2a:** synthesis of 6-fluoro-N-methyl-5-(piperazin-1-yl)pyridinamide

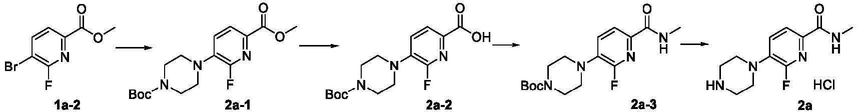

### Step 1: synthesis of tert-butyl 4-(2-fluoro-6-(methoxycarbonyl)pyridin-3-yl)piperazine-1-carboxylate

To a 50 mL flask was added **1a-2** (500 mg, 2.1 mmol), 1-tert-butoxycarbonylpiperazine (600 mg, 3.2 mmol), Ruphos Pd G3 (180 mg, 0.21 mmol), cesium carbonate (1.7 g, 5.2 mmol), and dioxane (15 mL), and the mixture was heated at 80°C under nitrogen overnight. The reaction solution was concentrated and the residue was purified by silica gel column (PE:EA=2:1) to obtain **2a-1** (640 mg, yellow solid), yield: 88%. LCMS (ESI): m/z 340.1[M+H]⁺; RT= 1.74 min (3.00 min).

### Step 2: synthesis of 5-(4-(tert-butoxycarbonyl)piperazin-1-yl)-6-fluoropyridinecarboxylic acid

To a dry 50 mL single-necked flask, **2a-1** (320 mg, 0.94 mmol) and THF (8 mL) was added. Lithium hydroxide monohydrate (200 mg, 4.7 mmol) aqueous solution (8 mL) was added dropwise. The mixture was stirred for 2 h. The reaction solution was adjusted to pH=6 with 1 M hydrochloric acid solution, and the reaction solution was concentrated and purified by reversed phase column (2%-40% aqueous acetonitrile) to obtain **2a-2** (300 mg, yellow solid), yield: 92%. LCMS (ESI): m/z 326.1[M+H]⁺; RT= 1.26 min (3.00 min).

### Step 3: synthesis of tert-butyl 4-(2-fluoro-6-(methylcarbamoyl)pyridin-3-yl)piperazine-1-carboxylate

To a dry 50 mL single-necked vial was added compound **2a-2** (300 mg, 0.92 mmol), 1-hydroxybenzotriazole (149 mg, 1.1 mmol), 1-(3-dimethylaminopropyl)-3-ethylcarbodiimide HCl (211 mg, 1.1 mmol), DIEA (237 mg, 1.84 mmol), DMF (6 mL) and methylamine hydrochloride (123 mg, 1.84 mmol). The mixture was stirred at rt overnight. The reaction solution was added with water (20 mL), extracted by EA (30 mL). The organic phase was washed with saturated saline (20 mL), dried with anhydrous sodium sulfate, filtered. The filtrate was concentrated, and the residue was purified by reversed phase column (20%~70% aqueous acetonitrile) to obtain **2a-3** (250 mg, white solid), yield: 80%. LCMS (ESI): m/z 283.1 [M-100+H]⁺; RT= 1.66 min (3.00 min).

### Step 4: synthesis of 6-fluoro-N-methyl-5-(piperazin-1-yl)pyridinamide hydrochloride

To a dry 20 mL single-necked flask was added **2a-3** (250 mg, 0.74 mmol), EA (3 mL), 4M hydrochloric acid dioxyhexane solution (1 mL) sequentially. The mixture was stirred at rt for 2 h and then concentrated to obtain the **2a** (200 mg, yellow solid). Yield: 100%. LCMS (ESI): *m*/*z* 239.1 [M+H]⁺, RT= 0.91 min (3.00 min). ¹HNMR (600 MHz, CD₃OD): 7.98-7.96 (m, 1H), 7.67-7.64 (m, 1H), 3.50-3.48 (m, 4H), 3.45-3.43 (m, 4H), 2.93 (d, *J* = 3.6 Hz, 3H).

Intermediate **3a:** synthesis of N-methyl-5-(piperazin-1-yl)pyridineamide hydrochloride

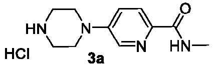

The synthesis method refers to that of intermediate **1a**, except that Boc-piperazine is used instead of (R)-4-Boc-2-methylpiperazine in step 2 of the preparation method of intermediate **1a**, and compound **1a-1** is used instead of compound **1a-2** in step 2. LCMS(ESI): m/z 221.2 [M+H]⁺; RT=0.285 min (6.00 min).

Intermediate **5a:** Methyl (6-fluoro-4-oxo-4,5-dihydropyrrolo[1,2-a]quinoxalin-7-yl)methanesulfonate

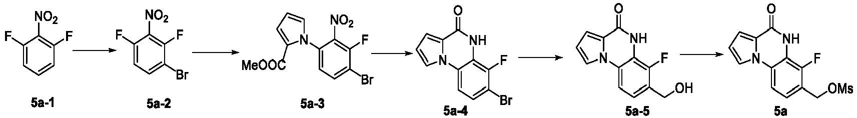

### Step 1: synthesis of 1-bromo-2,4-difluoro-3-nitrobenzene

**5a-1** (25.0 g, 157 mmol) and 100 mL of concentrated sulfuric acid were added to a 250 mL single-necked bottle. N-bromosuccinimide (33.6 g, 188 mmol) was slowly added under ice bath. The reaction was allowed to react overnight at 80°C. The reaction solution was slowly poured into ice water for dilution. The mixture was extracted with EA twice. After drying and concentration, the product **5a-2** (36 g, yellow oil) was obtained by column chromatography (PE). Yield: 96.6%, ¹H NMR (400 MHz, DMSO-*d₆*): δ 8.19-8.14 (m, 1H), 7.56-7.51 (m, 1H).

### Step 2: synthesis of 1-(4-bromo-3-fluoro-2-nitrophenyl)-1H-pyrrole-2-carboxylic acid methyl ester

**5a-2** (0.5 g, 2.1 mmol), 1H-pyrrole-2-carboxylic acid methyl ester (244 mg, 1.89 mmol) and cesium carbonate (1.37 g, 4.2 mmol) were added to 15 mL DMF. The mixture was reacted at rt for 5 h. The reaction solution was diluted with water, extracted twice with EA, dried and concentrated. The residue was purified by column chromatography (PE:EA=5:1) to obtain **5a-3** (4.0 g, yellow solid). Yield: 65.5%, LCMS (ESI): m/z 345.0 [M+H]⁺; RT= 1.783 min (2.50 min).

### Step 3: synthesis of 7-bromo-6-fluoropyrrolo[1,2-a]quinoxaline-4(5H)-one

**5a-3** (6.5 g, 18.9 mmol), iron powder (21 g, 37.9 mmol) was added to a single-mouth bottle containing 250 mL of acetic acid. The mixture was stirred at 110°C for 3h. Add EA to the reaction solution and filter it directly. The filtrate was concentrated and saturated sodium bicarbonate solution was added. The resulting mixture was extracted with EA three times. The combined organic phase was washed with water, dried and concentrated to obtain **5a-4** (650 mg, yellow solid). Yield: 12.2%, ¹H NMR (400 MHz, DMSO-*d₆*): δ 11.46 (s, 1H), 8.22 (m, 1H), 7.89 (d, *J*=8.0 Hz, 1H), 7.53-7.48 (m, 1H), 7.10 (d, *J*=3.2 Hz, 1H), 6.74-6.73 (m, 1H).

### Step 4: synthesis of 6-fluoro-7-(hydroxymethyl)pyrrolo[1,2-a]quinoxaline-4(5H)-one

The mixture of **5a-4** (700 mg, 2.49 mmol), XPhos Pd G2 (197 mg, 0.25 mmol) and (tributylstannyl)methanol (960 mg, 2.99 mmol) in anhydrous dioxane (15 mL) was stirred at 100°C overnight. The reaction solution was quenched with potassium fluoride solution, filtered, and the filtrate was concentrated to obtain **5a-5** (430 mg, yellow solid), yield: 74.4%. LCMS(ESI): m/z 233.1 [M+H]⁺; RT=0.877 min (2.00 min). ¹H NMR (400 MHz, DMSO-*d₆*) : δ 11.23 (s, 1H), 8.18-8.17 (m, 1H), 7.87 (d, *J*=8.4 Hz, 1H), 7.28-7.24 (m, 1H), 7.08-7.07 (s, 1H), 6.72-6.70 (m, 1H), 5.36-5.33 (m, 1H), 4.59 (d, *J*=5.6Hz, 2H).

### Step 5: synthesis of (6-fluoro-4-oxo-4,5-dihydropyrrolo[1,2-a]quinoxaline-7-yl)methylsulfonate

To a mixture of **5a-5** (150 mg, 0.65 mmol), triethylamine (164 mg, 1.63 mmol) in 8 mL THF (8 mL) was added methylsulfonyl chloride (89 mg, 0.78 mmol) under ice bath. The mixture was reacted at rt for 3h. The mixture was concentrated to obtain **5a** (120 mg, yellow solid). LCMS (ESI): m/z 309.1 [M-H]⁻; RT= 1.241 min (2.50 min).

Intermediate **6a:** 7-(1-Bromoethyl)pyrrolo[1,2-a]quinoxalin-4(5H)-one

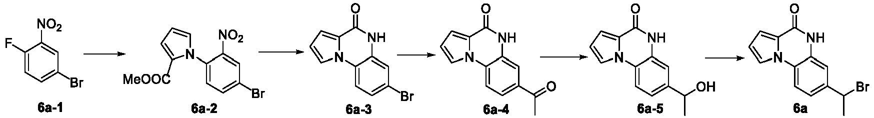

### Step 1: synthesis of methyl 1-(4-bromo-2-nitrophenyl)-1H-pyrrole-2-carboxylate

**6a-1** (500 mg, 2.27 mmol), methyl 1H-pyrrole-2-carboxylate (341.26 mg, 2.73 mmol) and cesium carbonate (1.48 g, 4.55 mmol) were added to DMF (10 mL). The mixture was stirred for 16 h at 80°C. The reaction solution was diluted with water (20 mL), extracted by EA (20 mL×3), dried and concentrated. The residue was purified by column chromatography (PE: EA=10:1) to afford **6a-2** (383 mg, 1.18 mmol, yield: 51.9 %). LCMS (ESI): m/z 325.0 [M+H]⁺; RT= 1.341 min (2.50 min).

### Step 2: synthesis of 7-bromopyrrolo[1,2-a]quinoxalin-4(5H)-one

**6a-2** (1.5 g, 4.61 mmol) and iron powder (5.15 g, 92.27 mmol) were added to acetic acid (50 mL) and the mixture was stirred at 110°C for 3 h and then concentrated to afford a dark brown solid, which was diluted by DCM. The organic phase was washed with saturated aqueous sodium bicarbonate and saturated saline, respectively, dried over anhydrous sodium sulfate, and concentrated to obtain **6a-3** (1.3 g, 4.49 mmol), yield: 100%, yellow solid). ¹H NMR (400 MHz, DMSO-*d₆*) : δ 11.35 (s, 1H), 8.20 (s, 1H), 8.03 (d, *J*=8.4 Hz, 1H), 7.45 (s, 1H), 7.37 (d, *J*=8.4 Hz, 1H), 7.06 (d, *J*=3.6 Hz, 1H), 6.71 (d, *J*=2.8 Hz, 1H).

### Step 3: synthesis of 7-acetylpyrrolo[1,2-a]quinoxalin-4(5H)-one

**6a-3** (500 mg, 1.9 mmol), tributyl(1-ethoxyvinyl)tin (1.37 g, 3.8 mmol) and PdCl₂(PPh₃)₂ (200 mg, 0.29 mmol) were added to a solution of 1,4-dioxane (25 mL). The mixture was reacted for 16 h at 100°C. After cooled to rt, 1M aqueous hydrochloric acid (10 mL) was added. The mixture was stirred for 10 min, extracted with EA (10 mL×3), dried and concentrated. The residue was purified by column chromatography (THF: PE=1:1) afforded **6a-4** (220 mg, 0.97 mmol, crude). Yield: 51%. LCMS (ESI): m/z 227 [M+H]⁺; RT= 0.947 (2.50 min).

### Step 4: synthesis of 7-(1-hydroxyethyl)pyrrolo[1,2-a]quinoxalin-4(5H)-one

To a solution of **6a-4** (220 mg, 0.97 mmol) in methanol (5 mL) was added sodium borohydride (73.58 mg, 1.94 mmol) at 0°C. The mixture was reacted at rt for 2 h. The reaction was quenched with saturated aqueous ammonium chloride solution (5 mL), extracted by EA, dried and concentrated. The residue was purified by column chromatography (DCM: methanol=1:1) to afford **6a-5** (200 mg, 0.88 mmol), yield: 90.3%, white solid. LCMS (ESI): *m*/*z* 229.1 [M-H]⁻, RT= 0.877 min (2.50 min). ¹H NMR (400 MHz, DMSO-*d₆*) : δ 11.23 (s, 1H), 8.15 (s, 1H), 7.98 (d, *J*=8.4 Hz, 1H), 7.33 (s, 1H), 7.16 (d, *J*=8.4 Hz, 1H), 7.02 (d, *J*=3.6 Hz, 1H), 6.67 (t, *J*=3.2 Hz, 1H), 5.30 (d, *J*=4.0 Hz, 1H), 4.78-4.75 (m, 1H), 1.35 (d, *J*=6.4 Hz, 3H).

### Step 5: synthesis of 7-(1-bromoethyl)pyrrolo[1,2-a]quinoxalin-4(5H)-one

To a solution of **6a-5** (200 mg, 0.87 mmol) in DCM (5 mL) was added phosphine tribromide (711.55 mg, 2.63 mmol) slowly at 0°C. The mixture was reacted at rt for 2 h. The reaction solution was quenched with water, extracted by EA, dried and concentrated to obtain the crude product **6a** (200 mg, 0.69 mmol), yield: 79.0%, white solid). LCMS (ESI): m/z 289.1 [M-H]⁻; RT=1.447 (2.50 min).

Intermediate **7a:** 7-(1-Bromomethyl)-6-F pyrazolo[1,2-a]quinoxalin-4(5H)-one

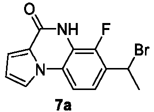

The synthesis method refers to that of intermediate **6a,** except that **5a-4** is used instead of **6a-3** used in step 4 of intermediate **6a.** LCMS(ESI): m/z 310.0 [M+H]⁺; RT=1.202 min (2.50 min).

Intermediate **8a:** 7-(Bromomethyl)pyrazolo[1,5-a]quinoxalin-4(5H)-one

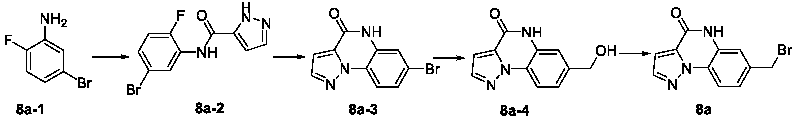

### Step 1: synthesis of N-(5-bromo-2-fluorophenyl)-1H-pyrazole-5-carboxamide

**8a-1** (3.0 g, 15.8 mmol), 1H-pyrazole-5-carboxylic acid (1.95 g, 17.4 mmol), DIEA (6.1 g, 47.4 mmol) and HATU (9.0 g, 23.7 mmol) were added to a single-necked bottle containing 30 mL of DMF. The mixture was reacted at rt for 16 h. The reaction solution was diluted with water, extracted with EA, washed with saturated sodium chloride solution, dried and concentrated. The residue was purified by column chromatography (PE:EA=5:1) to obtain **8a-2** (2.0 g, yellow solid). Yield: 44.8%, LCMS (ESI): m/z 286.0 [M+H]⁺; RT= 1.03 min (2.00 min).

### Step 2: synthesis of 7-bromopyrazolo[1,5-a]quinoxaline-4(5H)-one

To a solution **of 8a-2** (2.0 g, 7.07 mmol) in DMAC (30 mL) was add sodium hydride (565 mg, 14.1 mmol) under ice bath. The mixture was react at 145°C for 16h, and quenched with saturated ammonium chloride solution. The mixture was filtered, dried to obtain 8a-**3** (1.5 g, yellow solid). Yield: 80.7%, LCMS (ESI): m/z 264.0/266.0 [M+H]⁺; RT= 0.93 min (2.00 min). ¹H NMR (400 MHz, DMSO-*d₆*): δ 11.50 (s, 1H), 8.03-7.97 (m, 2H), 7.47-7.39 (m, 2H), 7.10 (s, 1H).

### Step 3: synthesis of 7-(hydroxymethyl)pyrazolo[1,5-a]quinoxalin-4(5H)-one

The mixture **of 8a-3** (500 mg, 1.90 mmol), (tributylstannyl)methanol (671 mg, 2.09 mmol) and XPhos Pd G2 (75 mg, 0.095 mmol) in dioxane (10 mL) was stirred at 80°C for 16 h. The reaction solution was concentrated. The residue was purified by column chromatography (DCM: methanol = 15:1) to obtain the **8a-4** (190 mg, yellow solid), yield: 45.0%. LCMS (ESI): m/z 214.1 [M-H]⁻; RT = 0.984 min (2.50 min).

### Step 4: synthesis of 7-(bromomethyl)pyrazolo[1,5-a]quinoxaline-4(5H)-one

To a mixture of **8a-4** (80 mg, 0.37 mmol), triphenylphosphine (293 mg, 1.12 mmol) in DCM (8 mL) was added carbon tetrabromide (248 mg, 0.74 mmol). The reaction was carried out at rt for 2 h. The reaction solution was concentrated to obtain the **8a** (80 mg, yellow solid, crude). LCMS (ESI): m/z 278.0 [M+H]⁺; RT= 1.405 min (2.50 min).

Intermediate **9a:** 7-(1-Bromoethyl)pyrazolo[1,5-a]quinoxalin-4(5H)-one

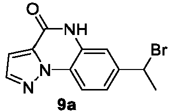

The synthesis method refers to that of intermediate **6a,** except that **8a-3** in step 2 of intermediate 8a is used instead of **6a-3** in step 4 of intermediate 6a. LCMS(ESI): m/z 290.0 [M-H]⁻, RT=1.300 min (2.50 min).

Intermediate **10a:** synthesis of (6-fluoro-4-oxo-4,5-dihydropyrazolo[1,5-a]quinoxaline-7-yl)methanesulfonate

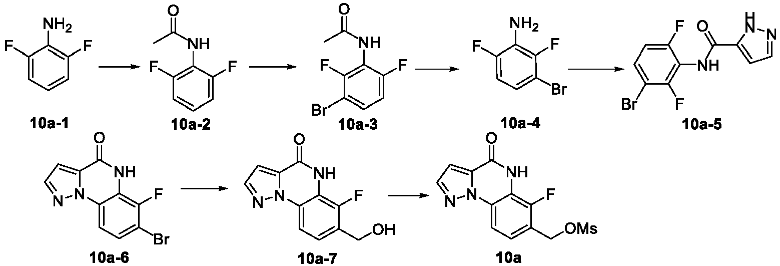

### Step 1: synthesis of N-(2,6-difluorophenyl)acetamide

To a solution of **10a-1** (2.00 g, 15.49 mmol) in DCM (20 mL) was added acetic anhydride (1.53 mL, 16.27 mmol). The mixture was stirred at rt for 16 h. The reaction solution was concentrated. The residue was diluted with water (20 mL) and the pH was adjusted to 8 with saturated sodium bicarbonate aqueous solution. A white solid precipitated and then filtered to obtain **10a-2** (2.60 g, white solid), yield: 98.07%, LCMS (ESI): m/z 172.2 [M+H]⁺; RT=1.036 min (2.50 min). ¹H NMR (400 MHz, DMSO-*d₆*): δ 9.69 (s, 1H), 7.37-7.30 (m, 1H), 7.17-7.13 (m, 2H), 2.08 (s, 3H).

### Step 2: synthesis of N-(3-bromo-2,6-difluorophenyl)acetamide

To a mixture of **10a-2** (1.00 g, 5.84 mmol) in concentrated sulfuric acid (10 mL) was added N-bromosuccinimide (1.04 g, 5.84 mmol) were added. The mixture was stirred at rt and react for 16 h. The reaction solution was poured into ice water (50 mL), and a white solid precipitated. Filter under reduced pressure and collect the filter cake to obtain **10a-3** (1.26 g, white solid), yield: 86.24%, LCMS (ESI): m/z 291.0 [M+H+MeCN]⁺; RT=1.290 min (2.50 min).

### Step 3: synthesis of 3-bromo-2,6-difluoroaniline

The mixture of **10a-3** (1.00 g, 4.00 mmol), ethanol (6 mL) in concentrated hydrochloric acid (3 mL) was heated to 70°C and react for 2h. The reaction solution was concentrated. The residue was adjusted to pH=8 with saturated sodium bicarbonate aqueous solution and extracted with EA. The organic phase was collected, washed with saturated saline, dried over anhydrous sodium sulfate, filtered. The filtrate was concentrated under reduced pressure to obtain **10a-4** (650 mg, yellow solid), yield: 78.14%, LCMS (ESI): m/z 207.9 [M+H+MeCN]⁺; RT=1.636 min (2.50 min). 1H NMR(400 MHz, DMSO-d6): δ 6.93-6.88 (m, 1H), 6.81-6.76 (m, 1H), 5.54 (s, 2H).

### Step 4: synthesis of N-(3-bromo-2,6-difluorophenyl)-1H-pyrazole-5-carboxamide

To a mixture of 1H-pyrazole-5-carboxylic acid (3.23 g, 28.85 mmol), oxalyl chloride (2.28 mL, 26.92 mmol) in anhydrous DCM (40 mL) was added DMF (0.10 mL).The mixture was stirred at rt for 3 h, then slowly added **10a-4** (2.00 g, 9.62 mmol) in DCM (30 mL) and pyridine (15 mL) at 0°C. The mixture was stirred at rt for 16 h. The reaction solution was diluted with saturated aqueous ammonium chloride (100 mL) and extracted with DCM (80 mL×2). The organic phase was collected, washed with saturated saline, dried over anhydrous sodium sulfate, filtered. The filtrate was concentrated under reduced pressure. The residue was purified by column chromatography (PE:EA=5:1) to obtain **10a-5** (1.80 g, nearly white solid). Yield: 61.97%. LCMS (ESI): m/z 303.9 [M+H]⁺; RT=1.287 min (2.50 min). ¹H NMR(400 MHz, DMSO-*d₆*): δ 13.49 (s, 1H), 10.01 (s, 1H), 7.92 (d, J = 1.6 Hz, 1H), 7.75-7.69 (m, 1H), 7.26-7.21 (m, 1H), 6.77 (t, J = 2.0 Hz, 1H).

### Step 5: synthesis of 7-bromo-6-fluoropyrazolo[1,5-a]quinoxaline-4(5H)-one

In a dry 50mL three-necked flask, sodium hydride (60%wt, 185 mg, 4.63 mmol), DMAC (5 mL), and a solution of **10a-5** (700 mg, 2.32 mmol) in DMAC (5 mL) were added in sequence at 0°C. The mixture was heat to 145°C and reacted for 16 h and then diluted with saturated aqueous ammonium chloride solution (50 mL). A precipitate was filtered, and the filtrate is extracted with EA (15 mL×2). Collect the organic phase, wash with saturated saline, dry over anhydrous sodium sulfate, filter, and concentrate the filtrate under reduced pressure. The residue was slurried with EA (10 mL), filtered, and the filter cake was collected to obtain the crude product **10a-6** (180 mg, yellow solid), LCMS (ESI): m/z 282.0 [M+H]⁺; RT = 1.470 min (2.50 min). ¹H NMR(400 MHz, DMSO-*d₆*): δ 12.08 (s, 1H), 8.13 (d, J = 2.0 Hz, 1H), 7.89 (dd, J₁ = 1.6 Hz, J₂ = 9.6 Hz, 1H), 7.59-7.56 (m, 1H), 7.22 (d, J = 2.0 Hz, 1H).

### Step 6: synthesis of 6-fluoro-7-(hydroxymethyl)pyrazolo[1,5-a]quinoxaline-4(5H)-one The mixture of 10a-6 (180 mg, 0.64 mmol) XPhos Pd G2 (50 mg, 0.06 mmol)

(tributyltin)methanol (246 mg, 0.77 mmol) in 1,4-dioxane (5 mL) was heated to 80°C and reacted for 16 h. The reaction solution was filtered, and the filtrate was concentrated. The residue was slurried with EA (10 mL), filtered, and the filter cake was collected to obtain **10a-7** (48 mg, yellow solid), yield: 32.25%, LCMS (ESI): m/z 234.1 [M+H]⁺; RT=0.998 min (2.50 min).

### Step 7: synthesis of (6-fluoro-4-oxo-4,5-dihydropyrazolo[1,5-a]quinoxaline-7-yl)methanesulfonate

To a solution of **10a-7** (48 mg, 0.21 mmol), triethylamine (0.09 mL, 0.62 mmol) in THF (5 mL) was added methanesulfonyl chloride (0.02 mL, 0.25 mmol). The reaction was carried out at rt for 1 h and concentrated. The residue was diluted with water (10 mL) and extracted with EA. The organic phase was collected, washed with saturated saline, dried over anhydrous sodium sulfate, filtered, and the filtrate was concentrated under reduced pressure to obtain **10a** (58 mg, yellow solid). LCMS (ESI): m/z 312.0 [M+H]⁺; RT = 1.223 min (2.50 min).

Intermediate **11a:** synthesis of 7-(bromomethyl)-6-fluoro-1-methyl-1,5-dihydro-4H-pyrazolo[4,3-c]quinolin-4-one

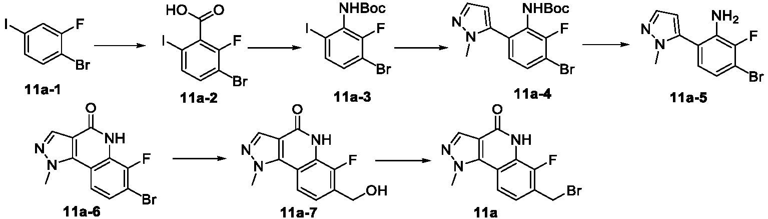

### Step 1: synthesis of 3-bromo-2-fluoro-6-iodobenzoic acid

To a solution of **11a-1** (4.0 g, 13.3 mmol) in 40 mL of THF was added LDA (7.3 mL, 14.6 mmol) -78°C. After 1 h, the temperature was raised to rt and reacted overnight. The reaction solution was quenched with saturated ammonium chloride, diluted with water, and the pH was adjusted to 8-9 with dilute sodium hydroxide solution. The mixture was extracted with EA twice. The pH of the aqueous phase was adjusted to acidic with dilute hydrochloric acid and extracted with EA twice. The EA phase was combined and washed with water twice, washed with saturated saline once, dried over anhydrous sodium sulfate, and concentrated to obtain **11a-2** (3.2 g, yellow solid). Yield: 69.8%, ¹H NMR (400 MHz, CDCl₃): δ 7.68 (d, *J=*8.4 Hz, 1H), 7.57-7.53 (m, 1H).

### Step 2: synthesis of tert-butyl (3-bromo-2-fluoro-6-iodophenyl)carbamate

The mixture of **11a-2** (3.0 g, 8.7 mmol), diphenylphosphoryl azide (2.87 g, 10.4 mmol), triethylamine (1.05 g, 10.4 mmol) was stirred at 120°C for 1 h. Then tert-butyl alcohol was added, and the reaction was continued for 3 h. The reaction solution was concentrated and the residue was purified by column chromatography (PE/EA=30/1) to obtain **11a-3** (2.9 mg, white solid). LCMS (ESI): m/z 413.8 [M-H]⁻; RT= 1.684 min (2.50 min).

### Step 3: synthesis of tert-butyl (3-bromo-2-fluoro-6-(1-methyl-1H-pyrazol-5-yl)phenyl)carbamate

To a mixture of **11a-3** (1.4 g, 3.37 mmol), 1-methyl-5-(4,4,5,5-tetramethyl-1,3,2-dioxaborolan-2-yl)-1H-pyrazole (631 mg, 3.04 mmol), potassium carbonate (1.40 g, 10.1 mmol) in dioxane (15 mL) and water (5 mL) was added Pd(dppf)Cl₂ (catalytic amount). The reaction was stirred at 70°C for 3h. After cooled to rt, the mixture was diluted with water and extracted with EA twice. The organic phases were combined and washed with water twice and saturated saline once, dried over anhydrous sodium sulfate, concentrated. The residue was purified by column chromatography (EA:PE=1:10) obtain **11a-4** (900 mg, yellow solid). Yield: 72.3%; LCMS (ESI): m/z 370.0 [M+H]⁺; RT=1.444 min (2.50 min).

### Step 4: synthesis of 3-bromo-2-fluoro-6-(1-methyl-1H-pyrazol-5-yl)aniline

The mixture of 11a-4 (900 mg, 2.44 mmol) in methanal/HCl (15 mL) was reacted at rt for 3h. The mixture was concentrated and diluted with water. pH was adjusted with saturated sodium bicarbonate solution. The mixture was extracted twice with EA. The EA phases were combined and washed twice with water, once with saturated saline, dried over anhydrous sodium sulfate, and concentrated to obtain **11a-5** (580 mg, yellow solid), yield: 88.4%; LCMS (ESI): m/z 270.3/272.3 [M+H]⁺; RT=1.22 min (2.00 min).

### Step 5: synthesis of 7-bromo-6-fluoro-1-methyl-1,5-dihydro-4H-pyrazolo[4,3-c]quinolin-4-one

**11a-5** (780 mg, 2.9 mmol), carbonyldiimidazole (1.41 g, 8.70 mmol) were added to a 50 mL single-necked bottle containing 15 mL DMF. The mixture was stirred at 170°C overnight, and then diluted with water. The solid precipitate was filtered and dried to obtain **11a-6** (650 mg, brown solid), yield: 76.0%. LCMS (ESI): m/z 296.2 [M+H]⁺; RT=0.85 min (2.00 min). ¹H NMR (400 MHz, DMSO-*d₆*) : δ 11.51 (s, 1H), 8.15 (s, 1H), 7.97 (d, *J*=8.8 Hz, 1H), 7.57-7.53 (m, 1H), 4.35 (s, 3H).

### Step 6: synthesis of 6-fluoro-7-(hydroxymethyl)-1-methyl-1,5-dihydro-4H-pyrazolo[4,3-c]quinolin-4-one

**11a-6** (200 mg, 0.68 mmol), (tributyltin)methanol (239 mg, 0.75 mmol) and XPhos Pd G2 (27 mg, 0.03 mmol) were added to 10 mL of dioxane. The mixture was stirred at 80°C for 16 h. Concentrate the reaction solution and slurry it with EA and filter it to obtain **11a-7** (140 mg, yellow solid). Yield: 83.8%, LCMS (ESI): m/z 248.2 [M+H]⁺; RT= 0.895 min (2.50 min).

### Step 7: synthesis of 7-(bromomethyl)-6-fluoro-1-methyl-1,5-dihydro-4H-pyrazolo[4,3-c]quinolin-4-one

The mixture of **11a-7** (70 mg, 0.28 mmol), triphenylphosphine (222 mg, 0.85 mmol) and carbon tetrabromide (188 mg, 0.57 mmol) in 8 mL DCM was reacted at rt for 2 h. The reaction solution was concentrated to obtain **11a** (50 mg, yellow solid, crude) LCMS (ESI): m/z 310.3 [M+H]⁺; RT = 0.64 min (2.00 min).

Intermediate **12a:** N-methyl-5-(pyrrolidin-3-yloxy)picolinamide hydrochloride

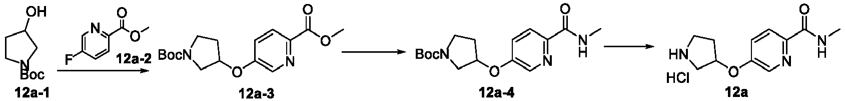

### Step 1: synthesis of 5-((1-(tert-butyloxycarbonyl)pyrrolidin-3-yl)oxy)picolinic acid methyl ester

To a mixture of **12a-1** (2 g, 10.7 mmol), **12a-2** (1.66 g, 10.7 mmol) in THF (40 mL) were added sodium hydride (641 mg, 16 mmol) in an ice bath under nitrogen. The reaction was carried out at rt for 1 h. The reaction solution was extracted with EA (30 mL×3). The EA phase was collected, washed with saturated saline, dried over anhydrous sodium sulfate, filtered. The filtrate was concentrated. The residue was purified by column chromatography (PE:EA = 3:1) to obtain **12a-3** (1 g, yellow oil), yield: 29%, LCMS (ESI): m/z 323.2 [M+H]⁺; RT=1.59 min (2.50 min).

### Step 2: synthesis of tert-Butyl 3-((6-(methylcarbamoyl)pyridin-3-yl)oxy)pyrrolidine-1-carboxylate

In a dry 100 mL single-necked flask, **12a-3** (400 mg, 1.24 mmol) and ethanol solution of methylamine (30%, 6 mL) was added. The mixture was stirred at rt for 16 h. The reaction solution was concentrated. The residue was diluted with water (10 mL) and extracted with EA (10 mL×3). The organic phase was collected, washed with saturated saline, dried over anhydrous sodium sulfate, filtered. The filtrate was concentrated to obtain **12a-4** (380 mg, yellow oil). LCMS(ESI): m/z 266 [M+H]⁺; RT=1.560 min (2.50 min). ¹H NMR(400 MHz, DMSO-*d₆*): δ 8.57 *(d, J*=4 Hz, 1H), 8.28 (d, *J*=2.4 Hz, 1H), 7.98 (d, *J*=8.8 Hz, 1H), 7.58-7.56 (m, 1H), 5.18 (s, 1H), 4.88 (d, *J*=3.2 Hz, 1H), 3.44-3.40 (m, 3H), 3.11 (s, 1H), 2.79 (d, *J=4.4* Hz, 3H), 1.40 (d, *J*=5.6 Hz, 9H),1.19-1.56 (m, 1H).

### Step 3: synthesis of N-methyl-5-(pyrrolidin-3-yloxy)picolinamide hydrochloride

To a solution of **12a-4** (380 mg, 1.18 mmol) in DCM (6 mL) was added dioxane/HCl (4.0 M, 6 mL) in an ice bath. The mixture was stirred at rt for 16 h. The reaction solution was concentrated to obtain **12a** (300 mg, yellow solid crude product), LCMS (ESI): m/z 220 [M+H]⁺; RT=0.603 min (2.5 min).

Intermediate **13a:** synthesis of 7-(1-Bromoethyl)-6-fluoropyrazolo[1,5-a]quinoxalin-4(5H)-one

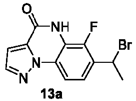

The synthesis method refers to that of intermediate **6a,** except that **10a-6** in step 5 of intermediate 10a was used instead of **6a-3** in step 4 of intermediate 6a. LCMS (ESI): m/z 310.0 [M-H]⁻; RT = 1.548 min (2.50 min).

Intermediate **14a:** synthesis of 7-(1-bromoethyl)-1-methyl-1,5-dihydro-4H-pyrazolo[4,3-c]quinolin-4-one

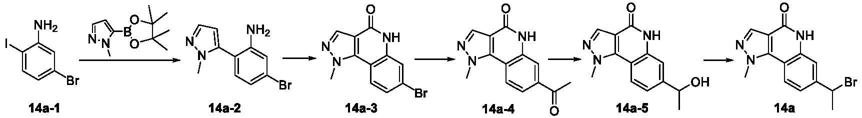

### Step 1: synthesis of 5-bromo-2-(1-methyl-1H-pyrazol-5-yl)aniline

To a three-necked vial containing 60 mL of 1,4-dioxane:water (3:1) were sequentially added **14a-1** (4.0 g, 13.3 mmol), potassium carbonate (3.7 g, 268 mmol), 1-methyl-5-(4,4,5,5-tetramethyl-1,3,2-dioxaborolan-2-yl)-1H-pyrazole (2.2 g, 10.7 mmol), and Pd(dppf)Cl₂ (972 mg, 1.34 mmol). The mixture was stirred for 4 h at 50°C protected by nitrogen. The reaction solution was diluted with water, extracted by EA. The organic phases were combined, washed with water and saturated saline water, dried over anhydrous sodium sulfate, and concentrated. The residue was purified by column chromatography (PE:EA=3:1) to obtain **14a-2** (2.3 g, yellow solid). Yield: 67.9 %, ¹H NMR (400 MHz, DMSO-*d₆*) : δ 7.49 (d, J=2.0 Hz, 1H), 6.97 (d, J=2.0 Hz, 1H), 6.92 (d, J=8.0 Hz, 1H), 6.76-6.73 (m, 1H), 6.26 (d, J=2.0 Hz, 1H), 5.21 (s, 2H), 3.63 (s, 3H).

### Step 2: synthesis of 7-bromo-1-methyl-1,5-dihydro-4H-pyrazolo[4,3-c]quinolin-4-one

The mixture of **14a-2** (2.25 g, 8.9 mmol) and carbonyldiimidazole (4.35 g, 26.80 mmol) in N-methylpyrrolidone (30 mL) was stirred overnight at 145°C. The reaction solution was diluted with water. A solid was precipitated, filtered, and dried to obtain **14a-3** (1.6 g, brownish-white solid) in the following yield: 45.1 %. LCMS(ESI): m/z 280 [M+H]⁺, RT=1.410 min (2.50 min). ¹H NMR (400 MHz, DMSO-*d₆*): δ 11.50 (s, 1H), 8.14-8.10 (m, 2H), 7.64 (d, J=2 Hz, 1H), 7.46-7.44 (m, 1H), 4.34 (s, 3H).

### Step 3: synthesis of 7-acetyl-1-methyl-1,5-dihydro-4H-pyrazolo[4,3-c]quinolin-4-one

A single-necked vial containing 10 mL of dioxane was charged with **14a-3** (600 mg, 2.16 mmol), (tributylstannyl)methanol (1.56 g, 4.32 mmol) and Pd(PPh₃)₂Cl₂ (227 mg, 0.32 mmol). The reaction was carried out at 95°C for 16 h. The reaction solution was filtered and concentrated. The residue was purified by column chromatography ( DCM : MeOH = 20 : 1) gave **14a- 4** (300 mg, yellow solid). Yield: 100 %, LCMS (ESI): m/z 242.2 [M+H]⁺; RT= 1.057 min (2.50 min). ¹H NMR (400 MHz, DMSO-*d₆*): δ 11.60 (s, 1H), 8.34 (d, J=8.4 Hz, 1H), 8.15 (s, 1H), 8.04 (d, J=1.6 Hz, 1H), 7.86-7.84 (m, 1H), 4.41 (s, 3H), 2.65 (s, 3H).

### Step 4: synthesis of 7-(1-hydroxyethyl)-1-methyl-1,5-dihydro-4H-pyrazolo[4,3-c]quinolin-4-one

To a solution of **14a-4** (300 mg, 1.24 mmol) in methanol (4 mL) was added sodium borohydride (57 mg, 1.50 mmol) at 0°C. The mixture was stirred at rt for 5 h. The mixture was quenched with saturated ammonium chloride (1 mL). pH was adjusted to neutral, diluted with water and EA (10 mL × 5). The organic phase was collected, washed with saturated saline, dried over anhydrous sodium sulfate, filtered. The filtrate was concentrated under reduced pressure to obtain **14a-5** (50 mg, yellow solid), yield: 16.5%, LCMS (ESI): m/z 244.1[M+H]⁺; RT= 0.860 min (2.50 min).

### Step 5: synthesis of 7-(1-bromoethyl)-1-methyl-1,5-dihydro-4H-pyrazolo[4,3-c]quinolin-4-one

A 100 mL single-necked vial containing 5 mL of DCM was charged with **14a-5** (50 mg, 0.20 mmol), triphenylphosphine (157 mg, 0.60 mmol) and carbon tetrabromide (133 mg, 0.40 mmol) and the reaction was carried out for 2 h at rt. The reaction solution was concentrated to obtain **14a** (30 mg, yellow solid). Yield: 47.7 %, LCMS (ESI): m/z 306.0 [M+H]⁺; RT= 1.477 min (2.50 min).

Intermediate **15a:** (1-methyl-4-oxo-4,5-dihydro-1H-pyrazolo[4,3-c]quinolin-7-yl)methyl methanesulfonate

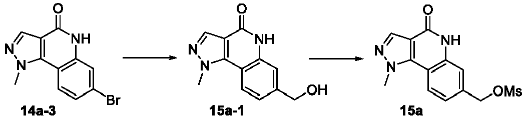

### Step 1: synthesis of 7-(hydroxymethyl)-1-methyl-1,5-dihydro-4H-pyrazolo[4,3-c]quinolin-4-one

To a 50mL dry single-necked flask was added **14a-3** (500 mg, 1.80 mmol), XPhos Pd G2 (142 mg, 0.18 mmol) in 1,4-dioxane (10 mL), (tributylstannyl)methanol (866 mg, 2.70 mmol) was added under nitrogen protection. The reaction was heated to 80°C for 16 h. The reaction was filtered and the filtrate was concentrated under reduced pressure. The residue was pulped with EA, filtered and the filter cake was collected to obtain **15a-1** (400 mg, black solid), yield: 97.05%, LCMS (ESI): m/z 230.1 [M+H]⁺; RT= 0.896 min (2.50 min).

### Step 2: synthesis of (1-methyl-4-oxo-4,5-dihydro-1H-pyrazolo[4,3-c]quinolin-7-yl)methyl methanesulfonate

To a dry single-necked flask was added **15a-2** (100 mg, 0.44 mmol), THF (5 mL), triethylamine (0.20 mL, 1.31 mmol) and methanesulfonyl chloride (0.06 mL, 0.87 mmol) sequentially. The reaction was carried out at rt for 16 h. The reaction was diluted with water (50 mL) and extracted by EA. The organic phase was washed with saturated saline, dried over anhydrous sodium sulfate, filtered. The filtrate was concentrated under reduced pressure to obtain crude **15a** (56 mg, yellow solid), crude, LCMS (ESI): m/z 308.1 [M+H]⁺; RT= 1.083 min (2.50 min).

Intermediate **16a:** 7-(1-Bromoethyl)-6-fluoro-1-methyl-1,5-dihydro-4H-pyrazolo[4,3-c]quinolin-4-one

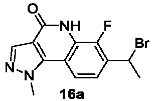

The synthesis method refers to that of Intermediate **14a,** except that Intermediate **11a-6** was used instead of Intermediate **14a-3,** LCMS (ESI): m/z 326.0 [M+H]⁺; RT= 1.393 min (2.50 min).

Intermediate **17a:** 6-Fluoro-7-(hydroxymethyl)-2-methyl-2,5-dihydro-4H-pyrazolo[4,3-c]quinolin-4-one

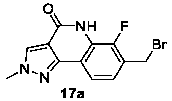

The synthesis method refers to that of Intermediate **11a,** except that (1-methyl-1H-pyrazol-3-yl)boronic acid was used instead of the intermediate 1-methyl-5-(4,4,5,5-tetramethyl-1,3,2-dioxaborolan-2-yl)-1H-pyrazole. LCMS (ESI): m/z 310.0 [M+H]⁺; RT= 1.327 min (2.50 min).

Intermediate **18a:** 7-(1-Bromoethyl)-6-fluoro-2-methyl-2,5-dihydro-4H-pyrazolo[4,3-c]quinolin-4-one

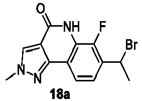

The synthesis method refers to that of intermediate 16a, LCMS (ESI): m/z 326.1 [M+H]⁺; RT= 0.599 min (2.50 min).

Intermediate **20a:** 7-(1-bromoethyl)-2-methyl-2,5-dihydro-4H-pyrazolo[3,4-c]quinolin-4-one

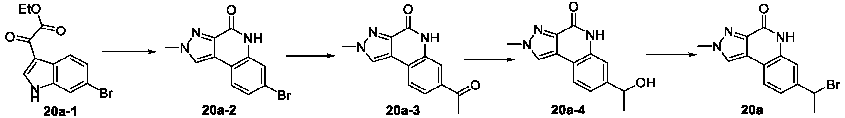

### Step 1: synthesis of 7-bromo-2-methyl-2,5-dihydro-4H-pyrazolo[3,4-c]quinolin-4-one

A dry single-necked flask was charged with **20a-1** (1.9 g, 6.4 mmol), anhydrous ethanol (30 mL), methylhydrazine sulphate (2.77 g, 19.2 mmol), and glacial acetic acid (0.5 mL). The reaction was carried out at 95°C for 24 h. The reaction solution was concentrated under reduced pressure, diluted with water and the pH was adjusted to 9, then filtered and the filter cake was dried under vacuum to obtain **20a-2** (1.3 g, white solid), Yield: 72.9%, LCMS (ESI): m/z 280.0 [M+H]⁺; RT=1.267 min (2.50 min). ¹H NMR (400 MHz, DMSO-*d*₆): δ 11.42 (s, 1H), 8.57 (s, 1H), 7.83 (d, J = 8.4 Hz, 1H), 7.51 (d, J = 1.6 Hz, 1H), 7.37-7.34 (m, 1H), 4.13 (s, 3H).Step 2: synthesis of 7-acetyl-2-methyl-2,5-dihydro-4H-pyrazolo[3,4-c]quinolin-4-one

A dry three-necked flask was charged with **20a-2** (400 mg, 1.44 mmol), 1,4-dioxane (10 mL) and Pd(PPh₃)₂Cl₂ (98 mg, 0.14 mmol). Under nitrogen, tributyl(1-ethoxyvinyl)tin (779 mg, 2.16 mmol) was added. The mixture was reacted at 95°C 16 h. Cooled to 50°C, hydrochloric acid solution (1.0 M, 4 mL) was slowly added and the resulting mixture was stirred for 1 h. Saturated potassium fluoride solution (10 mL) was added and stirred for another 1 h, filtered. The filtrate was extracted with EA (20 mL × 3). The EA phase was collected, washed with saturated saline, dried with anhydrous sodium sulfate, filtered, and the filtrate was concentrated. The residue was purified by column chromatography (DCM: methanol= 20:1) afforded **20a-3** (200 mg, yellow solid), yield: 57.7%, LCMS (ESI): m/z 242.1 [M+H]⁺; RT=0.877 min (2.50 min). ¹H NMR (400 MHz, DMSO-*d₆*): δ 11.50 (s, 1H), 8.77 (s, 1H), 8.00 (d, J = 8.0 Hz, 1H), 7.93 (d, J = 1.6 Hz, 1H), 7.80-7.78 (m, 1H), 4.15 (s, 3H), 2.61 (s, 3H).

### Step 3: synthesis of 7-(1-hydroxyethyl)-2-methyl-2,5-dihydro-4H-pyrazolo[3,4-c]quinolin-4-one

To a dry 100 mL single-necked flask ice bath was added **20a-3** (140 mg, 0.58 mmol), methanol (4 mL) and sodium borohydride (21 mg, 0.58 mmol). The mixture was stirred for 3 h at rt and diluted with water and DCM (15 mL × 4). The organic phase was collected, washed with saturated saline, dried over anhydrous sodium sulfate, filtered, and the filtrate was concentrated under reduced pressure to obtain the crude **20a-4** (110 mg, yellow solid), LCMS (ESI): m/z 244.1 [M+H]⁺; RT=1.045 min (2.50 min). ¹H NMR (400 MHz, DMSO-*d₆*): δ 11.28 (s, 1H), 8.59 (s, 1H), 7.79 (d, J = 7.6 Hz, 1H), 7.35 (s, 1H), 7.14 (d, J = 8.0 Hz, 1H), 5.26 (d, J = 4.0 Hz, 1H), 4.77-4.74 (m, 1H), 4.12 (s, 3H), 1.34 (d, J = 6.4 Hz, 3H).

### Step 4: synthesis of 7-(1-bromoethyl)-2-methyl-2,5-dihydro-4H-pyrazolo[3,4-c]quinolin-4-one

**20a-4** (100 mg, 0.41 mmol) and DCM (5 mL) were added sequentially in a dry three-necked flask. PBr₃ (333 mg, 1.23 mmol) was added slowly dropwise at 0 °C under N₂ protection. The reaction was carried out at rt for 3 h. The reaction solution was diluted with water (15 mL) and extracted with DCM (15 mL × 3). The organic phase was washed with saturated saline, dried over anhydrous sodium sulfate, filtered, and the filtrate was concentrated under reduced pressure to obtain the crude product 20a (100 mg, yellow solid), LCMS (ESI): m/z 306.1 [M+H]⁺; RT= 1.054 min (2.50 min).

Intermediate 21a: 7-(Bromomethyl)-2-methyl-2,5-dihydro-4H-pyrazolo[3,4-c]quinolin-4-one

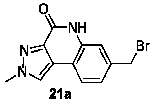

The synthesis method refers to that of intermediate **11a,** except that **20a-2** was used instead of **11a-6.** LCMS (ESI): m/z 292.0 [M+H]⁺; RT= 1.263 min (2.50 min).

Intermediate **22a:** 7-(Bromomethyl)-6-fluorothieno[2,3-c]quinolin-4(5H)-one

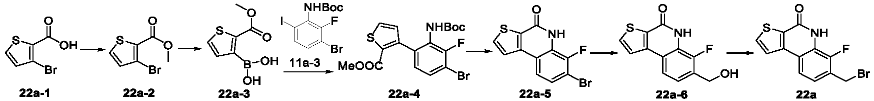

### Step 1: synthesis of methyl 3-bromothiophene-2-carboxylate

**22a-1** (1 g, 4.83 mmol) was dissolved in methanol (20 mL). Concentrated sulfuric acid (0.5 ml) was added dropwise. The mixture was refluxed at 80°C for 8 h. After cooling to rt, the mixture was concentrated, water (30 mL) was added, and the mixture was extracted three times with EA. The organic phases were combined and washed with saturated saline, dried over anhydrous sodium sulfate, concentrated and dried under vacuum obtain **22a-2** (950 mg, 4.30 mmol) as a white solid. Yield: 89%.

### Step 2: synthesis of (2-(methoxycarbonyl)thiophene-3-yl)boronic acid

**22a-2** (700 mg, 3.17 mmol), bis(pinacolato)diboron (1.61 g, 6.34 mmol), Pd(dppf)Cl₂ (695 mg, 0.76 mmol), potassium acetate (932 mg, 9.51 mmol) was added to dioxane (20 mL) in sequence. The mixture was stirred at 100°C for 8 h under nitrogen. The reaction solution was filtered with diatomaceous earth, and the filtrate was concentrated. The residue was purified by reversed phase column (acetonitrile: water (1‰ NH₄HCO₃)) to obtain **22a-3** (40 mg, 0.215 mmol), a white solid, yield: 6.8%. LCMS (ESI): m/z 186.9 [M+H]⁺; RT= 2.61 min (5.00 min).

### Step 3: synthesis of methyl 3-(4-bromo-2-((tert-butoxycarbonyl)amino)-3-fluorophenyl)thiophene-2-carboxylate

The mixture of **22a-3** (40 mg, 0.215 mmol), **11a-3** (98 mg, 0.236 mmol), Pd(dppf)Cl₂ (16 mg, 0.0215 mmol), and K₂CO₃ (89 mg, 0.645 mmol) in dioxane (8 mL) and water (2 mL) was stirred at 70°C for 1 h under nitrogen. The reaction solution was diluted with EA and washed twice with saturated saline, dried over anhydrous sodium sulfate, concentrated. The residue was purified by silica gel column (EA:PE=1:5) to obtain **22a-4** (71 mg, 0.165 mmol) as a white solid with a yield of 76.7%. LCMS (ESI): m/z 331.9 [M+H-100]⁺; RT= 1.85 min (3.00 min).

### Step 4: synthesis of 7-bromo-6-fluorothieno[2,3-c]quinolin-4(5-hydrogen)-one

**22a-4** (71 mg, 0.165 mmol) was dissolved in methanol (4 mL) at rt, and HCl/dioxane (2 mL, 4M) was added. The reaction solution was stirred at 45°C for 1 hour, and the reaction solution was concentrated and dried under vacuum to obtain **22a-5** (49 mg, 0.148 mmol), a white solid, with a yield of 90%. LCMS (ESI): m/z 299.9 [M+H]⁺; RT= 1.46 min (3.00 min)

Step 5: synthesis of 6-fluoro-7-(hydroxymethyl)thieno[2,3-c]quinolin-4(5-hydrogen)-one **22a-5** (49 mg, 0.148 mmol), (tributyltin)methanol (71 mg, 0.222 mmol), X-phos Pd G2 (11.6 mg, 0.0148 mmol) and dioxane (5 mL) was stirred at 100°C for 4 h under nitrogen. The mixture was concentrated, and the residue was purified by silica gel column (EA:PE=1:5) to obtain **22a-6** (31 mg, 0.124 mmol) as a white solid. Yield: 83.8%. LCMS (ESI): m/z 250.1 [M+H]⁺; RT= 0.95 min (3.00 min).

### Step 6: synthesis of 7-(Bromomethyl)-6-fluorothieno[2,3-c]quinolin-4(5H)-one

PBr₃ (168 mg, 0.622 mmol) was added to the solution of **22a-6** (31 mg, 0.124 mmol) in DCM (5 mL) at rt. The reaction solution was stirred at rt for 2 h and then concentrated to obtain crude product **22a** (50 mg, 100%), LCMS (ESI): m/z 311.9 [M+H]⁺; RT= 1.41 min (3.00 min).

Intermediate **23a:** 7-(Bromomethyl)-6-fluorothieno[3,4-c]quinolin-4(5H)-one

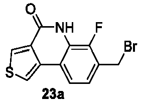

The synthesis method refers to that of intermediate **22a,** except that 4-bromothiophene-3-carboxylic acid was used instead of **22a-1.** LCMS (ESI): m/z 311.9[M+H]⁺; RT= 1.57 min (3.00 min).

Intermediate **24a:** 6-(piperazine-1-yl)nicotinate hydrochloride

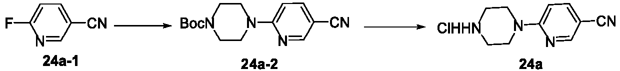

### Step 1: synthesis of tert-butyl 4-(5-cyanopyridin-2-yl)piperazine-1-carboxylate

In a 100 mL three-necked flask, add **24a-1** (500 mg, 4.09 mmol), 1-tert-butyloxycarbonylpiperazine (915.24 mg, 4.91 mmol) and acetonitrile (10 mL) in sequence, add DIEA (1.36 mL, 8.19 mmol) dropwise, and react at 65°C for 16 h under nitrogen. The solvent was removed and the residue was subjected to column chromatography (PE: EA = 5:1) to obtain **24a-2** (1.10 g, yellow solid), yield: 93.22%, LCMS (ESI): m/z 289.2 [M+H]⁺; RT=1.728 min (2.50 min).

### Step 2: synthesis of 6-(piperazine-1-yl)nicotinate hydrochloride

**24a-2** (1.10 g, 3.81 mmol) in DCM (15 mL) was added HCl/1,4-dioxane (1.5 mL) was added dropwise under ice bath. The mixture was stirred at rt for 2 h. The solvent was removed to obtain **24a** (850 mg, yellow oil), LCMS(ESI): m/z 189.2 [M+H]⁺; RT=0.352 min. ¹H NMR(400 MHz, DMSO-*d₆*): δ 9.54 (s, 2H), 8.55 (d, *J* = 2.4 Hz, 1H), 7.97-7.94 (m, 1H), 7.03 (d, *J* = 9.2 Hz, 1H), 3.93-3.91 (m, 4H), 3.16 (s, 4H).

Intermediate **25a:** 1-(2,4-difluorophenyl)piperazine

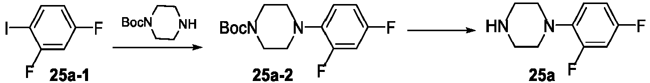

### Step 1: synthesis of tert-butyl 4-(2,4-difluorophenyl)piperazine-1-carboxylate

To a mixture of **25a-1** (2.00 g, 8.33 mmol), 1-tert-butyloxycarbonylpiperazine (2.33 g, 12.50 mmol), cesium carbonate (6.79 g, 20.83 mmol) in 1,4-dioxane (50 mL) was added Ruphos Pd G3 (698 mg, 0.83 mmol). The mixture was heat to 100°C under nitrogen for 16 h. Filter and remove the solvent. The residue was purified by column chromatography (PE: EA=20:1) to obtain **25a-2** (720 mg, black solid). Yield: 25.00%, LCMS (ESI): m/z 243.1 [M+H-56]⁺; RT=1.624 min (2.50 min).

### Step 2: synthesis of 1-(2,4-difluorophenyl)piperazine

**25a-2** (1.10 g, 3.81 mmol) in DCM (15 mL) was added HCl/dioxane (1.5 mL) in an ice bath and the mixture was stirred at rt for 2 h. The solvent was removed, and water and EA were added for extraction. The aqueous phase was adjusted to weakly basic with saturated sodium bicarbonate and extracted with EA. The EA phase was washed with saturated saline, dried over anhydrous sodium sulfate, and concentrated to obtain crude product **25a** (180 mg, black oil). LCMS (ESI): m/z 199.1 [M+H]⁺; RT=0.672 min (2.50 min). ¹H NMR(400 MHz, DMSO-*d₆*): δ 7.20-7.14 (m, 1H), 7.07-6.95 (m, 2H), 2.91-2.82 (m, 8H).

Intermediate **26a:** 7-(Bromomethyl)-2-methyl-2,5-dihydro-4H-pyrazolo[4,3-c]quinolin-4-one

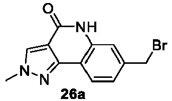

The synthesis method refers to that of intermediate **11a,** except that 5-bromo-2-iodoaniline was used instead of **11a-3.** LCMS (ESI): m/z 292.0 [M+H]⁺; RT= 1.234 min (2.50 min).

Intermediate **27a:** 7-(bromomethyl)-2-methyloxazolo[4,5-c]quinolin-4(5-hydrogen)-one

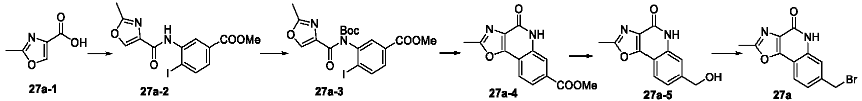

### Step 1: synthesis of methyl (4-iodo-3-(2-methyloxazole-4-carboxamide)benzoate

**27a-1** (340 mg, 2.68 mmol), methyl 3-amino-4-iodobenzoate (741 mg, 2.68 mmol), and T₃P (5.12 g, 8.04 mmol, 50% EA solution) was added to EA (20 mL). The mixture was stirred at 80°C for 16 h under nitrogen. After cooling to rt, water (20 mL) was added and stirred for 2 minutes before separation. The aqueous phase was extracted with EA, and the EA phase was washed with saturated sodium chloride water, dried over anhydrous sodium sulfate, concentrated and purified by silica gel column (EA:PE=3:1) to obtain **27a-2** (420 mg, 1.09 mmol), a light yellow solid, yield: 40.6%. LCMS (ESI): m/z 387.0 [M+H]⁺; RT= 1.74 min (3.0 min).

### Step 2: synthesis of methyl 3-(N-(tert-butoxycarbonyl)-2-methyloxazole-4-carboxamido)-4-iodobenzoate

To a 25 mL dry single-necked flask add **27a-2** (420 mg, 1.09 mmol), DCM (15 mL), di-tert-butyl dicarbonate (356 mg, 1.63 mmol) and DMAP (199 mg, 1.63 mmol) in sequence and the mixture was stirred at rt for 3 h. The reaction solution was concentrated under reduced pressure and the residue was purified by silica gel column (EA:PE=1:5) to obtain product **27a-3** (350 mg, 0.72 mmol) as white solid, yield: 66%. LCMS (ESI): m/z 387.0 [M+H-100]⁺; RT= 1.80 min (3.00 min).

### Step 3: synthesis of methyl 2-methyl-4-oxo-4,5-dihydrooxazolo[4,5-c]quinoline-7-carboxylate

**27a-3** (320 mg, 0.658 mmol), Pd(OAc)₂ (30 mg, 0.132 mmol), triphenylphosphine (34.6 mg, 0.132 mmol) and potassium carbonate (182 mg, 1.316 mmol) was added to DMF (5 mL), and the reaction was stirred at 100 °C for 1.5h under nitrogen in microwave reactor. The reaction solution is filtered and washed with EA. The filtrate is concentrated and purified by reversed phase column (acetonitrile: water (1‰HCOOH)). After freeze-drying, the product **27a-4** (140 mg, 0.534 mmol) was obtained as an off-white solid with a yield of 82.5%. LCMS (ESI): m/z 259.1 [M+H]⁺; RT= 1.28 min (3.00 min)

### Step 4: synthesis of 7-(hydroxymethyl)-2-methyloxazolo[4,5-c]quinolin-4(5H)-one

**27a-4** (140 mg, 0.534 mmol) was dissolved in THF (10 mL), and LiAlH₄ (1.08 mL, 1M/THF) was added dropwise in an ice bath and the mixture was stirred for 2 h. The reaction solution was quenched with methanol. The pH was adjusted to weakly acidic with trifluoroacetic acid, and concentrated under reduced pressure. The residue was dissolved in DMSO(5 mL) and purified by reversed phase column (acetonitrile: water (1‰ NH₄HCO₃)) to obtain **27a-5** (75 mg, 0.326 mmol), off-white solid, yield: 60%. LCMS (ESI): m/z 231.1 [M+H]⁺; RT= 1.07 min (3.00 min).

### Step 5: synthesis of 7-(bromomethyl)-2-methyloxazolo[4,5-c]quinolin-4(5-hydrogen)-one

**27a-5** (40 mg, 0.174 mmol) and DCM (5 mL) were added to a dry 25 mL single-necked flask at rt, and PBr₃ (235 mg, 0.869 mmol) was added dropwise. The reaction solution was stirred at rt for 2 h. The reaction solution was concentrated to obtain an oil (60 mg, crude product). LCMS (ESI): m/z 292.9 [M+H]⁺; RT= 1.39 min (3.00 min).

Intermediate **28a:** 7-(chloromethyl)-2-methylthiazolo[4,5-c]quinolin-4(5H)-one

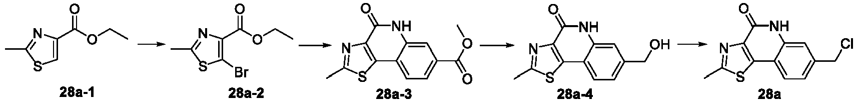

### Step 1: synthesis of ethyl 5-bromo-2-methylthiazole-4-carboxylate

**28a-1** (2.0 g, 11.7 mmol), N-bromosuccinimide (4.16 g, 23.4 mmol), and anhydrous acetonitrile (20 mL) were added to a 50 mL single-necked bottle. The mixture was heated to 90°C under Ar and stirred for 16 h, then cooled to rt, concentrated. The residue was purified by normal phase column (PE:EA=5:1) to obtain **28a-2** (1.3 g, yellow solid), yield: 45%. LCMS (ESI): m/z 250.1 [M+H]⁺; RT=1.30 min (3.0 min).

### Step 2: synthesis of methyl 2-methyl-4-oxo-4,5-dihydrothiazol[4,5-c]quinoline-7-carboxylate

**28a-2** (100 mg, 0.4 mmol), 2-amino-4-methoxycarbonylphenylboronic acid pinacol ester (166 mg, 0.6 mmol), Pd(dppf)Cl₂ (29 mg, 0.04 mmol) and potassium carbonate (166 mg, 1.2 mmol) was added to dioxane (5 mL) and water (1 mL). The mixture was heated to 80 °C with argon and stirred for 16 h, then cooled to rt. Water was added, and extracted with EA. The organic phase was dried over anhydrous sodium sulfate. The residue was purified by normal phase column (DCM: methanol = 10: 1) to obtain **28a-3** (50 mg, white solid), yield:45%. LCMS (ESI): m/z 275.1 [M+H]⁺; RT=1.03 min (3.0 min).

### Step 3: synthesis of 7-(hydroxymethyl)-2-methylthiazolo[4,5-c]quinolin-4(5H)-one

**28a-3** (50 mg, 0.18 mmol) and anhydrous THF (5 mL) were added to a 50 mL single-necked bottle, cooled to 0°C, and 1M LiAlH₄ solution (0.5 mL, 0.5 mmol) was slowly added dropwise. Stirred for 2 h, then quenched with methanol. 2 mL of trifluoroacetic acid was added dropwise, stirred for 10 min, concentrated. The residue was purified by reversed phase column (1%~30% acetonitrile/0.1% formic acid aqueous solution) to obtain **28a-4** (30 mg, white solid), yield: 45%. LCMS (ESI): m/z 247.1 [M+H]⁺; RT=0.54 min (3.0 min).

### Step 4: synthesis of 7-(chloromethyl)-2-methylthiazolo[4,5-c]quinolin-4(5H)-one

**28a-4** (30 mg, 0.12 mmol) and anhydrous DCM (5 mL) were added to a 50 mL single-necked bottle, cooled to 0°C, and thionyl chloride (1 mL) was slowly added dropwise. The mixture was stirred for 2 h and concentrated under reduced pressure to obtain crude product **28a** (30 mg, white solid). Yield: 100%. LCMS (ESI): m/z 265.1 [M+H]⁺; RT=1.41 min (3.0 min).

Intermediate **31a:** 6-fluoro-7-(bromomethyl)-[1,2,3]triazolo[1,5-a]quinoxaline-4(5H)-one

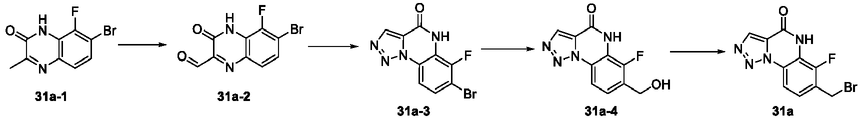

### Step 1: synthesis of 6-bromo-5-fluoro-3-oxo-3,4-dihydroquinoxaline-2-carboxaldehyde

**31a-1** (500 mg, 1.9 mmol), SeO₂ (328 mg, 2.9 mmol), and dioxane (10 mL) were added to a dry 50 mL single-necked flask at rt, heated to 100 °C and stirred overnight. The reaction solution was filtered, and the filtrate was concentrated to obtain **31a-2** (600 mg, oil). LCMS (ESI): m/z 271.2 [M+H]⁺; RT= 1.11 min (3.00 min).

### Step 2: synthesis of 7-bromo-6-fluoro-[1,2,3]triazolo[1,5-a]quinoxaline-4(5H)-one

**31a-2** (700 mg, 2.6 mmol), 4-methylbenzenesulfonyl hydrazide (579 mg, 3.1 mmol) were added to methanol (10 mL). The mixture was stirred at rt for 4 h. The filtrate was concentrated and the residue was purified by reversed phase preparative column (1%~50% acetonitrile) to obtain product **31a-3** (600 mg, red solid) with a yield of 71%. LCMS (ESI): m/z 282.8 [M+H]⁺; RT= 1.617 min (3.00 min).

### Step 3: synthesis of 6-fluoro-7-(hydroxymethyl)-[1,2,3]triazolo[1,5-a]quinoxaline-4(5H)-one

**31a-3** (200 mg, 0.71 mmol), (tributyltin)methanol (273 mg, 0.85 mmol), X-Phos Pd G2 (56 mg, 0.071 mmol) were added to dioxane (10 mL). The mixture was heated at 80°C overnight under nitrogen. The reaction solution was concentrated and purified by silica gel column (DCM: methanol = 30: 1) to obtain **31a-4** (100 mg, white solid), yield: 60%. LCMS (ESI): m/z 234. 9 [M+H]⁺; RT= 1.317 min (3.00 min).

### Step 4: synthesis of 6-fluoro-7-(bromomethyl)-[1,2,3]triazolo[1,5-a]quinoxaline-4(5H)-one

To a solution of **31a-4** (20 mg, 0.085 mol) in dioxane (3 mL) were added sequentially PBr₃ (69 mg, 0.25 mmol) dropwise under ice bath. The mixture was stirred at rt for 2 h, and the filtrate was concentrated to obtain a crude product (40 mg, oil). LCMS (ESI): m/z 296. 9 [M+H]⁺;RT= 1.45 min (3.00 min).

Intermediate **32a:** 7-(1-Bromoethyl)-6-fluoro-[1,2,3]triazolo[1,5-a]quinoxaline-4(5H)-one

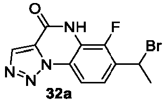

The synthesis method refers to that of intermediate 31a, except that in the Stille coupling reaction, tributyl(1-ethoxyethylene)tin and PdCl₂(PPh₃)₂ were used. LCMS (ESI): m/z 310.9 [M+H]⁺; RT= 1.55 min (3.00 min).

Intermediate **33a:** 8-(chloromethyl)imidazo[1,2-c]quinazolin-5(6H)-one

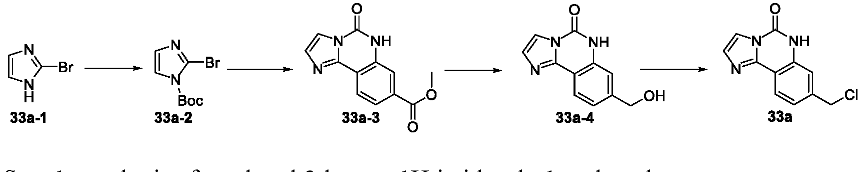

### Step 1: synthesis of tert-butyl 2-bromo-1H-imidazole-1-carboxylate

**33a-1** (200 mg, 1.36 mmol), di-tert-butyl dicarbonate (445 mg, 2.04 mmol), triethylamine (412 mg, 4.08 mmol) in DMF (5 mL) were stirred at rt for 2 h under argon. The reaction solution was added water and extracted with EA. The organic phase was dried over anhydrous sodium sulfate, concentrated. The residue was purified by normal phase column (PE:EA=5:1) to obtain **33a-2** (250 mg, white solid), yield: 74%. LCMS (ESI): m/z 146.9 [M+H-100]⁺; RT=1.58 min (3.0 min).

### Step 2: synthesis of methyl 5-carbonyl-5,6-dihydroimidazo[1,2-c]quinazoline-8-carboxylate

**33a-2** (100 mg, 0.4 mmol), methyl 3-amino-4-(4,4,5,5-tetramethyl-1,3,2-dioxaborolan-2-yl)benzoate (166 mg, 0.6 mmol), Pd(dppf)Cl₂ (29 mg, 0.04 mmol) and potassium carbonate (166 mg, 1.2 mmol) in dioxane (5 mL) and water (1 mL) were stirred at 80 °C for 16 h under argon. The mixture was cooled to rt and extracted with EA. The organic phase was dried over anhydrous sodium sulfate and the residue was purified by normal phase column (DCM: methanol = 10: 1) to obtain **33a-3** (50 mg, white solid) with a yield of 45%. LCMS (ESI): m/z 244.1 [M+H]⁺; RT=1.22 min (3.0 min).

### Step 3: synthesis of 8-(hydroxymethyl)imidazo[1,2-c]quinazolin-5(6H)-one

**33a-3** (50 mg, 0.20 mmol) and anhydrous THF (5 mL) were added to a 50 mL single-necked bottle, cooled to 0°C, and 1M LiAlH₄ solution (0.5 mL, 0.5 mmol) was slowly added dropwise. The mixture was stirred for 2 h, quenched with methanol, and 2 mL of trifluoroacetic acid was added dropwise. Stirred for 10 min, concentrated, and the residue was purified by reversed phase column (1%~30% acetonitrile/0.1% NH₄HCO₃) to obtain **33a-4** (20 mg, white solid), yield: 47%. LCMS (ESI): m/z 216.1 [M+H]⁺; RT=0.99 min (3.0 min).

### Step 4: synthesis of 8-(chloromethyl)imidazo[1,2-c]quinazolin-5(6H)-one

**33a-4** (20 mg, 0.09 mmol) and DCM (5 mL) were added into a single-necked vial, cooled to 0°C, and thionyl chloride (1 mL) was slowly added dropwise at 0 °C. The mixture was stirred for 2 h and concentrated under reduced pressure to obtain crude **33a** (20 mg, white solid). LCMS (ESI): m/z 234.1 [M+H]⁺; RT=1.28 min (3.0 min).Intermediate **34a:** synthesis of N-ethyl-6-fluoro-5-(piperazine-1-yl)picolinamide hydrochloride

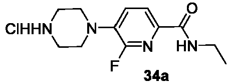

The synthesis method refers to that of intermediate 2a, except that ethylamine is used instead of methylamine hydrochloride in the condensation step. LCMS (ESI): m/z 253.2 [M+H]⁺; RT=0.527 min (2.5 min).

Intermediate **35a:** (R)-6-Fluoro-5-(piperazine-1-yl)-N-(tetrahydrofuran-3-yl)picolinamide hydrochloride

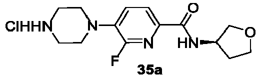

The synthesis method refers to that of intermediate **2a,** except that (R)-tetrahydrofuran-3-amine is used instead of methylamine hydrochloride in the condensation step. LCMS (ESI): m/z 395.2 [M+H]⁺; RT= 1.582 min (2.50 min).

Intermediate **36a:** 6-chloro-N-methyl-5-(piperazin-1-yl)picolinamide hydrochloride

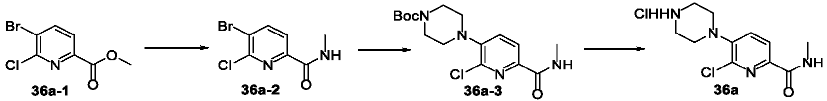

### Step 1: synthesis of 5-bromo-6-chloro-N-methylpicolinamide

**36a-1** (1.00 g, 3.99 mmol) in ethanol solution of methylamine (33% wt, 10 mL) was stirred at rt for 16 h. The reaction solution was concentrated under reduced pressure to obtain **36a-2** (0.94 g, yellow oil), yield: 94.37%, LCMS (ESI): m/z 250.9 [M+H]⁺; RT=1.448 min (2.50 min).

### Step 2: synthesis of tert-butyl 4-(2-chloro-6-(methylcarbamoyl)pyridin-3-yl)piperazine-1-carboxylate

**36a-2** (620 mg, 2.49 mmol), tert-butyl piperazine-1-carboxylate (370 mg, 1.99 mmol), BINAP (155 mg, 0.25 mmol), cesium carbonate (2020 mg, 6.21 mmol) and palladium acetate (56 mg, 0.25 mmol) were added to toluene (15 mL). The mixture was heated to 100°C under nitrogen. The mixture was filtered. the filtrate was concentrated. The residue was purified by Silica gel column chromatography (PE: EA = 1: 1) to obtain **36a-3** (180 mg, yellow oil). Yield: 20.41%. LCMS (ESI): m/z 355.1 [M+H]⁺; RT = 1.679 min (2.50 min). ¹H NMR (400 MHz, DMSO-d6): δ 8.46 (d, J = 4.8 Hz, 1H), 7.95 (d, J = 8.4 Hz, 1H), 7.68 (d, J = 8.4 Hz, 1H), 3.50 (s, 4H), 3.06-3.04 (m, 4H), 2.80 (d, J = 4.8 Hz, 3H), 1.43 (s, 9H).

### Step 3: synthesis of 6-chloro-N-methyl-5-(piperazin-1-yl)picolinamide hydrochloride

**36a-3** (180 mg, 0.51 mmol) in DCM (2 mL) was added HCl/dioxane (4.0 M, 2 mL). The mixture was stirred at rt for 2 h. The reaction solution was concentrated under reduced pressure to afford **36a** (140 mg, yellow solid). Yield: 94.59%, LCMS (ESI): m/z 255.1 [M+H]⁺; RT = 0.336 min & 0.461 min (2.50 min). ¹H NMR (400 MHz, DMSO-*d₆*): δ 9.40 (s, 2H), 8.50 (d, J = 4.4 Hz, 1H), 7.97 (d, J = 8.4 Hz, 1H), 7.76 (d, J = 8.4 Hz, 1H), 3.34-3.32 (m, 4H), 3.25 (m, 4H), 2.80 (d, J = 4.8 Hz, 3H).

Intermediate **37a:** N,6-dimethyl-5-(piperazin-1-yl)picolinamide hydrochloride

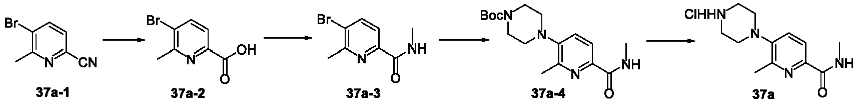

### Step 1: synthesis of 5-bromo-6-methylpicolinic acid

**37a-1** (500 mg, 2.54 mmol), methanol (6 mL), water (3 mL) and sodium hydroxide (507 mg, 12.69 mmol) were added to a dry flask in sequence. The mixture was reacted at 70°C for 1 h. The reaction solution was concentrated. Dilute with water (10 mL), adjust to pH = 4 with 3 M hydrochloric acid, filter, and collect the filter cake to obtain **37a-2** (300 mg, white solid), yield: 54.72%, LCMS (ESI): m/z 218.0 [M+H]⁺; RT = 1.208 min (2.50 min). ¹H NMR (400 MHz, DMSO-*d₆*): δ 8.12 (d, J = 8.0 Hz, 1H), 7.78 (d, J = 8.4 Hz, 1H), 2.67 (s, 3H).

### Step 2: synthesis of 5-bromo-N,6-dimethylpicolinamide

To **37a-2** (300 mg, 1.39 mmol) in DMF (3 mL) and DIEA (0.92 mL, 5.55 mmol) was added HATU (792 mg, 2.08 mmol) and methylamine THF solution (2.0 M, 1.39 mL, 2.78 mmol). The mixture was reacted at rt for 1 h. Dilute with water (30 mL) and extract with EA (10 mL×2). The organic phase was washed with saturated saline, dry with anhydrous sodium sulfate, filter, and concentrate. The residue was purified by preparative plate (PE: EA = 3:2) to obtain **37a-3** (215 mg, yellow solid), yield: 67.59%, LCMS (ESI): m/z 231.0 [M+H]⁺; RT = 1.427 min (2.50 min). ¹H NMR (400 MHz, DMSO-*d₆*): δ 8.67 (d, J = 4.0 Hz, 1H), 8.18 (d, J = 8.4 Hz, 1H), 7.75 (d, J = 8.4 Hz, 1H), 2.82 (d, J = 4.8 Hz, 3H), 2.65 (s, 3H).

### Step 3: synthesis of tert-butyl 4-(2-methyl-6-(methylcarbamoyl)pyridin-3-yl)piperazine-1-carboxylate

37a-3 (200 mg, 0.87 mmol), tert-butyl piperazine-1-carboxylate (179 mg, 0.96 mmol), BINAP(54 mg, 0.09 mmol), cesium carbonate (711 mg, 2.18 mmol) and palladium acetate (20 mg, 0.09 mmol) in toluene (8 mL) was heat to 100 °C under nitrogen for 16 h. Filter the reaction solution, collect the filtrate, and concentrate. The residue was purified by preparative plate (PE: EA = 1:1) to obtain **37a-4** (160 mg, yellow solid), yield: 54.80%, LCMS (ESI): m/z 335.1 [M+H]⁺; RT = 1.623 min (2.50 min). ¹H NMR (400 MHz, DMSO-*d₆*): δ 8.44 (d, J = 4.8 Hz, 1H), 7.80 (d, J = 8.4 Hz, 1H), 7.49 (d, J = 8.4 Hz, 1H), 3.49 (s, 4H), 2.89-2.87 (m, 4H), 2.81 (d, J = 4.8 Hz, 3H), 2.51 (s, 3H), 1.43 (s, 9H).

### Step 4: synthesis of N,6-dimethyl-5-(piperazin-1-yl)picolinamide hydrochloride

**37a-4** (160 mg, 0.48 mmol) in DCM (2 mL) was added HCl/dioxane (4.0 M, 2 mL) and reacted at rt for 4 h. The reaction solution was concentrated to afford 37a (129 mg, yellow solid). Yield: 99.58%, LCMS (ESI): m/z 235.2 [M+H]⁺; RT = 0.340 min & 0.450 min (2.50 min). ¹H NMR (400 MHz, DMSO-*d₆*): δ 9.44 (s, 2H), 8.56 (d, J = 4.8 Hz, 1H), 7.88 (d, J = 8.0 Hz, 1H), 7.61 (d, J = 8.4 Hz, 1H), 3.25-3.16 (m, 8H), 2.82 (d, J = 4.4 Hz, 3H), 2.54 (s, 3H).

Intermediate **38a:** synthesis of N-cyclopropyl-6-fluoro-5-(piperazine-1-yl)picolinamide hydrochloride

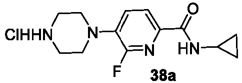

The synthesis method refers to that of intermediate **2a,** except that cyclopropylamine is used instead of methylamine hydrochloride in the condensation step. LCMS (ESI): m/z 264.2 [M+H]⁺; RT=0.958 min (2.5 min).

Intermediate **40a:** 8-(Bromomethyl)-2-methylimidazo[1,2-c]quinazolin-5(6H)-one

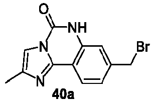

The synthesis method refers to that of intermediate **33a,** except that 2-bromo-4-methyl-1H-imidazole is used as the starting material and PBr₃ is used as the reaction reagent in step 4. LCMS (ESI): m/z 292.0 [M+H]⁺; RT= 1.25 min (3.00 min).

Intermediate **41a:** synthesis of 8-(bromomethyl)-7-fluoro-dihydroimidazo[1,2-c]quinazolin-5(3H)-one

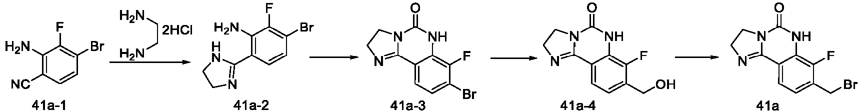

### Step 1: synthesis of 3-bromo-6-(4,5-dihydro-1H-imidazol-2-yl)-2-fluoroaniline

**41a-1** (500 mg, 2.33 mmol) in methanol (20 mL) was added ethylenediamine dihydrochloride (3.1 g, 23.3 mmol) and sodium carbonate (3.7 g, 35 mmol). The mixture was heated under reflux for 72 h and concentrated. Add water (20 mL), filter, dissolve the solid in DMSO (5 mL), and purify by reverse phase column (acetonitrile: water) to obtain **41a-2** (130 mg, 0.504 mmol), white solid, yield 21.6%. LCMS (ESI): m/z 260.0 [M+H]⁺; RT= 0.75 min (3.00 min).

### Step 2: synthesis of 8-bromo-7-fluoro-2,6-dihydroimidazo[1,2-c]quinazolin-5(3H)-one

**41a-2** (50 mg, 0.194 mmol) in DMF (5 mL) was added N,N'-carbonyldiimidazole (94 mg, 0.582 mmol) and DMAP (24 mg, 0.194 mmol). The mixture was stirred at 100°C for 3h. After cooled to rt, the solid was collected by filtration, washed with EA, and dried in vacuo to obtain 41a-3 (41 mg, 0.144 mmol), white solid, yield: 74.2%. LCMS (ESI): m/z 286.0 [M+H]⁺; RT= 1.22 min (3.00 min).

### Step 3: synthesis of 7-fluoro-8-(hydroxymethyl)-2,6-dihydroimidazo[1,2-c]quinazolin-5(3H)-one

The mixture of **41a-3** (41 mg, 0.144 mmol), (tributyltin)methanol (69 mg, 0.216 mmol) and X-Phos Pd G2 (11.3 mg, 0.0144 mmol) in dioxane (5 mL) was stirred at 100°C for 6 h under nitrogen, then concentrated. The residue was purified by silica gel column (methanol: DCM = 1:10) to obtain **41a-4** (29 mg, 0.123 mmol), white solid, yield: 85.7%. LCMS (ESI): m/z 236.1 [M+H]⁺; RT = 0.91 min (3.00 min).

### Step 4: synthesis of 8-(bromomethyl)-7-fluoro-dihydroimidazo[1,2-c]quinazolin-5(3H)-one

**41a-4** (29 mg, 0.123 mmol) in DCM (8 mL) was added PBr₃ (0.5 mL). The mixture was stirred for 2 h at rt. The reaction solution was concentrated to remove excess PBr₃ and dried to obtain crude product **41a** (35 mg). LCMS (ESI): m/z 298.2 [M+H]⁺; RT= 1.23 min (3.00 min).

Intermediate **42a:** synthesis of 8-(bromomethyl)-2,6-dihydroimidazo[1,2-c]quinazolin-5(3H)-one

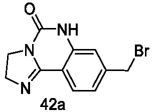

The synthesis method refers to that of intermediate **41a,** except that 2-amino-4-bromobenzonitrile is used as the starting material. LCMS (ESI): m/z 280.0 [M+H]⁺; RT= 0.580 min (2.50 min).

Intermediate **43a:** synthesis of 8-(Bromomethyl)-7-methyl-dihydroimidazo[1,2-c]quinazolin-5(3H)-one

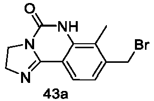

The synthesis method refers to that of intermediate **41a,** except that 2-amino-4-bromo-3-methylbenzonitrile is used as the starting material. LCMS (ESI): m/z 294.1 [M+H]⁺; RT= 1.30 min (3.00 min).

Intermediate **44a:** synthesis of N-cyclopropyl-6-methyl-5-(piperazine-1-yl)picolinamide hydrochloride

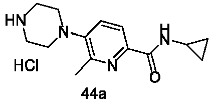

The synthesis method refers to that of intermediate **37a,** except that cyclopropylamine is used as the starting material. LCMS (ESI): m/z 261.1 [M+H]⁺; RT=1.05 min (3.0 min).

Intermediate **45a:** synthesis of 8-(bromomethyl)-7-chloro-dihydroimidazo[1,2-c]quinazolin-5(3H)-one and **46a:** 8-(bromomethyl)-9-chloro-2,6-dihydroimidazo[1,2-c]quinazolin-5(3H)-one

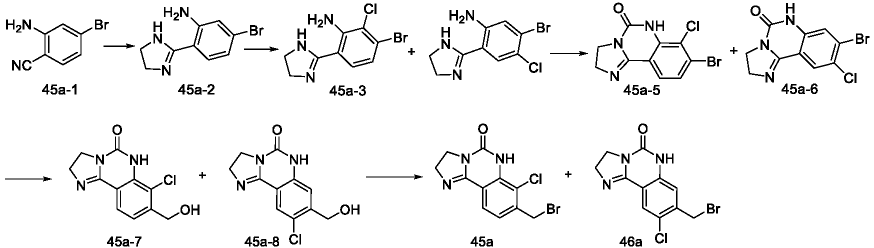

### Step 1: synthesis of 5-bromo-2-(4,5-dihydro-1H-imidazol-2-yl)aniline

The mixture of **45a-1** (5 g, 25.5 mmol), phosphorus pentasulfide (487 mg, 2.55 mmol) and ethylenediamine (10 mL) were reacted at 100 °C for 4 h. Water (100 mL) was added to the reaction solution, then filtered. The filter cake was washed with water, and dried to obtain **45a-2** (1.5 g, light yellow solid), yield: 82%. LCMS (ESI): m/z 240.1 [M+H]⁺; RT= 0.98 min (3.00 min).

### Step 2: synthesis of 3-bromo-2-chloro-6-(4,5-dihydro-1H-imidazol-2-yl)aniline

In a dry 50 mL single-necked flask, **45a-2** (500 mg, 2.09 mmol), N-chlorosuccinimide (335 mg, 2.5 mmol), *p*-toluenesulfonic acid (40 mg, 0.21 mmol), and DMF (10 mL) were added in sequence. The mixture was reacted at 90°C for 6 h. Water (30 mL) was added and the mixture was extracted with EA (40 mL). The organic phase was washed with saturated saline (20 mL), dried over anhydrous sodium sulfate, and concentrated. The residue was purified by silica gel column (PE:EA=3:1) to obtain a mixture of **45a-3** and **45a-4** (100 mg, yellow solid), yield: 17%. LCMS (ESI): m/z 274.0 [M+H]⁺; RT= 1.19&1.21 min (3.00 min).

### Step 3: synthesis of 8-bromo-7-chloro-2,6-dihydroimidazo[1,2-c]quinazolin-5(3H)-one

**45a-3** and **45a-4** (mixture, 100 mg, 0.37 mmol), N,N'-carbonyldiimidazole (118 mg, 0.73 mmol), DMAP (45 mg, 0.37 mmol), and DMF (3 mL) were added to a single-necked flask in sequence and heated to 100°C for 2 h. Water (10 mL) was added to the reaction solution, filtered. The filter cake was washed with water, and dried to obtain a mixture of **45a-5** and **45a-6** (90 mg, yellow solid), yield: 82%. LCMS (ESI): m/z 299.9 [M+H]⁺; RT= 1.29&1.32 min (3.00 min).

### Step 4: synthesis of 7-chloro-8-(hydroxymethyl)-2,6-dihydroimidazo[1,2-c]quinazolin-5(3H)-one

In a dry flask, a mixture of **45a-5** and **45a-6** (90 mg, 0.3 mmol), (tributyltin)methanol (192 mg, 0.6 mmol), X-Phos Pd G2 (23 mg, 0.03 mmol), and dioxane (5 mL) were added in sequence. The mixture was stirred at 100°C for 6 h under nitrogen, and concentrated. The residue was purified by reversed phase preparative column (1%~50% acetonitrile) to obtain **45a-7** (15 mg, yellow solid) and **45a-8** (25 mg, yellow solid).

45a-7: LCMS (ESI): m/z 252.1 [M+H]⁺; RT= 0.95 min (3.00 min). ¹H NMR (600 MHz, DMSO-d₆): δ 7.80 (d, J = 8.0 Hz, 1H), 7.30 (d, J = 8.0 Hz, 1H), 3.96 (m, 2H), 3.87 (m, 2H).

### Step 5: synthesis of 8-(bromomethyl)-7-chloro-dihydroimidazo[1,2-c]quinazolin-5(3H)-one

**45a-7** (15 mg, 0.06 mmol) in dioxane (3 mL) were added PBr₃ (48 mg, 0.18 mmol) in an ice bath. The mixture was stirred at rt for 2 h and concentrated to obtain crude product **45a** (20 mg, yellow solid). LCMS (ESI): m/z 314.0&316.0 [M+H]⁺, RT=1.28 min (3.00 min).

### Step 6: synthesis of 8-(bromomethyl)-9-chloro-2,6-dihydroimidazo[1,2-c]quinazolin-5(3H)-one

**45a-8** (25 mg, 0.1 mmol) in dioxane (3 mL) was added PBr₃ (81 mg, 0.3 mmol) dropwise in an ice bath. The mixture was stirred at rt for 2 h and concentrated to obtain **46a** (30 mg, yellow solid). LCMS (ESI): m/z 314.0&316.0 [M+H]⁺; RT=1.32 min (3.00 min).

Intermediate **47a:** synthesis of 8-(bromomethyl)-7-fluoro-2,2-dimethyl-2,6-dihydroimidazo[1,2-c]quinazolin-5(3H)-one

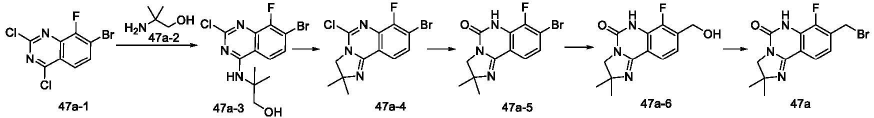

### Step 1: synthesis of 2-((7-bromo-2-chloro-8-fluoroquinazolin-4-yl)amino)-2-methylpropan-1-ol

**47a-1** (300 mg, 1.01 mmol) in THF (5 mL) was added **47a-2** (108 mg, 1.21 mmol) and triethylamine (306 mg, 3.03 mmol). The mixture was stirred at rt for 2 h. The reaction solution was diluted with water (15 mL), extracted three times with EA (20 mL). The organic phase was washed with saline, dried over anhydrous sodium sulfate, and concentrated to obtain **47a-3** (320 mg 0.92 mmol), white solid, yield: 91%. LCMS (ESI): m/z 349.9 [M+H]⁺; RT=1.62 min (3.0 min).

### Step 2: synthesis of 8-bromo-5-chloro-7-fluoro-2,2-dimethyl-2,3-dihydroimidazo[1,2-c]quinazoline

**47a-3** (320 mg 0.92 mmol), phosphorus oxychloride (1 mL) was added into toluene (10 mL) in an ice bath. The mixture was reacted at rt for 3 h. The pH value was adjusted to 7-8 with sodium carbonate aqueous solution. The mixture was extracted with EA, dried over anhydrous sodium sulfate, concentrated, and purified by silica gel column (PE:EA=1:1) to obtain **47a-4** (215 mg, 0.651 mmol) as a white solid, with a yield of 70.8%. LCMS (ESI): m/z 331.9 [M+H]⁺; RT=1.62 min (3.0 min).

### Step 3: synthesis of 8-bromo-7-fluoro-2,2-dimethyl-2,6-dihydroimidazo[1,2-c]quinazolin-5(3H)-one

**47a-4** (215 mg, 0.651 mmol) in acetic acid (5 mL) was heated to 110°C for 2 h. The solution was concentrated to remove acetic acid. Water (15 mL) was added to the residue. The pH was adjusted to 7-8 with saturated sodium bicarbonate solution. The mixture was extracted with EA (20 mL) three times. The EA phase was washed with saline, dried over anhydrous sodium sulfate, and concentrated to obtain **47a-5** (180 mg, 0.577 mmol), white solid, yield: 93.8%. LCMS (ESI): m/z 314.0 [M+H]⁺; RT=1.32 min (3.0 min).

### Step 4: synthesis of 7-Fluoro-8-(hydroxymethyl)-2,2-dimethyl-2,6-dihydroimidazo[1,2-c]quinazolin-5(3H)-one

**47a-5** (80 mg, 0.256 mmol), (tributyltin)methanol (165 mg, 0.513 mmol), X-Phos Pd G2 (20 mg, 0.0256 mmol) were added to anhydrous dioxane (5 mL). The mixture was heated to 110°C under argon, stirred for 2 h, then concentrated. The residue was purified by column chromatography (DCM: methanol = 10:1) to obtain **47a-6** (48 mg, 0.154 mmol) as a white solid, yield: 60%. LCMS (ESI): m/z 264.2 [M+H]⁺; RT=1.02 min (3.0 min).

### Step 5: synthesis of 8-(bromomethyl)-7-fluoro-2,2-dimethyl-2,6-dihydroimidazo[1,2-c]quinazolin-5(3H)-one

**47a-6** (48 mg, 0.154 mmol) was in DCM (10 mL) was added PBr₃ (0.5 mL) at rt. The mixture was stirred for 2 h. The reaction solution was concentrated to remove the solvent and excess PBr₃. The crude product **47a** (60 mg) was dried in vacuo. LCMS (ESI): m/z 326.2 [M+H]⁺; RT=1.38 min (3.0 min).

Intermediates **48a-56a, 59a-60a:** synthesized in the same way as **41a** or **47a,** except that the raw material intermediates in the table below are used as starting materials instead of ethylenediamine or **47a-2**

| Inter medi ates | Structure | Name | LCMS (ESI): [M+H]⁺ | Raw material |
|---|---|---|---|---|
| 48a | 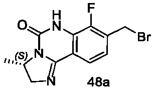 | (S)-8-(Bromomethyl)-7-fluoro-3-methyl-2,6-dihydroimidazo[1,2-c]quinazolin-5(3H)-one | 312.0 | (R)-2-Aminopropan-1-ol instead of 47a-2 |
| 49a | 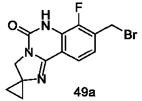 | 8'-(Bromomethyl)-7'-fluoro-3'-H-spiro[cyclopropane-1,2'-imidazo[1,2-c]quinazolin]-5'(6'H)-one | 324.1 | 1-Aminocyclopropan emethanol instead of 47a-2 |
| 50a | 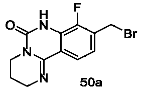 | 9-(Bromomethyl)-8-fluoro-2,3,4,7-tetrahydro-6H-pyrimido[1,2-c]quinazolin-6-one | 314.0 | Propylenediamine instead of ethylenediamine |
| 51a | 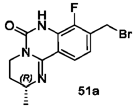 | (R)-9-(Bromomethyl)-8-fluoro-2-methyl-2,3,4,7-tetrahydro-6H-pyrimido[1,2-c]quinazolin-6-one | 326.0 | (R)-2-Aminobutan-1-ol instead of 47a-2 |
| 52a | 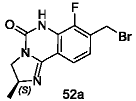 | (S)-8-(Bromomethyl)-7-fluoro-2-methyl-2,6-dihydroimidazo[1,2-c]quinazolin-5(3H)-one | 312.0 | (S)-2-Aminopropan-1-ol instead of 47a-2 |
| 53a | 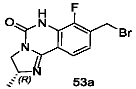 | (R)-8-(bromomethyl)-7-fluoro-2-methyl-2,6-dihydroimidazo[1,2-c]quinazolin-5(3H)-one | 312.1 | (R)-2-Aminopropan-1-ol instead of 47a-2 |
| 54a | 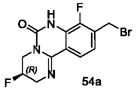 | (R)-9-(Bromomethyl)-3,8-difluoro-2,3,4,7-tetrahydro-6-pyrimido[1,2-c]quinazolin-6-one | 330.0 | (R)-3-amino-2-fluoropropan -1-ol instead of 47a-2 |
| 55a | 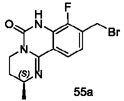 | (S)-9-(Bromomethyl)-8-fluoro-2-methyl-2,3,4,7-tetrahydro-6H-pyrimido[1,2-c]quinazolin-6-one | 326.0 | (S)-2-Aminobutan-1-ol instead of 47a-2 |
| 56a | 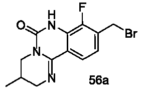 | 9-(Bromomethyl)-8-fluoro-3-methyl-2,3,4,7-tetrahydro-6-pyrimido[1,2-c]quinazolin-6-one | 326.0 | 2-Methylpropane-1,3-diamine substituted for ethylenediamine |
| 59a | 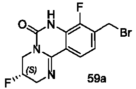 | (S)-9-(Bromomethyl)-3,8-difluoro-2,3,4,7-tetrahydro-6-pyrimido[1,2-c]quinazolin-6-one | 330.0 | (S)-3-amino-2-fluoropropan-1 -ol instead of 47a-2 |
| 60a | 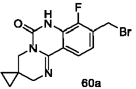 | 9'-(bromomethyl)-8'-fluoro-2',7'-dihydro-4'H,6'H-spiro[cyclopropane-1,3'-pyrimido[1,2-c]quinazolin]-6'-one | 338.0 | (1-(Aminomethyl)cyc lopropyl) methanol instead of 47a-2 |

Intermediate **57a:** 9-(chloromethyl)-3,3,8-trifluoro-2,3,4,7-tetrahydro-6-pyrimido[1,2-c]quinazolin-6-one

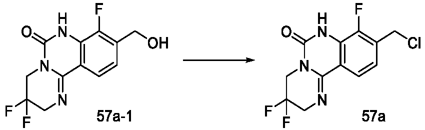

**57a-1** (20 mg, 0.07 mmol, synthesized as **47a-6,** except that 3-amino-2,2-difluoropropan-1-ol was used instead of **47a-2**), anhydrous DCM (10 mL) was added to a 50 mL single-necked vial. The mixture was cooled to 0°C, and SOCl₂ (1 mL) was slowly added with stirring for 2 h. Concentration was carried out to obtain the crude **57a** (25 mg, white solid), yield: 100%. LCMS (ESI): m/z 304.1 [M+H]⁺; RT=1.50 min (3.0 min).

Intermediate **58a:** 9-(chloromethyl)-8-fluoro-4-methyl-2,3,4,7-tetrahydro-6-pyrimido[1,2-c]quinazolin-6-one

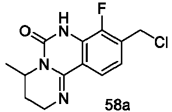

The synthesis method refers to that of **57a,** except that **47a-2** was replaced by 4-amino-2-butanol. LCMS (ESI): m/z 282.1 [M+H]⁺; RT=1.28 min (3.0 min).

Intermediate **61a:** N-ethyl-6-methyl-5-(piperazin-1-yl)pyridine carboxamide hydrochloride

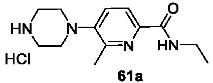

The synthesis method refers to that of **37a,** except that ethylamine was used instead of methylamine, LCMS (ESI): m/z 249.3 [M+H]⁺; RT= 1.0 min (3.00 min).

Intermediate **62a:** 6-chloro-N-ethyl-5-(piperazin-1-yl)pyridine carboxamide hydrochloride

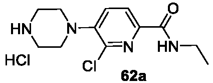

The synthesis method refers to that of **36a,** except that ethylamine was used instead of methylamine, LCMS (ESI): m/z 269.3 [M+H]⁺; RT= 1.2 min (3.00 min).

Intermediate **63a:** 6-Chloro-N-cyclopropyl-5-(piperazin-1-yl)pyridine carboxamide hydrochloride

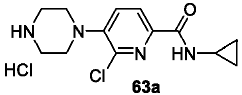

The synthesis method refers to that of **36a**, except that cyclopropylamine was used instead of methylamine, LCMS (ESI): m/z 281.3 [M+H]⁺; RT= 1.3 min (3.00 min).

Intermediate **64a:** 8'-(1-chloroethyl)-7'-fluoro-3'H-spiro[cyclopropane-1,2'-imidazo[1,2-c]quinazolin]-5'(6'H)-one

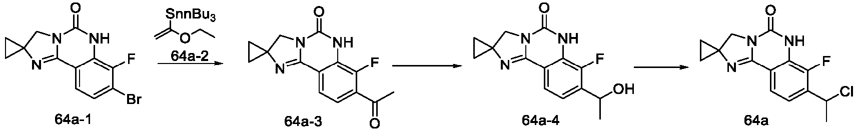

### Step 1: synthesis of 8'-acetyl-7'-fluoro-3'-H-spiro[cyclopropane-1,2'-imidazo[1,2-c]quinazoline]-5'(6'H)-one

**64a-1** (50 mg, 0.16 mmol), **64a-2** (87 mg, 0.24 mmol) in anhydrous dioxane (5mL) was added Pd(PPh₃)₂Cl₂ (11 mg, 0.016 mmol) and the mixture was stirred at 100°C for 6 h. After concentration, the residue was added with 5N hydrochloric acid (10 mL) and stirred at rt for 1 h, then neutralized with saturated sodium bicarbonate aqueous solution to basic. The mixture was extracted with EA, and the organic phase was collected, dried with anhydrous sodium sulfate, concentrated, and purified by normal phase column chromatography (PE:EA=3:1) to obtain compound **64a-3** (20 mg, white solid), yield: 46%. LCMS (ESI): m/z 274.1 [M+H]⁺; RT=1.21 min (3.0 min).

### Step 2: synthesis of 7'-Fluoro-8'-(1-hydroxyethyl)-3'-H-spiro[cyclopropane-1,2'-imidazo[1,2-c]quinazoline]-5'(6'H)-one

**64a-3** (50 mg, 0.18 mmol) in anhydrous methanol (10 mL) was added sodium borohydride (69 mg, 1.8 mmol). The mixture was stirred at 0°C for 2h. The mixture was concentrated, extracted with EA (30 mL×3) and water (20 mL). The organic phase was dried over anhydrous sodium sulfate and concentrated to obtain compound **64a-4** (30 mg, white solid), with a yield of 60%. LCMS (ESI): m/z 276.2 [M+H]⁺; RT=1.09 min (3.0 min).

### Step 3: synthesis of 8'-(1-chloroethyl)-7'-fluoro-3'-H-spiro[cyclopropane-1,2'-imidazo[1,2-c]quinazoline]-5'(6'H)-one

**64a-4** (20 mg, 0.073 mmol) in anhydrous dichloromethane (10 mL) was added thionyl chloride (1 mL) at 0°C. The mixture was stirred for 2 h, and concentrated under reduced pressure to obtain crude product **64a** (20 mg, white solid), yield: 100%. LCMS (ESI): m/z 294.1 [M+H]⁺; RT=1.38 min (3.0 min).

Intermediate **65a:** (2R)-8-(1-bromoethyl)-7-fluoro-2-methyl-2,6-dihydroimidazo[1,2-c]quinazolin-5(3H)-one

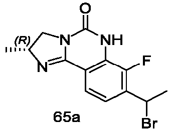

The synthesis method refers to that of **64a,** except that **64a-1** was replaced by (R)-8-bromo-7-fluoro-2-methyl-2,6-dihydroimidazo[1,2-c]quinazolin-5(3H)-one, LCMS (ESI): m/z 326.0 [M+H]⁺; RT=1.37 min (3.0 min).

Intermediate **66a:** (R)-(2-methyl-5-oxo-2,3,5,6-tetrahydroimidazo[1,2-c]quinazolin-8-yl)methanesulfonate

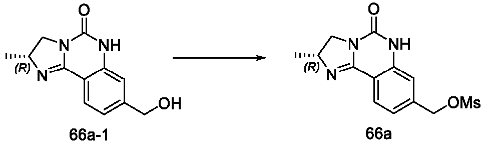

To a solution of **66a-1** (100 mg, 0.43 mmol) and triethylamine (0.18 mL, 1.29 mmol) in THF (10 mL) was added methanesulfonyl chloride (0.05 mL, 0.65 mmol) at 0 °C. The reaction was carried out for 2 h at rt. The reaction was carried out at rt for 2 h. The reaction solution was concentrated to obtain **66a** (120 mg, crude, yellow solid), LCMS (ESI): m/z 310.0 [M+H]⁺; RT = 0.807 min (2.50 min).

Intermediate **67a:** 9-(bromomethyl)-2,3,4,7-tetrahydro-6-pyrimido[1,2-c]quinazolin-6-one

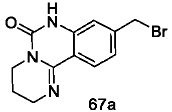

The synthesis method refers to that of **45a,** except that 2-amino-4-bromobenzonitrile and 1,2-diaminopropane was used as starting materials. LCMS (ESI): m/z 296.0 [M+H]⁺; RT= 0.715 min (2.50 min).

Intermediate **68a:** N-methyl-5-(piperazin-1-yl)pyridinamide hydrochloride

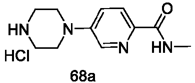

The synthesis method refers to that of Intermediate **2a,** except that step 1 was not performed and the coupling reaction with 4-Boc piperazine was performed using methyl 5-bromopyridine-2-carboxylate. LCMS (ESI): m/z 221.2 [M+H]⁺; RT=0.285 min (6.00 min).

Intermediate **69a:** 2-chloro-N-methyl-1',2',3',6'-tetrahydro-[3,4'-bipyridyl]-6-carboxamide hydrochloride

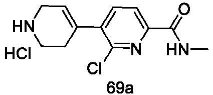

The synthesis method refers to that of Intermediate **36a,** except that N-Boc-1,2,5,6-tetrahydropyridine-4-boronic acid pinacol ester was used as starting material, LCMS (ESI): m/z 252.2 [M+H]⁺; RT= 0.95 min (3.00 min).

Intermediate **70a:** 6-chloro-N-methyl-5-(piperidin-4-yl)pyridine carboxamide hydrochloride

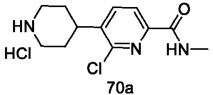

**69a** (240 mg, 0.85 mmol), platinum dioxide (30 mg) were sequentially added to methanol (5 mL) and the mixture was stirred under hydrogen for 4 h at rt. The reaction solution was filtered, and the filtrate was concentrated to obtain **70a** (130 mg, white solid), LCMS (ESI): m/z 254.3 [M+H]⁺; RT= 0.97 min (3.00 min).

Intermediate **71a:** (R) -6-chloro-N-methyl-5-(3-methylpiperazin-1-yl)pyridinamide

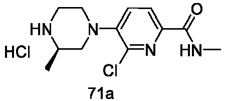

The synthesis method refers to that of Intermediate **36a,** except that (S)-1-N-Boc-2-methylpiperazine was used as starting material, LCMS (ESI): m/z 269.2 [M+H]⁺; RT=0.91 min (3.0 min).

Intermediate**72a**: 6-chloro-N-(2,2-difluoroethyl)-5-(piperazin-1-yl)pyridinamide

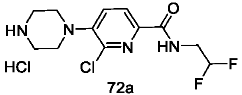

The synthesis method refers to that of Intermediate **36a,** except that 2,2-difluoroethan-1-amine was used as starting material, LCMS (ESI): m/z 305.2[M+H]⁺; RT=1.02 min (3.0 min).

Intermediate **73a:** 6-(difluoromethyl)-N-methyl-5-(piperazin-1-yl)pyridinamide

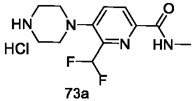

The synthesis method refers to that of Intermediate **36a,** except that 5-bromo-6-(difluoromethyl)-N-methylpyridinamide was used as starting material, LCMS (ESI): m/z 271.3 [M+H]⁺; RT=1.54 min (3.0 min).

Intermediate **74a:** N-methyl-5-(((2R,3S)-2-methylazetidin-3-yl)oxy)pyridinamide

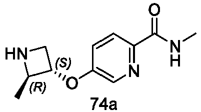

The synthesis method refers to that of Intermediate **12a,** except that tert-butyl (2R,3R)-3-hydroxy-2-methylazetidine-1-carboxylate was used as starting material. LCMS (ESI): m/z 222.2 [M+H]⁺; RT=0.44 min (3.0 min).

Synthesis of compound **4**

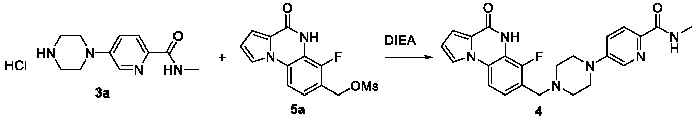

A 25 mL single mouth vial containing 5 mL of acetonitrile was charged with **5a** (150 mg, 0.48 mmol), **3a** (124 mg, 0.48 mmol) and DIEA (187 mg, 1.45 mmol). The reaction was carried out at 70°C for 2h. The reaction solution was prepared by pre-HPLC (formic acid) to obtain compound **4** (40 mg, white solid), yield: 19 %.

LCMS(ESI): m/z 435.05 [M+H]⁺; RT=3.198 min (6.00 min).

¹H NMR (400 MHz, DMSO-*d₆*) : δ 11.30 (s, 1H), 8.39 (d, J=4.8 Hz, 1H), 8.26 (s, 1H), 8.19 (s, 1H), 7.89 (d, J=8.4 Hz, 1H), 7.83 (d, J=8.8 Hz, 1H), 7.39 (d, J=8.4 Hz, 1H), 7.25 (s, 1H), 7.08 (d, J=3.2 Hz, 1H), 6.73-6.71 (m, 1H), 3.66 (s, 2H), 3.34-3.23 (m, 4H), 2.78 (d, J=4.8 Hz, 3H), 2.64-2.54 (m, 4H).

### Synthesis of compounds 5-204

Compounds **5-204** were synthesised according to the method described in Compound 4 by replacing intermediates **5a** and **3a,** respectively, with the intermediates in the table below, and the structural formulae of the compounds of each embodiment are given in the preceding table.

| Com poun ds | LCMS (ESI): [M+H]⁺ | ¹H NMR (400 MHz) DMSO-*d₆* | inter medi ate |
|---|---|---|---|
| 5 | 449.1 | δ 11.19 (s, 1H), 8.39 (d, *J*=4.8 Hz, 1H), 8.16 (s, 1H), 8.01 (d, *J*=8.4 Hz, 1H), 7.84 (d, *J*=7.6 Hz, 1H), 7.58-7.53 (m, 1H), 7.30 (s, 1H), 7.20-7.18 (m, 1H), 7.03-7.01 (m, 1H), 6.69-6.67 (m, 1H), 3.54-3.49 (m, 1H), 3.16 (s, 4H), 2.77 (d, *J*=4.8 Hz, 3H), 2.63-2.60 (m, 2H), 2.52-2.51 (m, 2H), 1.35 (d, *J=6.8* Hz, 3H). | 6a, 2a |
| 6 | 431.1 | δ 11.19 (s, 1H), 8.39 (d, J=4.8 Hz, 1H), 8.25 (d, J=2.4 Hz, 1H), 8.16 (s, 1H), 8.01 (d, J=8.4 Hz, 1H), 7.82 (d, J=8.8 Hz, 1H), 7.39-7.36 (m, 1H), 7.31 (s, 1H), 7.19 (d, J=8.4 Hz, 1H), 7.02 (s, 1H), 6.69-6.67 (m, 1H), 3.41-3.32 (m, 5H), 2.78 (d, J=4.4 Hz, 3H), 2.62-2.59 (m, 2H), 2.51-2.47 (m, 2H), 1.36 (d, J=6.4 Hz, 3H). | 6a, 3a |
| 7 | 467.1 | δ 11.27 (s, 1H), 8.40-8.37 (m, 1H), 8.20-8.19 (m, 1H), 7.89 (d, J = 8.8 Hz, 1H), 7.82 (d, J = 1.2 Hz, 1H), 7.56-7.51 (m, 1H), 7.28-7.24 (m, 1H), 7.08-7.07 (m, 1H), 6.72-6.70 (m, 1H), 3.95-3.93 (m, 1H), 3.16-3.14 (m, 4H), 2.77 (d, J = 9.2 Hz, 3H), 2.61-2.50 (m, 4H), 1.40 (d, J = 6.8 Hz, 3H). | 7a, 2a |
| 8 | 449.1 | δ 11.27 (s, 1H), 8.39-8.36 (m, 1H), 8.23 (d, J = 2.4 Hz, 1H), 8.18 (d, J = 1.2 Hz, 1H), 7.89 (d, J = 8.4 Hz, 1H), 7.81 (d, J = 8.8 Hz, 1H), 7.37-7.28 (m, 1H), 7.27-7.24 (m, 1H), 7.08-7.07 (m, 1H), 6.72-6.70 (m, 1H), 3.94-3.89 (m, 1H), 3.34-3.33 (m, 4H), 2.77 (d, J = 4.8 Hz, 3H), 2.60-2.56 (m, 4H), 1.41 (d, J = 2.4 Hz, 3H). | 7a, 3a |
| 9 | 418.1 | δ 11.19 (s, 1H), 8.43 (s, 1H), 8.32-8.12 (m, 3H), 7.87 (d, J=8.4 Hz, 1H), 7.47 (s, 3H), 7.19 (s, 1H), 3.65-3.57 (m, 2H), 3.26-3.17 (m, 4H), 2.80 (d, J=4.8 Hz, 3H), 2.64-2.58 (m, 4H). | 8a, 3a |
| 12 | 450.1 | δ 11.85 (s, 1H), 8.40 (d, J = 3.6 Hz, 1H), 8.20-8.07 (m, 2H), 7.84 (d, J = 7.6 Hz, 1H), 7.58-7.53 (m, 1H), 7.42 (s, 1H), 7.32 (d, J = 8.4 Hz, 1H), 7.15 (s, 1H), 3.58-3.54 (m, 1H), 3.17 (s, 4H), 2.77 (d, J = 4.0 Hz, 3H), 2.62 (s, 4H), 1.37 (d, J = 6.0 Hz, 3H). | 9a, 2a |
| 13 | 468.3 | δ 8.40-8.36 (m, 1H), 8.10 (d, J = 2.0 Hz, 1H), 7.96 (d, J = 8.8 Hz, 1H), 7.83 (d, J = 8.0 Hz, 1H), 7.56-7.52 (m, 1H), 7.40-7.36 (m, 1H), 7.18 (d, J = 2.0 Hz, 1H), 4.0-3.95 (m, 1H), 3.18-3.12 (m, 4H), 2.76 (d, J = 4.8 Hz, 3H), 2.64-2.54 (m, 4H), 1.41 (d, J = 6.8 Hz, 3H). | 13a, 2a |
| 15 | 464.2 | δ 11.36 (s, 1H), 8.39 (d, J=5.2 Hz, 1H), 8.18 (d, *J*=8.4 Hz, 1H), 8.08 (s, 1H), 7.84 (d, *J*=8.0 Hz, 1H), 7.58-7.56 (m, 1H), 7.49 (s, 1H), 7.32-7.30 (m, 1H), 4.36 (s, 3H), 3.55 (d, *J=6.8* Hz, 2H), 3.17 (s, 5H), 2.76 (d, *J*=4.8 Hz, 3H), 2.65-2.63 (m, 2H), 1.37 (d, *J=6.8* Hz, 3H). | 14a, 2a |
| 16 | 450.20 | δ 11.39 (s, 1H), 8.41 (d, *J* = 4.8 Hz, 1H), 8.18 (d, *J* = 8.0 Hz, 1H), 8.08 (s, 1H), 7.87-7.85 (m, 1H), 7.61-7.56 (m, 1H), 7.47 (s, 1H), 7.30 (d, *J* = 8.4 Hz, 1H), 4.37 (s, 3H), 3.65 (s, 2H), 3.20 (s, 4H), 2.78 (d, *J*=4.8 Hz, 3H), 2.60 (s, 4H). | 15a, 2a |
| 17 | 482.2 | δ 11.33 (s, 1H), 8.41-4.37 (m, 1H), 8.14 (s, 1H), 8.04 (d, *J*=8.4 Hz, 1H), 7.83 (d, *J*=8.0 Hz, 1H), 7.57-7.53 (m, 1H), 7.40-7.37 (m, 1H), 4.37 (s, 3H), 4.03-3.98 (m, 1H), 3.17-3.15 (m, 4H), 2.76 (d, *J*=5.2 Hz, 3H), 2.65-2.55 (m, 4H), 1.42 (d, *J*=6.8 Hz, 3H). | 16a, 2a |
| 19 | 482.3 | δ 11.02 (s, 1H), 8.59 (s, 1H), 8.40-4.36 (m, 1H), 7.83-7.80 (m, 2H), 7.56-7.53 (m, 1H), 7.29-7.26 (m, 1H), 4.10 (s, 3H), 3.99-3.94 (m, 1H), 3.15-3.13 (m, 4H), 2.75 (d, J=4.8 Hz, 3H), 2.63-2.53 (m, 4H), 1.40 (d, J=6.8 Hz, 3H). | 18a, 2a |
| 21 | 464.4 | δ 8.45 (s, 1H), 7.89-7.85 (m, 2H), 7.51-7.49 (m, 1H), 7.47 (s, 1H), 7.28 (d, J = 8.0 Hz, 1H), 4.19 (s, 3H), 3.57-3.55 (m, 1H), 3.25 (s, 4H), 2.90 (s, 3H), 2.79-2.75 (m, 2H), 2.65-2.61 (m, 2H), 1.47 (d, J = 6.8 Hz, 3H). | 20a, 2a |
| 22 | 450.4 | δ 11.27 (s, 1H), 8.61 (s, 1H), 8.40-8.39 (m, 1H), 7.85-7.80 (m, 2H), 7.59-7.54 (m, 1H), 7.32 (s, 1H), 7.16-7.14 (m, 1H), 4.12 (s, 3H), 3.57 (s, 2H), 3.18 (s, 4H), 2.76 (d, *J*=4.8 Hz, 3H), 2.56 (s, 4H). | 21a, 2a |
| 25 | 468.9 | δ 8.59 (s, 1H), 8.40-8.35 (m, 2H), 8.15 (d, J = 8.6 Hz, 1H), 7.83 (d, J = 8.2 Hz, 1H), 7.55 (dd, J = 10.5, 8.2 Hz, 1H), 7.48-7.38 (m, 1H), 4.00 (q, J = 6.8 Hz, 1H), 3.19-3.16 (m, 4H), 2.76 (d, J = 4.8 Hz, 3H), 2.67-2.53 (m, 4H), 1.43 (d, J = 6.8 Hz, 3H). | 32a, 2a |
| 26 | 454.9 | δ 8.55 (s, 1H), 8.44-8.26 (m, 3H), 8.12 (d, *J* = 8.4 Hz, 1H), 7.84 (d, *J =* 7.2 Hz, 1H), 7.56 (d, *J =* 2.2 Hz, 1H), 7.37 (s, 1H), 3.72 (s, 2H), 3.18-3.16 (m, 4H), 2.76 (d, *J =* 4.8 Hz, 3H), 2.62-2.59 (m, 4H). | 31a, 2a |
| 34 | 480.4 | δ 10.60 (s, 1H), 8.45 (t, J = 6.1 Hz, 1H), 7.79 (d, J = 8.3 Hz, 1H), 7.60 (d, J = 8.1 Hz, 1H), 7.47 (d, J = 8.3 Hz, 1H), 7.14 (dd, J = 8.1, 6.2 Hz, 1H), 4.28 (dt, J = 10.0, 7.1 Hz, 1H), 3.98 (t, J = 10.4 Hz, 1H), 3.65 (s, 2H), 3.41-3.38 (m, 1H), 3.31-3.27 (m 2H), 2.94 (m, 4H), 2.60 (m, 4H), 2.48 (s, 3H), 1.26 (d, J=6.7 Hz, 3H), 1.10 (t, J=7.2 Hz, 3H). | 53a, 61a |
| 75 | 419.3 | δ 11.86 (s, 1H), 8.53 (d, *J*=4.4 Hz, 1H), 8.25-8.22 (m, 2H), 8.09-8.06 (m, 1H), 7.95 (d, *J*=8.4 Hz, 1H), 7.49-7.46 (m, 1H), 7.38 (d, *J*=1.2 Hz, 1H), 7.29-7.26 (m, 1H), 7.14 (d, *J*=2.0 Hz, 1H), 5.08-5.04 (m, 1H), 3.73-3.65 (m, 2H), 2.93-2.89 (m, 1H), 2.79-2.72 (m, 5H), 2.48-2.35 (m, 2H), 1.85-1.81 (m, 1H). | 8a, 12a |
| 77 | 470.2 | δ 11.81 (s, 1H), 8.40 (q, J = 4.4 Hz, 1H), 8.23 (d, J = 5.2 Hz, 1H), 8.07 (d, J = 5.2 Hz, 1H), 7.99 (d, J = 8.2 Hz, 1H), 7.87 -7.81 (m, 1H), 7.56 (dd, J = 10.6, 8.2 Hz, 1H), 7.35-7.28 (m, 1H), 3.72 (s, 2H), 3.17 (s, 4H), 2.76 (d, J = 4.8 Hz, 3H), 2.61 (s, 4H). | 22a, 2a |
| 80 | 469.9 | δ 11.12 (s, 1H), 8.58 (d, J = 3.0 Hz, 1H), 8.47-8.34 (m, 2H), 7.91 (d, J = 8.2 Hz, 1H), 7.84 (d, J = 8.2 Hz, 1H), 7.55 (dt, J = 16.6, 8.2 Hz, 1H), 7.24-7.20 (m, 1H), 3.68 (s, 2H), 3.40-3.36 (m, 4H), 2.76 (d, J = 4.8 Hz, 3H), 2.63-2.60 (m, 4H). | 23a, 2a |
| 98 | 418.3 | δ 8.44 (d, J = 2.0 Hz, 1H), 8.10 (d, J = 2.0 Hz, 1H), 7.95 (d, J = 8.8 Hz, 1H), 7.83-7.80 (m, 1H), 7.40-7.36 (m, 1H), 7.18 (d, J = 2.0 Hz, 1H), 6.89 (d, J = 9.2 Hz, 1H), 3.96-3.94 (m, 1H), 3.66-3.63 (m, 4H), 2.46-2.42 (m, 4H), 1.40 (d, J = 6.4 Hz, 3H). | 13a, 24a |
| 99 | 428.7 | δ 11.98 (s, 1H), 8.14-8.11 (m, 1H), 7.96 (d, J = 8.8 Hz, 1H), 7.39 (s, 1H), 7.20-7.15 (m, 2H), 7.06-6.98 (m, 2H), 3.96-3.95 (m, 1H), 2.95 (s, 4H), 2.63-2.51 (m, 4H), 1.41-1.37 (m, 3H). | 13a, 25a |
| 101 | 450.4 | δ 11.10 (s, 1H), 8.53 (s, 1H), 8.44-8.40 (m, 1H), 7.95 (d, *J*=8.0 Hz, 1H), 7.86-7.83 (m, 1H), 7.60-7.55 (m, 1H), 7.34 (s, 1H), 7.19-7.17 (m, 1H), 4.08 (s, 3H), 3.59 (s, 2H), 3.18 (s, 4H), 2.76 (d, *J*=4.0 Hz, 3H), 2.57 (s, 4H). | 26a, 2a |
| 102 | 451.3 | δ 11.89 (m, 1H), 8.41 (q, J = 4.6 Hz, 1H), 7.88-7.81 (m, 2H), 7.58 (dd, J = 10.6, 8.2 Hz, 1H), 7.49 (s, 1H), 7.31 (dd, J = 8.2, 1.2 Hz, 1H), 3.65 (s, 2H), 3.19 (m, 4H), 2.77 (d, J = 4.8 Hz, 3H), 2.68-2.64 (s, 3H), 2.59 (m, 4H). | 27a, 2a |
| 103 | 467.1 | δ 11.87 (s, 1H), 8.40 (q, J = 4.6 Hz, 1H), 7.85 (dd, J = 8.0, 1.2 Hz, 1H), 7.71 (s, 1H), 7.57 (dd, J = 10.6, 8.2 Hz, 1H), 7.44 (s, 1H), 7.24 (dd, J = 8.1, 1.2 Hz, 1H), 3.63 (s, 3H), 3.18 (d, J = 4.5 Hz, 4H), 2.82 (s, 3H), 2.77 (d, J = 4.8 Hz, 3H), 2.58 (s, 4H). | 28a, 2a |
| 112 | 510.4 | δ 8.40-8.38 (m, 1H), 8.11 (d, J=2.0 Hz, 1H), 7.95 (d, J=8.4 Hz, 1H), 7.85-7.83 (m, 1H), 7.59-7.55 (m, 1H), 7.38-7.34 (m, 1H), 7.20 (d, J=2.0 Hz, 1H), 4.46-4.41 (m, 1H), 3.87-3.79 (m, 2H), 3.73-3.68 (m, 3H), 3.58-3.55 (m, 1H), 3.18 (s, 4H), 2.61 (s, 4H), 2.14-2.08 (m, 1H), 2.00-1.93 (m, 1H). | 10a, 35a |
| 113 | 470.3 | δ 11.99-11.94 (m, 1H), 8.44-8.41 (m, 1H), 7.96-7.91 (m, 2H), 7.66 (d, J = 8.0 Hz, 1H), 7.38-7.34 (m, 1H), 7.20 (d, J = 2.0 Hz, 1H), 3.72 (s, 2H), 3.11 (s, 4H), 2.79 (d, J = 4.8 Hz, 3H), 2.63 (s, 4H). | 10a, 36a |
| 117 | 450.3 | δ 11.71 (s, 1H), 8.41 (q, J = 4.8 Hz, 1H), 8.05 (d, J = 8.0 Hz, 1H), 7.85 (d, J = 8.0 Hz, 1H), 7.58 (dd, J = 11.2, 7.4 Hz, 2H), 7.36 (s, 1H), 7.28 (d, J = 8.0 Hz, 1H), 3.63 (s, 2H), 3.19 (d, J = 4.6 Hz, 4H), 2.77 (d, J = 4.8 Hz, 3H), 2.59 (d, J = 4.2 Hz, 4H), 2.31 (s, 3H). | 40a, 2a |
| 123 | 456.3 | δ 8.42-8.40 (m, 1H), 7.84 (d, J = 4.0 Hz, 1H), 7.61-7.57 (m, 2H), 7.16-7.14 (m, 1H), 3.98-3.95 (m, 2H), 3.87-3.84 (m, 2H), 3.65 (s, 2H), 3.18 (d, J = 4.0 Hz, 4H), 2.77 (s, 3H), 2.60-2.59 (m, 4H). | 41a, 2a |
| 124 | 438.2 | δ 10.47 (s, 1H), 8.41-8.39 (m, 1H), 7.84 (d, J=7.2 Hz, 1H), 7.74 (d, J=8.4 Hz, 1H), 7.59-7.55 (m, 1H), 7.06 (d, J=3.2 Hz, 2H), 3.96-3.91 (m, 2H), 3.84-3.79 (m, 2H), 3.55 (s, 2H), 3.18 (s, 4H), 2.76 (d, J=4.8 Hz, 3H), 2.55-2.54 (m, 4H). | 42a, 2a |
| 125 | 454.2 | δ 10.48 (s, 1H), 8.44-8.42 (m, 1H), 7.94 (d, *J*=8.0 Hz, 1H), 7.74 (d, J=7.6 Hz, 1H), 7.67 (d, *J*=8.4 Hz, 1H), 7.07 (d, *J*=8.4Hz, 2H), 3.93-3.91 (m, 2H), 3.84-3.82 (m, 2H), 3.56 (s, 2H), 3.12 (s, 4H), 2.79 (d, *J*=4.8 Hz, 3H), 2.57 (s, 4H). | 42a, 36a |
| 126 | 472.2 | δ 10.60 (s, 1H), 8.43 (d, J = 4.8 Hz, 1H), 7.94 (d, J = 8.2 Hz, 1H), 7.67-7.59 (m, 2H), 7.20-7.11 (m, 1H), 3.96 (t, J = 8.8 Hz, 2H), 3.85 (t, J = 8.8 Hz, 2H), 3.66 (s, 2H), 3.11 (m, 4H), 2.79 (d, J = 4.8 Hz, 3H), 2.61 (m, 4H). | 41a, 36a |
| 127 | 452.3 | δ 9.63 (s, 1H), 8.40 (q, J = 4.6 Hz, 1H), 7.83 (dd, J = 8.0, 0.8 Hz, 1H), 7.64 (d, J = 8.0 Hz, 1H), 7.55 (dd, J = 10.4, 8.2 Hz, 1H), 7.07 (d, J = 8.0 Hz, 1H), 3.93 (d, J=9.2 Hz, 2H), 3.86 (d, J = 9.2 Hz, 2H), 3.54 (s, 2H), 3.14 (m, 4H), 2.76 (d, J=4.8 Hz, 3H), 2.55 (m, 4H), 2.29 (s, 3H). | 43a, 2a |
| 128 | 470.3 | δ 10.58 (m, 1H), 8.43 (t, J = 6.0 Hz, 1H), 7.84 (d, J = 8.0 Hz, 1H), 7.62-7.52 (m, 2H), 7.18-7.10 (m, 1H), 3.95 (t, J = 9.6 Hz, 2H), 3.84 (t, J = 9.8 Hz, 2H), 3.63 (s, 2H), 3.26 (dd, J = 13.6, 6.9 Hz, 2H), 3.16 (m, 4H), 2.62-2.55 (m, 4H), 1.08 (t, J = 7.2 Hz, 3H). | 41a, 34a |
| 129 | 452.1 | δ 10.48 (s, 1H), 8.41 (q, J = 4.8 Hz, 1H), 7.78 (d, J = 8.2 Hz, 1H), 7.59 (d, J = 8.2 Hz, 1H), 7.46 (d, J = 8.4 Hz, 1H), 7.19-7.09 (m, 1H), 3.95 (t, J = 9.7 Hz, 2H), 3.84 (t, J = 9.5 Hz, 2H), 3.65 (s, 2H), 2.94 (s, 4H), 2.79 (d, J = 4.8 Hz, 3H), 2.59 (s, 4H), 2.48 (s, 3H). | 41a, 37a |
| 130 | 478.1 | δ 10.57 (s, 1H), 8.32 (d, J = 4.8 Hz, 1H), 7.78 (d, J = 8.2 Hz, 1H), 7.59 (d, J = 8.2 Hz, 1H), 7.46 (d, J = 8.4 Hz, 1H), 7.19-7.09 (m, 1H), 3.95 (t, J = 9.6 Hz, 2H), 3.84 (t, J = 9.6 Hz, 2H), 3.64 (s, 2H), 2.93 (s, 4H), 2.86-2.83 (m, 1H), 2.59 (s, 4H), 2.46 (s, 3H), 0.71-0.66 (m, 2H), 0.65-0.61 (m, 2H). | 41a, 44a |
| 131 | 470.1 | δ 10.61 (s, 1H), 8.40 (q, J = 4.6 Hz, 1H), 7.86-7.81 (m, 1H), 7.63-7.51 (m, 2H), 7.18-7.10 (m, 1H), 4.34- 4.23 (m, 1H), 3.98 (t, J = 10.4 Hz, 1H), 3.64 (s, 2H), 3.42-3.38 (m, 1H), 3.17 (s, 4H), 2.77 (t, d = 4.8 Hz, 3H), 2.58 (s, 4H), 1.26 (d, J = 6.6 Hz, 3H). | |
| 132 | 470.3 | δ 10.62 (s, 1H), 8.40 (q, J = 4.6 Hz, 1H), 7.84 (dd, J = 8.0, 1.2 Hz, 1H), 7.63-7.49 (m, 2H), 7.14 (dd, J = 8.0, 6.4 Hz, 1H), 4.35-4.22 (m, 1H), 3.98 (t, J = 10.4 Hz, 1H), 3.64 (s, 2H), 3.43-3.36 (m, 1H), 3.17 (s, 4H), 2.77 (t, J = 7.2 Hz, 3H), 2.56 (d, J = 13.6 Hz, 4H), 1.26 (d, J = 6.8 Hz, 3H). | 53a, 2a |
| 133 | 470.3 | δ 10.54 (s, 1H), 8.41-8.38 (m, 1H), 7.84-7.83 (m, 1H), 7.60-7.58 (m, 1H), 7.57-7.54 (m, 2H), 7.13 (dd, *J =* 7.8, 6.0 Hz, 1H), 4.30-4.27 (m, 1H), 3.99-3.96 (m, 1H), 3.64 (s, 2H), 3.41-3.37 (m, 1H), 3.17-3.15 (m, 4H), 2.77 (d, *J* = 4.8 Hz, 3H), 2.59-2.57 (m, 4H), 1.25 (d, *J =* 6.6 Hz, 3H) | 52a, 2a |
| 134 | 472.3 | δ 8.41 (d, J = 4.8 Hz, 1H), 7.88-7.80 (m, 1H), 7.79 (d, J = 8.1 Hz, 1H), 7.59-7.56 (m, 1H), 7.29 (d, J = 8.0 Hz, 1H), 3.99-3.96 (m, 2H), 3.88-3.85 (m, 2H), 3.68 (s, 2H), 3.19-3.18 (m, 4H), 2.76 (d, J = 4.8 Hz, 3H), 2.64-2.57 (m, 4H). | 45a, 2a |
| 135 | 472.3 | δ 10.59 (s, 1H), 8.42-8.41 (m, 1H), 7.86 (d, J = 7.2 Hz, 1H), 7.73 (s, 1H), 7.59 (dd, J = 10.4, 8.2 Hz, 1H), 7.32 (s, 1H), 3.96-3.93 (m, 2H), 3.86-3.81 (m, 2H), 3.65 (s, 2H), 3.23-3.19 (m, 4H), 2.77 (d, J = 4.8 Hz, 3H), 2.68-2.65 (m, 4H). | 46a, 2a |
| 136 | 484.3 | δ 10.56 (m, 1H), 8.40 (d, J=4.7 Hz, 1H), 7.84 (d, J= 7.9 Hz, 1H), 7.57 (dd, J = 17.9, 8.1 Hz, 2H), 7.19-7.07 (m, 1H), 3.64 (s, 2H), 3.59 (s, 2H), 3.17 (m, 4H), 2.77 (d, J=4.7 Hz, 3H), 2.58 (m, 4H), 1.29 (s, 6H). | 47a, 2a |
| 137 | 470.3 | δ 10.58 (s, 1H), 8.55-8.21 (m, 1H), 7.98-7.68 (m, 1H), 7.71-7.40 (m, 2H), 7.15 (dd, *J* = 8.0, 6.4 Hz, 1H), 4.53-4.30 (m, 1H), 4.14-4.11 (m, 1H), 3.64 (s, 2H), 3.55-3.50 (m, 1H), 3.17-3.12 (m, 4H), 2.77 (d, *J* = 4.8 Hz, 3H), 2.59-2.57 (m, 4H), 1.33 (d, *J =* 6.2 Hz, 3H). | 48a, 2a |
| 138 | 482.1 | δ 10.59 (s, 1H), 8.40 (d, J = 4.8 Hz, 1H), 7.84 (d, J = 7.9 Hz, 1H), 7.59-7.50 (m, 2H), 7.16-7.08 (m, 1H), 3.91 (s, 2H), 3.64 (s, 2H), 3.17 (s, 4H), 2.77 (d, J = 4.8 Hz, 3H), 2.58 (s, 4H), 1.10-1.00 (m, 2H), 0.89-0.86 (m, 2H). | 49a, 2a |
| 139 | 470.3 | δ 10.70 (s, 1H), 8.40 (q, *J =* 4.6 Hz, 1H), 7.90-7.79 (m, 1H), 7.73 (d, *J=8.2* Hz, 1H), 7.56 (dd, *J*=10.6, 8.2 Hz, 1H), 7.12-7.00 (m, 1H), 3.83-3.72 (m, 2H), 3.62 (s, 2H), 3.51 (t, *J*=5.3 Hz, 2H), 3.17-3.15 (m, 4H), 2.77 (d, *J=4.8* Hz, 3H), 2.57-2.55 (m, 4H), 1.85-1.82 (m, 2H). | 50a, 2a |
| 140 | 466.3 | δ 8.41 (q, *J =* 4.6 Hz, 1H), 7.79 (t, *J =* 8.6 Hz, 1H), 7.73 (d, *J =* 8.2 Hz, 1H), 7.46 (dd, *J* = 8.2, 5.8 Hz, 1H), 7.12-7.03 (m, 1H), 3.77-3.75 (m, 2H), 3.63 (s, 2H), 3.50 (t, *J*=5.4 Hz, 1H), 2.93-2.87 (m, 4H), 2.80 (t, *J*=4.0 Hz, 3H), 2.62-2.61 (m, 4H), 2.48 (s, 3H), 1.84-1.82 (m, 1H). | 50a, 37a |
| 141 | 486.3 | δ 8.43 (q, J = 4.8 Hz, 1H), 7.93 (d, J = 8.2 Hz, 1H), 7.73 (d, J = 8.2 Hz, 1H), 7.65 (d, J = 8.2 Hz, 1H), 7.07 (dd, J = 8.0, 6.4 Hz, 1H), 3.81-3.72 (m, 2H), 3.64 (s, 2H), 3.50 (t, J = 5.6 Hz, 2H), 3.10 (s, 4H), 2.79 (d, J = 4.8 Hz, 3H), 2.59 (s, 4H), 1.89-1.79 (m, 2H). | 50a, 36a |
| 142 | 484.3 | δ 10.71 (s, 1H), 8.40 (q, J = 4.6 Hz, 1H), 7.83 (d, J = 8.0 Hz, 1H), 7.74 (d, J = 8.2 Hz, 1H), 7.55 (dd, J = 10.5, 8.2 Hz, 1H), 7.10-7.03 (m, 1H), 4.01-3.93 (m, 1H), 3.61 (s, 2H), 3.54 (m, 2H), 3.16 (m, 4H), 2.76 (d, J = 4.8 Hz, 3H), 2.56 (m, 4H), 2.04-1.94 (m, 1H), 1.49-1.40 (m, 1H), 1.22 (d, J = 6.8 Hz, 3H). | 55a, 2a |
| 143 | 484.3 | δ 10.66 (s, 1H), 8.54-8.28 (m, 1H), 7.84 (d, J = 7.0 Hz, 1H), 7.75 (d, J = 8.2 Hz, 1H), 7.56 (dd, J = 10.6, 8.2 Hz, 1H), 7.29- 6.91 (m, 1H), 4.00-3.96 (m, 1H), 3.62 (s, 2H), 3.58-3.52 (m, 2H), 3.19-3.16 (m, 4H), 2.77 (d, J = 4.8 Hz, 3H), 2.57-2.55 (m, 4H), 2.05-1.92 (m, 1H), 1.53-1.39 (m, 1H), 1.23 (d, J = 6.6 Hz, 3H). | 51a, 2a |
| 144 | 484.3 | δ 10.71 (s, 1H), 8.40 (q, *J* = 4.6 Hz, 1H), 7.83 (dd, *J* = 8.0, 1.0 Hz, 1H), 7.74 (d, *J* = 8.2 Hz, 1H), 7.55 (dd, *J* = 10.6, 8.2 Hz, 1H), 7.07 (dd, *J* = 8.0, 6.6 Hz, 1H), 4.07-4.04 (m, 1H), 3.67-3.53 (m, 3H), 3.20-3.12 (m, 4H), 3.12- 3.00 (m, 2H), 2.76 (d, *J* = 4.8 Hz, 3H), 2.62-2.54 (m, 4H), 1.90-1.84 (m, 1H), 0.98 (d, *J* = 6.6 Hz, 3H). | 56a, 2a |
| 145 | 484.3 | δ 10.88 (s, 1H), 8.43-8.39 (m, 1H), 7.79-7.76 (m, 2H), 7.47 (d, *J* = 8.4 Hz, 1H), 7.14-7.10 (m, 1H), 5.28 (d, *J* = 47.2 Hz, 1H), 4.40-4.35 (m, 1H), 3.80-3.53 (m, 5H), 2.94-2.92 (m, 4H), 2.79 (d, *J* = 4.8 Hz, 3H), 2.59-2.57 (m, 4H), 2.48 (m, 3H). | 54a, 37a |
| 146 | 504.2 | δ 10.89 (s, 1H), 8.44-8.41 (m, 1H), 7.92 (d, *J* = 7.8 Hz, 1H), 7.76 (d, *J =* 8.4 Hz,1H), 7.65 (d, *J* = 8.4 Hz,1H), 7.13-7.10 (m, 1H), 5.28 (d, *J= 46.8* Hz, 1H), 4.39-4.35 (m, 1H), 3.80-3.70 (m, 1H), 3.66-3.62(m, 1H), 3.10-2.95 (m, 4H), 2.78 (d, *J* = 4.8 Hz, 3H), 2.60-2.57 (m, 4H). | 54a, 36a |
| 147 | 488.3 | δ 10.90 (s, 1H), 8.41-8.38 (m, 1H), 7.84-7.83 (m, 1H), 7.77-7.75 (m, 1H), 7.57-7.54 (m, 1H), 7.13-7.10 (m, 1H), 5.33-5.24 (m, 1H), 4.40-4.35 (m, 1H), 3.83-3.78 (m, 1H), 3.74-3.55 (m, 4H), 3.17-3.15 (m, 4H), 2.77 (d, J = 4.8 Hz, 3H), 2.59-2.57 (m, 4H). | 54a, 2a |
| 148 | 506.2 | δ 8.40 (d, J = 4.8 Hz, 1H), 7.84 (dd, J = 8.2, 0.8 Hz, 1H), 7.79 (d, J = 8.4 Hz, 1H), 7.55 (dd, J = 10.6, 8.2 Hz, 1H), 7.16 (dd, J = 8.2, 6.4 Hz, 1H), 4.12 (t, J = 12.6 Hz, 2H), 3.92 (t, J = 12.6 Hz, 2H), 3.64 (s, 2H), 3.20-3.11 (m, 4H), 2.76 (d, J = 4.8 Hz, 3H), 2.61-2.53 (m, 4H). | 57a, 2a |
| 149 | 488.3 | δ 8.41-8.38 (m, 1H), 8.30 (s, 1H), 7.84-7.83 (m, 1H), 7.77-7.75 (m, 1H), 7.57-7.54 (m, 1H), 7.13-7.10 (m, 1H), 5.28 (d, *J* = 46.8 Hz, 1H), 4.39-4.35 (m, 1H), 3.83-3.70 (m, 1H), 3.69-3.63 (m, 4H), 3.17-3.15 (m, 4H), 2.76 (d, *J* = 4.8 Hz, 3H), 2.58-2.57 (m, 4H). | 59a, 2a |
| 150 | 484.1 | δ 10.70 (s, 1H), 8.40 (q, J = 4.8 Hz, 1H), 7.83 (dd, J = 8.0, 1.2 Hz, 1H), 7.76 (d, J = 8.2 Hz, 1H), 7.55 (dd, J = 10.6, 8.2 Hz, 1H), 7.11-7.03 (m, 1H), 4.71-4.58 (m, 1H), 3.62 (s, 2H), 3.58-4.48 (m, 2H), 3.19 -3.11 (m, 4H), 2.76 (d, J = 4.8 Hz, 3H), 2.56 (dd, J = 11.0, 6.5 Hz, 4H), 1.82-1.70 (m, 2H), 1.19 (d, J = 6.6 Hz, 3H). | 58a, 2a |
| 151 | 466.4 | δ 10.60 (s, 1H), 8.43-8.39 (m, 1H), 7.78 (d, *J* = 8.4 Hz, 1H), 7.59 (d, *J* = 8.4 Hz, 1H), 7.47 (d, *J* = 8.4Hz, 1H), 7.16-7.12 (m, 1H), 4.31-4.25 (m, 1H), 4.00-3.95 (m, 1H), 3.65 (s, 2H), 3.41-3.37 (m, 1H), 2.95-2.90 (m, 4H), 2.80 (s, 3H), 2.53-2.50 (m, 4H), 1.26 (d, *J* = 6.8 Hz, 3H). | 52a, 37a |
| 152 | 486.2 | δ 8.44-8.42 (m, 1H), 7.93 (d, *J* = 8.4 Hz, 1H), 7.65 (d, *J*=7.8 Hz, 1H), 7.59 (d, J=7.8 Hz, 1H), 7.15-7.12 (m, 1H), 4.31-4.27 (m, 1H), 3.99-3.96 (m, 1H), 3.65 (s, 2H), 3.41-3.38 (m, 1H),3.10-2.95 (m, 4H), 2.78 (d, *J*=4.8 Hz, 3H), 2.62-2.58 (m, 4H), 1.26 (d, *J*=6.6 Hz, 3H). | 52a, 36a |
| 153 | 466.3 | δ 10.63 (s, 1H), 8.41 (q, J = 4.7 Hz, 1H), 7.79 (d, J = 8.2 Hz, 1H), 7.60 (d, J = 8.1 Hz, 1H), 7.47 (d, J = 8.4 Hz, 1H), 7.15 (dd, J = 8.0, 6.4 Hz, 1H), 4.29 (dt, J = 9.8, 7.2 Hz, 1H), 3.98 (t, J = 10.4 Hz, 1H), 3.66 (s, 2H), 3.40 (dd, J = 10.8, 7.4 Hz, 1H), 2.94 (s, 4H), 2.80 (d, J = 4.9 Hz, 3H), 2.60 (s, 4H), 2.48 (s, 3H), 1.26 (d, J = 6.6 Hz, 3H). | 53a, 37a |
| 154 | 486.1 | δ 8.43 (q, J = 4.7 Hz, 1H), 7.93 (d, J = 8.2 Hz, 1H), 7.65 (d, J = 8.4 Hz, 1H), 7.60 (d, J = 8.2 Hz, 1H), 7.14 (dd, J = 8.0, 6.4 Hz, 1H), 4.32-4.24 (m, 1H), 3.98 (t, J = 10.4 Hz, 1H), 3.66 (s, 2H), 3.40 (dd, J = 10.6, 7.6 Hz, 1H), 3.10 (s, 4H), 2.79 (d, J = 4.8 Hz, 3H), 2.60 (s, 4H), 1.26 (d, J = 6.8 Hz, 3H). | 53a, 36a |
| 156 | 486.3 | δ 10.58 (s, 1H), 8.44-8.42 (m, 1H), 7.94-7.92 (m, 1H), 7.66-7.65 (m, 1H), 7.61-7.59 (m, 1H), 7.15-7.12 (m, 1H), 4.41-4.38 (m, 1H), 4.13-4.08 (m, 1H), 3.65 (s, 2H), 3.53-3.50 (m, 1H), 3.15-3.12 (m, 4H), 2.78 (d, J = 3.2 Hz, 3H), 2.59-2.57 (m, 4H), 1.32 (d, J = 4.0 Hz, 3H). | 48a, 36a |
| 157 | 478.3 | δ 10.55 (s, 1H), 7.79 (dd, J = 8.2, 3.8 Hz, 1H), 7.53 (d, J = 8.1 Hz, 1H), 7.47 (d, J = 8.4 Hz, 1H), 7.13 (dd, J = 7.8, 6.4 Hz, 1H), 3.91 (s, 2H), 3.65 (s, 2H), 2.94 (s, 4H), 2.80 (d, J = 4.8 Hz, 3H), 2.60 (s, 4H), 2.48 (s, 3H), 1.05 (q, J = 4.6 Hz, 2H), 0.87 (q, J = 4.8 Hz, 2H). | 49a, 37a |
| 158 | 498.2 | δ 8.43 (q, J = 4.8 Hz, 1H), 7.93 (d, J = 8.2 Hz, 1H), 7.65 (d, J = 8.2 Hz, 1H), 7.53 (d, J = 8.2 Hz, 1H), 7.12 (dd, J = 7.8, 6.4 Hz, 1H), 3.91 (s, 2H), 3.65 (s, 2H), 3.11 (s, 4H), 2.79 (d, J = 4.8 Hz, 3H), 2.60 (s, 4H), 1.05 (q, J = 4.6 Hz, 2H), 0.87 (q, J = 4.8Hz, 2H). | 49a, 36a |
| 159 | 522.2 | δ 8.43 (d, J = 4.8 Hz, 1H), 7.93 (d, J = 8.2 Hz, 1H), 7.79 (d, J = 8.2 Hz, 1H), 7.65 (d, J = 8.2 Hz, 1H), 7.16 (dd, J = 8.2, 6.6 Hz, 1H), 4.12 (t, J = 12.6 Hz, 2H), 3.91 (t, J = 12.8 Hz, 2H), 3.66 (s, 2H), 3.10 (s, 4H), 2.78 (d, J = 4.8 Hz, 3H), 2.60 (s, 4H). | 57a, 36a |
| 160 | 502.3 | δ 11.13 (s, 1H), 8.41 (q, J = 4.7 Hz, 1H), 7.80-7.77 (m, 2H), 7.47 (d, J = 8.4 Hz, 1H), 7.16 (dd, J = 8.2, 6.6 Hz, 1H), 4.12 (t, J = 12.6 Hz, 2H), 3.92 (t, J = 12.6 Hz, 2H), 3.66 (s, 2H), 2.94 (s, 4H), 2.80 (d, J = 4.8 Hz, 3H), 2.60 (s, 4H), 2.48 (s, 3H). | 57a, 37a |
| 162 | 496.3 | δ 10.73 (s, 1H), 8.55-8.23 (m, 1H), 7.84 (d, *J* = 8.0 Hz, 1H), 7.75 (d, *J =* 8.2 Hz, 1H), 7.56 (dd, *J* = 10.5, 8.2 Hz, 1H), 7.19-6.92 (m, 1H), 3.63 (s, 2H), 3.58 (s, 2H), 3.17-3.16 (m, 4H), 2.76 (d, *J* = 4.8 Hz, 3H), 2.58-2.56 (m, 4H), 0.77-0.30 (m, 4H). | 60a, 2a |
| 164 | 453.4 | δ 10.31 (m, 1H), 8.54 (q, J = 4.8 Hz, 1H), 8.23 (d, J = 2.8 Hz, 1H), 7.94 (d, J = 8.7 Hz, 1H), 7.57 (d, J = 8.0 Hz, 1H), 7.46 (dd, J = 8.7, 2.9 Hz, 1H), 7.11 (dd, J = 8.1, 6.2 Hz, 1H), 5.03 (td, J = 6.6, 2.9 Hz, 1H), 4.27 (dt, J = 10.1, 7.1 Hz, 1H), 3.97 (t, J = 10.4 Hz, 1H), 3.70 (d, J = 2.4 Hz, 2H), 3.39 (dd, J = 10.7, 7.4 Hz, 1H), 2.91 (dd, J = 10.5, 6.1 Hz, 1H), 2.85-2.66 (m, 5H), 2.49-2.43 (m, 1H), 2.40-2.30 (m, 1H), 1.79 (dt, J = 17.3, 7.0 Hz, 1H), 1.25 (d, J = 6.6 Hz, 3H). | 53a, 12a |
| 171 | 484.3 | δ 8.43 (t, J = 6.0 Hz, 1H), 7.84 (dd, J = 8.0, 1.0 Hz, 1H), 7.73 (d, J = 8.2 Hz, 1H), 7.55 (dd, J = 10.6, 8.2 Hz, 1H), 7.10-7.02 (m, 1H), 3.76 (t, J = 5.8 Hz, 2H), 3.62 (s, 2H), 3.50 (t, J = 5.6 Hz, 2H), 3.26 (dd, J = 7.0, 6.2 Hz, 2H), 3.19-3.11 (m, 4H), 2.60-2.53 (m, 4H), 1.88-1.77 (m, 2H), 1.09 (t, J = 7.2 Hz, 3H) | 50a, 34a |
| 172 | 484.4 | δ 8.44 (t, J = 6.0 Hz, 1H), 7.84 (dd, J = 8.0, 1.0 Hz, 1H), 7.64-7.50 (m, 2H), 7.14 (dd, J = 8.0, 6.2 Hz, 1H), 4.31-4.25 (m, 1H), 3.98 (t, J = 10.4 Hz, 1H), 3.64 (s, 2H), 3.39 (dd, J = 10.6, 7.5 Hz, 1H), 3.28-3.22 (m, 2H), 3.20-3.10 (m, 4H), 2.63-2.54 (m, 4H), 1.25 (t, J = 8.2 Hz, 4H), 1.08 (t, J = 7.2 Hz, 3H). | 53a, 34a |
| 173 | 496.3 | δ 10.62 (s, 1H), 8.40 (q, J = 4.6 Hz, 1H), 7.84 (dd, J = 8.0, 1.2 Hz, 1H), 7.63-7.49 (m, 2H), 7.14 (dd, J = 8.0, 6.4 Hz, 1H), 4.35-4.22 (m, 1H), 3.98 (t, J = 10.4 Hz, 1H), 3.64 (s, 2H), 3.43-3.36 (m, 1H), 3.17 (s, 4H), 2.77 (t, J = 7.2 Hz, 3H), 2.56 (d, J = 13.6 Hz, 4H), 1.26 (d, J = 6.8 Hz, 3H). | 64a, 2a |
| 179 | 484.4 | δ 10.54 (s, 1H), 8.38 (q, J = 4.6 Hz, 1H), 7.85-7.82 (m, 1H), 7.60-7.52 (m, 2H), 7.15-7.12 (m, 1H), 4.28-4.25 (m, 1H), 3.98-3.95 (m, 1H), 3.90-3.88 (m, 1H), 3.40-3.37 (m, 1H), 3.14 (s, 4H), 2.75 (d, J = 3.2 Hz, 3H), 2.57-2.50(m, 4H), 1.36-1.34(m, 3H), 1.25-1.22-0.85 (m, 3H). | 65a, 2a |
| 182 | 496.2 | δ 10.58 (s, 1H), 8.34 (d, J = 5.2 Hz, 1H), 7.83 (d, J = 7.2 Hz, 1H), 7.73 (d, J = 8.4 Hz, 1H), 7.56-7.53 (m, 1H), 7.08-7.05 (m, 1H), 3.73 (t, J = 5.2 Hz, 2H), 3.61 (s, 2H), 3.50 (t, J = 5.6 Hz, 2H), 3.16-3.14 (m, 4H), 2.87-2.83 (m, 1H), 2.56-2.52 (m, 4H), 1.84 -1.82 (m, 2H), 0.66-0.64 (m, 4H). | 50a, 38a |
| 183 | 500.2 | δ 8.47 (q, J = 6 Hz, 1H), 7.93 (d, J = 8.0 Hz, 1H), 7.73 (d, J = 8.0 Hz, 1H), 7.65 (d, J = 8.0 Hz, 1H), 7.06 (dd, J = 8.0, 6.4 Hz, 1H), 3.78-3.75 (m, 2H), 3.64 (s, 2H), 3.50 (t, J = 5.2 Hz, 2H), 3.28-3.26 (m, 2H), 3.10-3.08 (m, 4H), 2.59-2.66 (m, 4H), 1.84 -1.82 (m, 2H), 1.10 (t, J = 7.2 Hz, 3H). | 50a, 62a |
| 184 | 480.3 | δ 8.44 (q, J = 6.0 Hz, 1H), 7.93 (d, J = 8.4 Hz, 1H), 7.73 (d, J = 8.4 Hz, 1H), 7.47 (d, J = 8.4 Hz, 1H), 7.09-7.06 (m, 1H), 3.78-3.75 (m, 2H), 3.64 (s, 2H), 3.50 (t, J = 4.8 Hz, 2H), 3.28-3.26 (m, 2H), 2.96-2.93 (m, 4H), 2.60-2.58 (m, 4H), 2.56 (s, 3H), 1.84 -1.82 (m, 2H), 1.10 (t, J = 7.2 Hz, 3H). | 50a, 61a |
| 185 | 500.3 | δ 10.61 (s, 1H), 8.47 (t, J = 6.1 Hz, 1H), 7.94 (d, J = 8.2 Hz, 1H), 7.66 (d, J = 8.2 Hz, 1H), 7.60 (d, J = 8.1 Hz, 1H), 7.14 (dd, J = 8.1, 6.1 Hz, 1H), 4.34-4.23 (m, 1H), 3.98 (t, J = 10.3 Hz, 1H), 3.66 (s, 2H), 3.40 (dd, J = 10.6, 7.4 Hz, 1H), 3.28 (td, J = 7.2, 6.0 Hz, 2H), 3.11 (m, 4H), 2.65 (m, 4H), 1.26 (d, J = 6.6 Hz, 3H), 1.10 (t, J = 7.2 Hz, 3H). | 53a, 62a |
| 186 | 520.0 | δ 11.15 (s, 1H), 8.43 (t, J = 6.0 Hz, 1H), 7.85-7.78 (m, 2H), 7.58-7.53 (m, 1H), 7.18-7.14(m, 1H), 4.12 (t, J = 12.4 Hz,2H), 3.91 (t, 12.8 Hz,2H), 3.65 (s, 2H), 3.30-3.23 (m, 2H), 3.16 (s, 4H), 2.58 (s, 4H).1.06 (t, J = 6.8 Hz, 3H). | 57a, 34a |
| 187 | 420.3 | δ 10.60 (s, 1H), 8.47-8.46 (m, 1H), 7.83 (dd, J = 9.0, 2.4 Hz, 1H), 7.59 (d, J = 7.8 Hz, 1H), 7.15-7.12 (m, 1H), 6.92 (d, J = 9.0 Hz, 1H), 4.30-4.26 (m, 1H), 3.97 (t, J = 10.2 Hz, 1H), 3.67-3.66 (m, 4H), 3.61 (s, 2H), 3.41-3.37 (m, 1H), 2.47 (t, J = 4.8 Hz, 4H), 1.26 (d, J = 6.6 Hz, 3H). | 53a, 24a |
| 188 | 420.3 | δ 10.48 (s, 1H), 8.46 (d, J = 2.4 Hz, 1H), 7.85-7.82 (m, 1H), 7.72 (d, J = 8.4 Hz, 1H), 7.07 (t, J = 7.2 Hz, 1H), 6.91 (d, J = 9.2 Hz, 1H), 3.76 (t, J = 5.2 Hz, 2H), 3.66-3.64 (m, 4H), 3.59 (s, 2H), 3.50 (t, J = 5.2 Hz, 2H), 2.46 (t, J = 4.8 Hz, 4H), 1.84-1.82 (m, 2H). | 50a, 24a |
| 189 | 468.3 | δ 10.50 (s, 1H), 8.44 (d, J = 4.4 Hz, 1H), 7.94 (d, J = 8.0 Hz, 1H), 7.75 (d, J = 8.0 Hz, 1H), 7.66 (d, J = 8.4 Hz, 1H), 7.07 (d, J = 8.4 Hz, 2H), 4.27-4.23 (m, 1H), 3.98-3.93 (m, 1H), 3.56 (s, 3H), 3.12 (s, 4H), 2.80 (d, J = 4.8 Hz, 3H), 2.57 (s, 4H), 1.25 (d, J = 6.8 Hz, 3H). | 66a, 36a |
| 190 | 448.4 | δ 10.49 (s, 1H), 8.43 (d, J = 4.8 Hz, 1H), 7.80 (d, J = 8.0 Hz, 1H), 7.75 (d, J = 8.4 Hz, 1H), 7.48 (d, J = 8.4 Hz, 1H), 7.07 (d, J = 6.4 Hz, 2H), 4.27-4.23 (m, 1H), 3.98-3.93 (m, 1H), 3.56 (s, 3H), 2.95 (s, 4H), 2.81 (d, J = 4.4 Hz, 3H), 2.57 (s, 4H), 2.49 (s, 3H), 1.25 (d, J = 6.8 Hz, 3H). | 66a, 37a |
| 191 | 468.2 | δ 10.58 (s, 1H), 8.43-8.39 (m, 1H), 7.93 (d, J=8.4 Hz, 1H), 7.89 (d, J=8.4 Hz, 1H), 7.66 (d, J=8.4 Hz, 1H), 7.01 (d, J=8.4 Hz, 1H), 6.98 (s, 1H), 3.78-3.73 (m, 2H), 3.54 (s, 2H), 3.50-3.48 (m, 2H), 3.11 (s, 4H), 2.79 (d, J=4.8 Hz, 3H), 2.56 (s, 4H), 1.83-1.79 (m, 2H). | 67a, 36a |
| 192 | 448.3 | δ 10.58 (s, 1H), 8.43 (s, 1H), 7.89 (dd, J=2.8, 8.0 Hz, 1H), 7.80 (dd, J=2.8, 8.4 Hz, 1H), 7.48 (dd, J=2.8, 8.0 Hz, 1H), 7.02-7.00 (m, 2H), 3.75 (s, 2H), 3.54 (s, 2H), 3.48 (s, 2H), 2.94 (s, 4H), 2.82 (d, J=4.0 Hz, 3H), 2.56-2.48 (m, 7H), 1.82 (s, 2H). | 67a, 37a |
| 193 | 452.4 | δ 10.60 (s, 1H), 8.39 (q, J = 4.7 Hz, 1H), 8.26 (d, J = 2.9 Hz, 1H), 7.82 (d, J = 8.8 Hz, 1H), 7.59 (d, J = 8.1 Hz, 1H), 7.38 (dd, J = 8.8, 2.9 Hz, 1H), 7.14 (dd, J = 8.1, 6.2 Hz, 1H), 4.35-4.21 (m, 1H), 3.98 (t, J = 10.4 Hz, 1H), 3.63 (s, 2H), 3.39 (dd, J = 10.7, 7.4 Hz, 1H), 3.34 (s, 4H), 2.78 (d, J = 4.9 Hz, 3H), 2.56 (t, J = 5.1 Hz, 4H), 1.26 (d, J = 6.6 Hz, 3H). | 53a, 68a |
| 194 | 485.2 | δ 10.57 (s, 1H), 8.55 (q, J = 4.4 Hz, 1H), 7.96 (d, J = 8.0 Hz, 1H), 7.96 (d, J = 8.0 Hz, 1H), 7.59 (d, J = 8.0 Hz, 1H), 7.15-7.11 (m, 1H), 4.31-4.25 (m, 1H), 3.97 (t, J = 10.4 Hz, 1H), 3.61 (s, 2H), 3.41-3.37 (m, 1H), 2.97-2.86 (m, 3H), 2.79 (d, J = 4.8 Hz, 3H), 2.18-2.13 (m, 2H), 1.80-1.64 (m, 4H), 1.26 (d, J = 6.8 Hz, 3H). | 53a, 70a |
| 195 | 430.1 | δ 12.54 (s, 1H), 12.23 (s, 1H), 8.66 (d, J = 7.6Hz, 1H), 7.81 (d, J = 1.6Hz, 1H), 7.31-7.22 (m, 1H), 7.18-7.12 (m, 1H), 7.05-7.01 (m, 1H), 4.55 (s, 2H), 4.00 (s, 2H), 3.61 (s, 2H), 3.50-3.37 (m, 8H), 2.12 (s, 2H). | 50a, 25a |
| 197 | 500.3 | δ 10.56 (s, 1H), 8.43-8.41 (m, 1H), 7.93 (d, J = 8.4 Hz, 1H), 7.65-7.58 (m, 2H), 7.18-7.14 (m, 1H), 4.29-4.25 (m, 1H), 4.03-3.95(m, 2H),3.44-3.37 (m, 2H), 3.28-3.22 (m, 2H), 2.89-2.84 (m, 1H), 2.79(d, J = 4.8 Hz, 3H), 2.76-2.66 (m, 3H), 2.40-2.35(m, 1H), 1.26 (d, J = 6.4 Hz, 3H), 1.26 (d, J = 6.0 Hz, 3H). | 53a, 71a |
| 198 | 536.2 | δ 10.56 (s, 1H), 8.70-8.74 (m, 1H), 7.79 (d, J=8.0 Hz, 1H), 7.68 (d, J = 8.4 Hz, 1H), 7.60 (d, J = 8.0 Hz, 1H), 7.16-7.12 (m, 1H), 6.27-5.97 (m, 1H), 4.29-4.27 (m, 1H),4.00-3.95 (m, 1H), 3.69-3.64 (m, 4H), 3.42-3.37(m, 1H), 3.12 (s, 4H), 2.60(s, 4H), 1.26 (d, J=6.4 Hz, 3H). | 53a, 72a |
| 199 | 453.2 | δ 10.48 (s, 1H), 8.57-8.56 (m, 1H), 8.22 (d, J=3.0 Hz, 1H), 7.94 (d, J = 9.0 Hz, 1H), 7.57 (d, J = 8.4 Hz, 1H), 7.44-7.42 (m, 1H), 7.11-7.09 (m, 1H), 4.59-4.56 (m, 1H), 4.30-4.26 (m, 1H), 3.97 (t, J = 10.2 Hz, 1H), 3.86-3.81 (m, 2H), 3.62 (d, J= 13.2 Hz, 1H), 3.40-3.32 (m, 2H), 2.79-2.76 (m, 4H), 1.26 (d, J=6.6 Hz, 3H), 1.20 (d, J=6.6 Hz, 3H). | 53a, 74a |
| 200 | 430.2 | δ 10.59 (s, 1H), 7.59 (d, J = 8.0 Hz, 1H), 7.17-6.97 (m, 4H), 4.31-4.23 (m, 1H), 4.00-3.95 (m, 1H), 3.62 (s, 2H), 3.41-3.37 (m, 1H), 2.96 (s, 4H), 2.56 (s, 4H), 1.26 (d, J = 6.4 Hz, 3H). | 53a, 25a |
| 201 | 512.3 | δ 8.33 (d, J = 8.4 Hz, 1H), 7.97 (d, J = 8.4 Hz, 1H), 7.85-7.83 (m, 1H), 7.75 (d, J = 8.0 Hz, 1H), 4.71-4.65 (m, 3H), 4.46-4.40 (m, 1H), 3.91-3.86 (m, 1H), 3.30-3.39 (m, 8H), 2.88-2.83 (m, 1H), 1.48 (d, J = 6.4 Hz, 3H), 0.71-0.66 (m, 4H). | 53a, 63a |
| 202 | 512.2 | δ 12.53 (s, 1H), 11.93 (s, 1H), 8.55-8.41 (m, 3H), 7.96-7.94 (d, J = 8.0Hz, 1H), 7.74-7.71 (m, 1H), 4.00 (s, 2H), 3.62 (s, 4H), 3.51 (s, 8H), 2.87 (s, 1H), 2.12 (s, 2H), 0.72-0.67 (m, 4H). | 50a, 63a |
| 203 | 502.3 | δ 10.61 (s, 1H), 8.40 (d, J = 4.8 Hz, 1H), 8.09 (d, J = 8.4 Hz, 1H), 7.86 (d, 8.8 Hz, 1H), 7.60 (d, 8.0 Hz, 1H), 7.27-7.00 (m, 2H), 4.31-4.25 (m, 1H), 4.01-3.95 (m, 1H), 3.66 (s, 2H), 3.42-3.37 (m, 1H), 3.01 (s, 4H), 2.83 (d, J = 4.8 Hz, 3H), 2.62 (s, 4H), 1.26(d, J = 6.4 Hz, 3H). | 53a, 73a |
| 204 | 502.2 | δ 12.58 (s, 1H), 12.17 (s, 1H), 8.64 (d, 8.8 Hz, 1H), 8.47-8.43 (m, 1H), 8.15-8.11 (m, 1H), 7.92 (d, 8.8 Hz, 1H), 7.81 (s, 1H), 7.38-7.12 (m, 1H), 4.57 (s, 2H), 4.00 (t, J = 5.2 Hz, 2H), 3.62 (d, J = 4.8 Hz, 2H), 3.32-3.26 (m, 8H), 2.84 (d, J = 4.8 Hz, 3H), 2.12 (d, J = 4.4 Hz, 2H). | 50a, 73a |

| Example | LCMS (ESI): [M+H]⁺ *m*/*z* | Example | LCMS (ESI): [M+H]⁺ *m*/*z* | Example | LCMS (ESI): [M+H]⁺ *m*/*z* | Example | LCMS (ESI): [M+H]⁺ *m*/*z* |
|---|---|---|---|---|---|---|---|
| 161 | 482.2 | 163 | 484.2 | 165 | 471.1 | 166 | 502.2 |
| 167 | 471.1 | 168 | 489.2 | 169 | 486.2 | 170 | 500.1 |
| 174 | 496.2 | 175 | 496.2 | 176 | 520.2 | 177 | 520.2 |
| 178 | 520.2 | 180 | 484.4 | 181 | 484.4 | 196 | 500.2 |

The present invention is illustrated in detail by specific embodiments and with reference to the data. It should be understood that these embodiments are intended to exemplify the invention only and are intended to illustrate specific combinations, methods of preparation, and the functions and effects thereof, and are not intended to limit the scope of the invention in any way. The beneficial effects of the pharmaceutical combinations of the present invention can also be determined by other test models known to those skilled in the relevant field.

### Biological Tests

### Example 1: Assessment of PARP1/2 inhibitory activity

The PARP1/2 inhibitory activity of the compounds of the present invention was tested in an assay using Histone as a substrate.

Aim of the assay: to test the PARP1/2 enzymatic activity inhibition IC₅₀ values of the compounds of the present application according to established experimental methods. AZD-2281 (Olaparib) was used as a positive control.

### Experimental Reagents:

Recombinant human PARP1 protein (Abcam, cat. ab279663); recombinant human PARP2 protein (BPS, cat. 80502); recombinant histone H1 (Active Motif, cat. 81126); NAD+, Biotin-Labelled (BPS, cat. 80610); SuperBlock (TBS) Blocking Buffer (Thermo Scientific^{™}, cat. 37535); Streptavidin (HRP) (Abcam, cat. ab7403); LumiGLO^{®} Peroxidase Chemiluminescent Substrate Kit (Seracare, cat. 5430-0040); 20xPBS (CST, cat. 9808S); 20xPBST (CST, cat. 9809S); AZD2281(Selleck, cat. S1060)

### Experimental method I: PARP1 inhibitory activity

1. Compound Configuration: Dilute the compounds with DMSO in 384-well plates to a 1000-fold final concentration and set aside.

### 2. Microplate Coating:

1) Dilute Histone with PBS, add 25uL of Histone mixture to each well and incubate for 2 h. 2) Wash each well 5 times using PBST solution. Remove the solution with a clean paper towel.3) Add 75 uL of Blocking buffer to each well and incubate for 1 h at rt. 4) Wash each well 5 times using PBST solution. Remove the solution with a clean paper towel.

### 3. Ribosylation reaction:

1) Transfer 25 nL of 1000× final concentration of compound to a 384 reaction plate; add 25 nL of 100% DMSO to Min control wells and Max control wells. 2) Use 1× Assay buffer to prepare 2.5× final concentration of PARP1 solution. 3) Add 10 uL of enzyme solution to the compound wells and Max control wells; add 10 uL of 1× Assay buffer. 4) Centrifuge at 1000 rpm for 60 s and incubate at rt for 15 min. 5) Prepare 1.67× final concentration of substrate solution with 1× Assay buffer, add 500 µM NAD+ to the substrate solution, and add 15 µL of substrate solution to each well to start the reaction. 6) Centrifuge at 1000 rpm for 60 seconds and incubate at rt for 2 h. 7) Each well was washed 5 times with PBST solution. Remove the solution with a clean paper towel.

### 4. Assay:

1) Prepare Streptavidin-HRP solution, add 25 µL to each well, centrifuge at 1000 rpm for 60 sec, and incubate at rt for 30 min. 2) Wash each well 5 times using PBST solution. Remove the solution with a clean paper towel. 3) Add 50 µL of ELISA Chemiluminescent Substrate per well. 4) Centrifuge at 1000 rpm for 60 seconds and read with EnSight after 5 min.

### 5. Data analysis:

The inhibition rate was calculated using the following formula: % inhibition = (max signal - compound signal) / (max signal - min signal) × 100, where 'min signal' is the mean value of negative control wells and 'max signal' is the mean value of positive control wells.

Fitting the quantitative effect curve: using the log value of the concentration as the X-axis and the percentage inhibition as the Y-axis; using the log(inhibitor) vs. response-variable slope method in software GraphPadPrism5 to fit the quantitative effect curve to calculate the IC₅₀ value for the inhibition of the enzyme activity by the compounds of the present invention. The fitting formula is: Y=Bottom+ (Top-Bottom)/(1+10^((logIC₅₀-X)*HillSlope)).

### Experimental method II: PARP2 inhibitory activity

The PARP2 inhibitory activity assay was performed as in Experimental Method 1, except that PARP1 solution in step 2 of "3. Ribosylation reaction" was replaced with recombinant human PARP2 solution, and 500 µM NAD+ was not added in step 5 of the ribosylation reaction.

**Table 1. IC₅₀ for inhibition of PARP1/2 enzymes by compounds of the present invention**

| Compound | PARP1 IC₅₀ (nM) | PARP2 IC₅₀ (nM) | PARP1/2 Fold selectivity | Compound | PARP1 IC₅₀ (nM) | PARP2 IC₅₀ (nM) | PARP1/2 Fold selectivity |
|---|---|---|---|---|---|---|---|
| 4 | 0.33 | 42.7 | 129.4 | 133 | 1.2 | / | / |
| 5 | 0.15 | 17.3 | 115.3 | 134 | 2.8 | 3677 | 1313.2 |
| 6 | 0.42 | 21.7 | 51.7 | 135 | 9.6 | / | / |
| 7 | 0.38 | 203.8 | 536.3 | 136 | 11.3 | 8871 | 785 |
| 8 | 2.04 | 118.2 | 57.9 | 137 | 1.8 | 1399 | 466.3 |
| 12 | 0.57 | 213.7 | 374.9 | 138 | 0.7 | 49.6 | 70.9 |
| 13 | 2.87 | 3153 | 1098.6 | 139 | 1.0 | 1275 | 1275 |
| 15 | 3.6 | 312.5 | 86.8 | 140 | 1.5 | 2754 | 1836 |
| 16 | 0.22 | 9.9 | 45 | 141 | 1.3 | 1948 | 1498.5 |
| 17 | 3.68 | 624.2 | 169.6 | 142 | 5.6 | >10000 | >1756 |
| 19 | 273 | / | / | 143 | 4.6 | / | / |
| 21 | 2.75 | 3443 | 1250 | 144 | 5.2 | >10000 | >1923 |
| 22 | 0.8 | 358.1 | 447.6 | 145 | 0.6 | 1298 | 2163 |
| 26 | 17.7 | >10000 | >565 | 146 | 0.6 | 971.2 | 1618.7 |
| 34 | 1.83 | 780.3 | 426.4 | 147 | 1.0 | 277.7 | 277.7 |
| 75 | 16.47 | 1869 | 113.5 | 148 | 0.8 | 93.9 | 117.4 |
| 77 | 0.6 | 13.4 | 22.3 | 149 | 1.6 | 892.6 | 557.9 |
| 80 | 0.85 | 45.6 | 53.6 | 150 | 17.6 | >10000 | >568 |
| 98 | 18.4 | / | / | 151 | 1.1 | 4230 | 3845.5 |
| 99 | 571 | / | / | 152 | 1.5 | / | / |
| 101 | 1.7 | 1002 | 589.4 | 153 | 1.3 | 1355 | 1042 |
| 102 | 1.0 | 521.6 | 521.6 | 154 | 1.6 | 1131 | 706.9 |
| 103 | 0.9 | 829.2 | 922.1 | 155 | 1.46 | 892.6 | 611.4 |
| 112 | 14.2 | / | / | 156 | 1.9 | 4596 | 2418.9 |
| 113 | 0.8 | 1301.0 | 1626.3 | 157 | 0.5 | 201.7 | 403.4 |
| 117 | 0.5 | / | / | 158 | 0.48 | 513.4 | 1069.6 |
| 123 | 0.8 | 1429 | 1786 | 159 | 0.47 | 97.5 | 207.4 |
| 124 | 1.07 | 561.3 | 524.6 | 160 | 0.59 | 246.3 | 417.5 |
| 125 | 0.9 | 1057 | 1174 | 162 | 6.28 | >1000 | >159 |
| 126 | 1.3 | 3466 | 2666 | 164 | 44.9 | 2521 | 56 |
| 127 | 7.3 | 501.7 | 68.7 | 171 | 0.7 | 1075 | 1535.7 |
| 128 | 2.2 | 1544 | 701.8 | 172 | 0.7 | 548.6 | 783.7 |
| 129 | 1.7 | 6073 | 3572 | 173 | 0.95 | 514.4 | 541.5 |
| 130 | 3.4 | / | / | 179 | 0.84 | 952.5 | 1133.9 |
| 131 | 1.2 | 1277 | 1064 | 182 | 1.83 | 546.8 | 299 |
| 132 | 1.5 | 287.7 | 191.8 | 193 | 1.7 | 309 | 180 |
| 183 | 0.36 | 878.8 | 2441 | 194 | 2.2 | 3339 | 1522 |
| 184 | 1.13 | 1563 | 1383 | 195 | 40.8 | >10000 | >245 |
| 185 | 1.09 | 894 | 820.2 | 197 | 0.3 | 1815 | 5209 |
| 186 | 0.58 | 113.1 | 195 | 198 | 0.5 | 1240 | 2551 |
| 187 | 0.95 | 649.8 | 685.4 | 199 | 0.6 | 1157 | 1899 |
| 188 | 1.56 | 728.3 | 466.3 | 200 | 19.3 | 4940 | 256 |
| 189 | 0.67 | 215.6 | 321 | 201 | 1.2 | 1555 | 1324 |
| 190 | 0.34 | 207 | 605.6 | 202 | 1.6 | 2867 | 1752 |
| 191 | 0.9 | 296 | 323 | 203 | 1.4 | 1892 | 1355 |
| 192 | 1.0 | 534.3 | 541 | 204 | 1.5 | 3220 | 2090 |
| | | | | AZD-2281 | 26.85 | 0.35 | 0.013 |

| | | | | | | | |
|---|---|---|---|---|---|---|---|
| Note: '/' indicates that the data were not tested. | | | | | | | |

Based on the results in Table 1, it is evident that compounds of the present invention are highly selective for PARP1 and may reduce the toxicity produced by PARP2 without significantly sacrificing the efficacy. The inventors also surprisedly found that the compounds of the present invention have good physical and chemical stability, good bioavailability (e.g., low clearance) and good druggability. Thus, the compounds of the present invention have fewer side effects than olaparib (AZD-2281) and have high values in clinic..

### Example 2: MDA-MB-436 cell proliferation inhibition test

Human breast cancer MDA-MB-436 (purchased from ATCC) cells were cultured in DMEM medium (supplemented with 10% fetal bovine serum with 1% dual antibody) at 37 °C, 5% CO₂. The cells were taken from the logarithmic growth phase, digested and prepare cell suspension witha certain concentration, which was inoculated into 96-well plates. 100 µL of cell suspension was added to each well of the 96-well plates, incubated overnight, then different concentrations of compounds were added, and the cells were incubated in the cell culture incubator for 7 days. At the end of incubation, 50 µL of CellTiter-Glo reagent was added to each well, mixed with a microplate and shaked for 2 min, and left at rt for 60 min, then the fluorescence value was read with a Spark^{®} multimode microplate reader. The cell proliferation inhibition percentage was calculated according to the formula: [(1-(RLU_{compound}- RLU_{blank})/(RLU_{control}-RLU_{blank})) × 100%],and IC₅₀ values were fitted by using GraphPad Prism 6.0 software.

**Table 2. proliferation inhibitory activity of compounds of the present invention on MDA-MB-436 cells**

| Compound | MDA-MB-436 IC₅₀ (nM) | Compound | MDA-MB-436 IC₅₀ (nM) | Compound | MDA-MB-436 IC₅₀ (nM) |
|---|---|---|---|---|---|
| 12 | <1.52 | 22 | 4.6 | 101 | 9.3 |
| 102 | 3.7 | 103 | 1.4 | 113 | <1.52 |
| 123 | 27.4 | 126 | 5.4 | 129 | <1.52 |
| 131 | 15.3 | 132 | 12.1 | 133 | 28.7 |
| 137 | 22.6 | 138 | 3.0 | 139 | 10.4 |
| 140 | 12.8 | 141 | 5.8 | 145 | 8.9 |
| 146 | 12.4 | 147 | 2.1 | 148 | 3.6 |
| 151 | 17.5 | 152 | 21.5 | 153 | 8.1 |
| 154 | 0.33 | 156 | 25.0 | 157 | 3.6 |
| 158 | 2.2 | 159 | 2.2 | 160 | 5.7 |
| 172 | 5.7 | 183 | 9.2 | 186 | 6.4 |
| AZD-9574 | 15.7 | | | | |

The experimental results show that the compounds of the present invention have significant proliferation inhibitory activity against MDA-MB-436 cells.

### Example 3: MDR1-MDCK II cell model to assess bidirectional permeability

MDR1-MDCK II cells were inoculated into 96-well plates at a concentration of 3.3 × 10⁵ cells/mL and grown for 4-7 days to form a monolayer of fused cells. 2 µM of the test compound was added to the wells at the apical side or the basolateral side of the monolayer of cells and incubated for 2.5 h at 37.0°C in a 5.0% CO₂ incubator. Cell monolayer integrity was determined by Lucifer yellowrejection assay. The buffer was removed from the apical side and basolateral side, and the concentration of the subject compounds was determined using LC-MS/MS. The concentration data were used to calculate the apparent permeabilitycoefficients of apical-to-basolateral (A-B) and basolateral-to-apical (B-A)side, and to calculate efflux ratio with the formula: Efflux Ratio = Papp (B-A) / Papp (A-B)

**Table 3. bidirectional permeability of MDR1-MDCK II cells for compounds of the present invention**

| Compound | MDR1-MDCK ∥ Papp (10⁻⁶ cm/s) (A-B) | MDR1-MDCK ∥ Papp (10⁻⁶ cm/s) (B-A) | ER ratio |
|---|---|---|---|
| 132 | 12.8 | 26.8 | 2.1 |
| 138 | 14.7 | 17.0 | 1.15 |
| 139 | 16.7 | 21.8 | 1.31 |
| 141 | 16.2 | 23.1 | 1.42 |
| 154 | 13.9 | 22.8 | 1.64 |
| 156 | 16.3 | 21.1 | 1.29 |
| 157 | 15.1 | 21.6 | 1.40 |
| 158 | 14.7 | 14.1 | 0.96 |
| 160 | 14.3 | 8.78 | 0.61 |
| AZD-9574 | 19.4 | 19.4 | 1.0 |

The experimental results show that the compounds of the present invention have high cell permeability and low efflux rates in MDR1-MDCK II cells.

### Example 4: Preliminary Pharmacokinetic Tests

1. Healthy ICR mice were administered the tested compounds intravenously (1 mg/kg) or by gavage (5 mg/kg). Nine male mice weighing 30-35 g for each route of administration were randomly divided into three groups of three mice each.

The mice were fasted for 12h before the test and allowed to drink water freely. The mice were fed 4h after the administration.

### 2. Blood collection time point and sample processing

Intravenous and gavage administration: 0.25h, 0.5h, 1.0h, 2.0h, 3.0h, 4.0h, 6.0h, 8.0h and 24h after administration.

Blood was collected continuously, and 3 animals were collected at each time point. Plasma collection and processing: 30-40 µL of venous blood was collected from the mouse retroorbital venous plexus at the above set time points, placed in an EDTA-K2 test tube, centrifuged at 3500 rpm for 10 min, and plasma was separated and frozen in a -20 °C refrigerator.

### 3. Sample testing and data analysis

The concentration of the compounds in mouse plasma was determined by LC/MS/MS. Pharmacokinetic parameters after administration were calculated using the non-atrial model of Phoenix 8.3 software (Pharsight, USA).

### 4. Experimental results

**Table 4. pharmacokinetic parameters of the compounds of the invention in mouse plasma**

| Compound | Method of administration | T_{1/2} (hr) | CL (mL/min/kg) | AUC₀₋ₜ (ng·hr/mL) | bioavailability (%) |
|---|---|---|---|---|---|
| 132 | i.v. | 3.0 | 2.8 | 6633 | 98.7 |
| | p.o. | 4.4 | / | 33088 | |
| 139 | i.v. | 3.1 | 5.9 | 2824 | 98.0 |
| | p.o. | 3.2 | / | 13839 | |
| 141 | i.v. | 1.2 | 5.5 | 3021 | 92.6 |
| | p.o. | 2.5 | / | 13982 | |
| 154 | i.v. | 1.1 | 2.3 | 7244 | 62.1 |
| | p.o. | 2.3 | / | 22489 | |
| 158 | i.v. | 0.8 | 5.5 | 3087 | 108.5 |
| | p.o. | 1.0 | / | 10977 | |
| 160 | i.v. | 0.86 | 1.6 | 10521 | 70.7 |
| | p.o. | 0.9 | / | 26090 | |

| | | | | | |
|---|---|---|---|---|---|
| Note: i.v.: intravenous (1 mg/kg); p.o.: oral administration (5 mg/kg). | | | | | |

The experimental results show that the compound of the present invention exhibits a long half-life (T1/2), a low clearance rate (CL) and a high bioavailability when orally dosed in mice.

As shown in Table 1, the compounds of the present invention have high activity against PARP1 and high selectivity against PARP2, and can reduce the toxicity produced by PARP2 without significantly sacrificing the efficacy. As shown in Table 2, the compounds of the present invention have strong proliferation inhibitory activity against human breast cancer cells (MDA-MB-436). The inventors also unexpectedly found that the compounds of the present invention have high cell permeability and low efflux rate in MDR1-MDCKII cells. The compounds of the present invention have good physical and chemical stability, good bioavailability (e.g., low clearance rate) and good druggability. Therefore, the compounds of the present invention have fewer side effects than olaparib (AZD-2281) and have high clinical application value.

## Claims

1. A compound of formula (I), or a stereoisomer, a geometric isomer, a tautomer, a pharmaceutically acceptable salt, a crystal form, a solvate, a hydrate or a prodrug thereof, 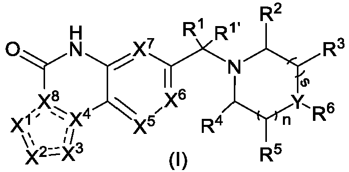 wherein,
X¹ is independently selected from -N-, -NR¹⁴-, -CR⁷-, -CR⁷R^{7'}, -CH₂CR⁷R^{7'}-, - CR⁷R^{7'}-CH₂-, O and S;
X² is independently selected from -N-, -NR¹⁵-, -CR⁸-, -CR⁸R^{8'}-, O and S;
X³ is independently selected from -N-, -NR¹⁶-, -CR⁹-, O and S;
X⁴ and X⁸ are each independently selected from -N- and -C-;
-̅ -̅ -̅ -̅ -̅ -̅ is a single or double bond; and X¹, X², X³, X⁴ together with X⁸ form five-membered heteroaryl, or partially saturated five-membered or six-membered heterocyclyl; wherein, the heteroaryl or the heterocyclyl each independently contains 1, 2 or 3 heteroatoms independently selected from N, O and S;
R⁷, R^{7'}, R⁸, R^{8'}, R⁹ are each independently selected from hydrogen, halogen, hydroxyl, cyano, C₁-C₃ alkoxy, unsubstituted or substituted C₃-C₆ cycloalkyl and unsubstituted or substituted C₁-C₆ alkyl; or, R⁷ together with R^{7'}, or R⁸ together with R^{8'}, form C₃-C₆ cycloalkyl ; R⁷, R^{7'}, R⁸, R^{8'}, R⁹ are each independently preferably hydrogen, halogen or C₁-C₄ alkyl; R⁷, R^{7'}, R⁸, R^{8'}, R⁹ are each independently more preferably hydrogen, F or methyl; or preferably, R⁷ together with R^{7'}, or R⁸ together with R^{8'}, form C₃-C₄ cycloalkyl, such as cyclopropyl or cyclopentyl;
R¹⁴, R¹⁵, R¹⁶ are each independently selected from hydrogen, unsubstituted or substituted C₃-C₆ cycloalkyl, unsubstituted or substituted C₁-C₆ alkyl; R¹⁴, R¹⁵, R¹⁶ are each independently preferably hydrogen or C₁-C₃ alkyl; R¹⁴, R¹⁵, R¹⁶ are each independently more preferably methyl;
X⁵, X⁶ are each independently selected from -N- and -CR¹⁰-; R¹⁰ is selected from hydrogen, halogen, cyano, unsubstituted or substituted C₁-C₆ alkoxy, unsubstituted or substituted C₁-C₆ alkyl; R¹⁰ is preferably hydrogen, halogen, cyano or C₁-C₄ alkyl; R¹⁰ is more preferably hydrogen, fluorine, chlorine or methyl;
X⁷ is -N- or -CR¹⁷-; R¹⁷ is selected from hydrogen, halogen, cyano, unsubstituted or substituted C₁-C₆ alkoxy, unsubstituted or substituted C₁-C₆ alkyl; R¹⁷ is preferably hydrogen, halogen, cyano or C₁-C₄ alkyl; R¹⁷ is more preferably hydrogen, fluorine, chlorine or methyl;
R¹, R^{1'}, R², R³, R⁴, R⁵ are each independently selected from hydrogen, unsubstituted or substituted C₁-C₆ alkyl; or R⁴, R⁵ together with the carbon atoms bound thereto form C₃-C₆ cycloalkyl;
s, n are each independently selected from 0, 1 and 2;
Y is N or CH;
R⁶ is selected from: 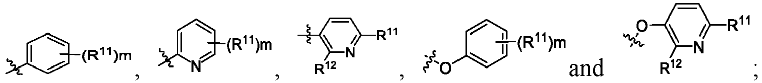
each R¹¹ is independently selected from halogen, cyano, C₁-C₃ alkoxy, carbonyl, - CONHR¹³, amino, preferably selected from halogen, -CONHR¹³ and cyano; more preferably selected from -CONHR¹³;
m is 0, 1, 2 or 3;
R¹² is selected from hydrogen, cyano, halogen, unsubstituted or substituted C₁-C₄ alkyl;
R¹³ is hydrogen, unsubstituted or substituted C₁-C₆ alkyl, unsubstituted or substituted C₃-C₈ cycloalkyl, unsubstituted or substituted C₁-C₆ alkoxy, or unsubstituted or substituted 3-8 membered heterocycloalkyl; preferably, R¹³ is hydrogen, unsubstituted or halogen substituted C₁-C₄ alkyl, C₃-C₆ cycloalkyl or C₁-C₆ alkoxy; the heterocycloalkyl refers to heterocycloalkyl containing 1-3 heteroatoms selected from N, O, and S; preferably, R¹³ is methyl, ethyl, C₂-C₃ alkoxy, cyclopropyl, epoxypropyl, oxetylbutyl, or oxetylpentyl;
wherein, the substitution in R¹, R^{1'}, R², R³, R⁴, R⁵, R⁷, R^{7'}, R⁸, R^{8'}, R⁹, R¹⁰, R¹², R¹³, R¹⁴, R¹⁵, R¹⁶, and R¹⁷ refers to being substituted by one or more selected from C₁-C₄ alkyl, C₁-C₆ alkoxy, halogen, hydroxyl, cyano, amino, carboxyl, and C₃-C₆ cycloalkyl;
provided that:
when X⁵ and X⁸ are both -N-, at least one of X¹, X², X³, and X⁴ is -N-;
when X⁴ and X⁵ are both -N-, X³ is also -N-;
when X³ is oxygen and X¹, X², X⁴, X⁸ are -C- and X⁷ is -CR¹⁷- , R¹, R^{1'}, and R¹⁷ are not hydrogen at the same time;
preferably, provided that:
at least one of X¹, X², X³ is -N- when X⁸ is -N-.

2. The compound as shown in formula (I), or the stereoisomer, the geometric isomer, the tautomer, the pharmaceutically acceptable salt, the crystal form, the solvate, the hydrate, or the prodrug thereof according to claim 1, wherein 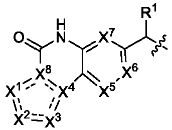 in formula (I) is selected from the following structures: 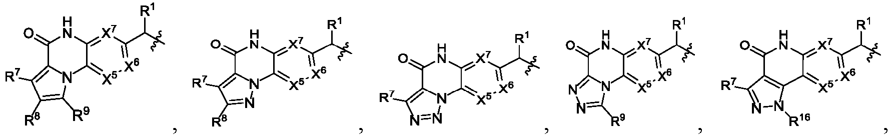 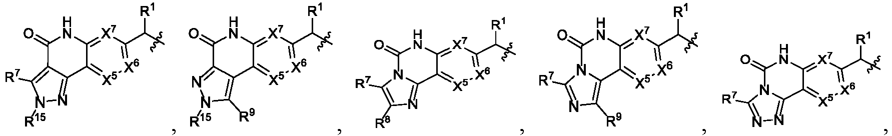 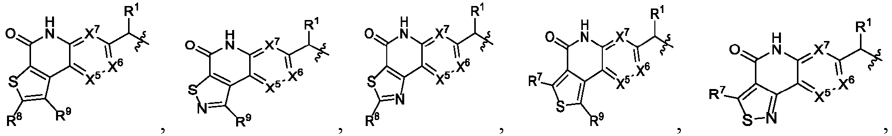 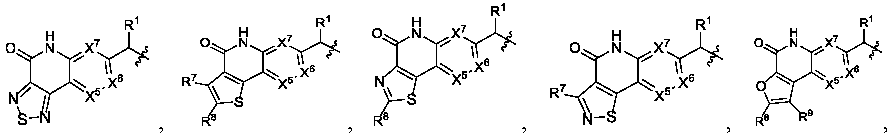 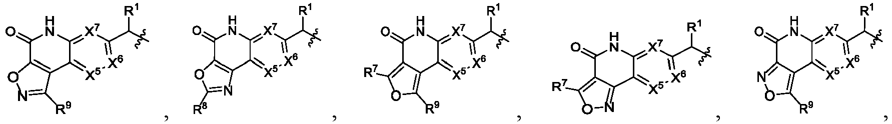 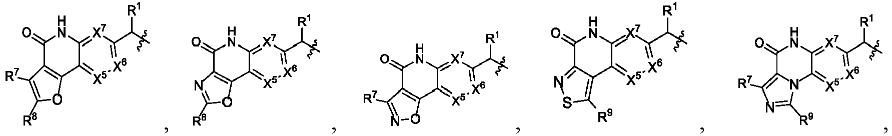 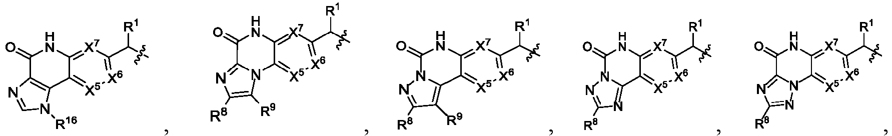 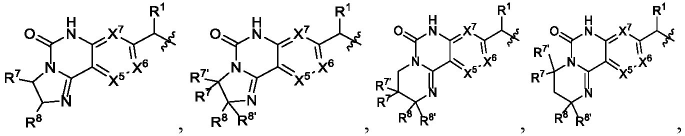 wherein, each substitution defined as said in claim 1.

3. The compound as shown in formula (I), or the stereoisomer, the geometric isomer, the tautomer, the pharmaceutically acceptable salt, the crystal form, the solvate, the hydrate, or the prodrug thereof according to claim 1, wherein, 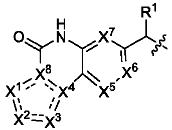 in formula (I) is selected from the following structures: 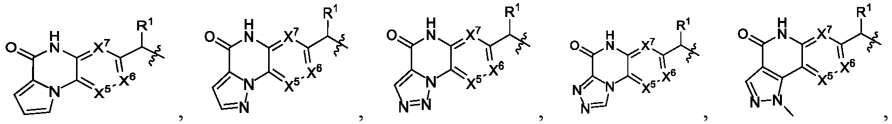 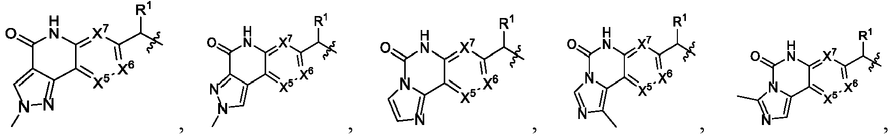 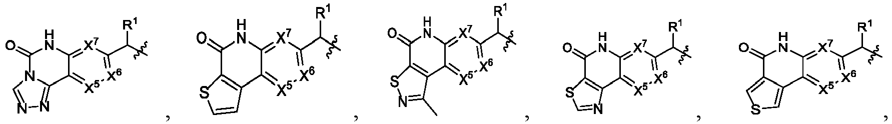 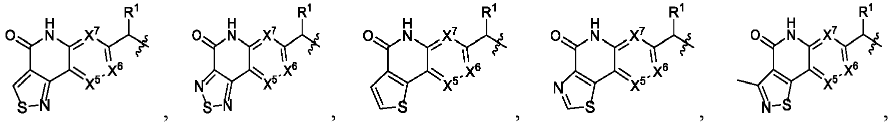 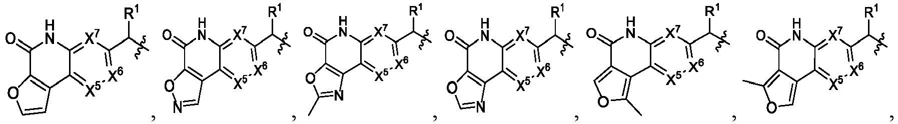 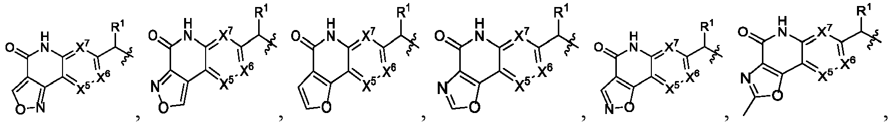 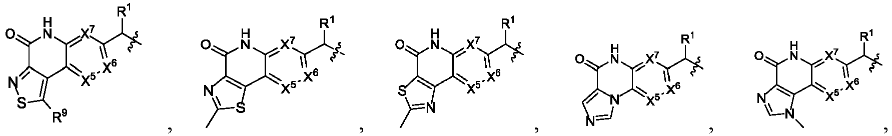 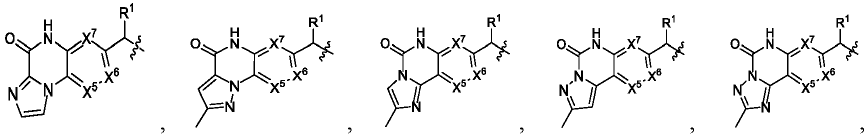 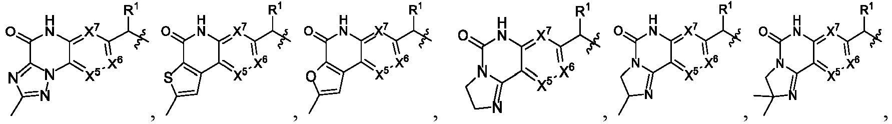 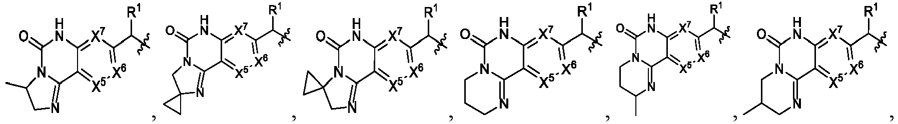 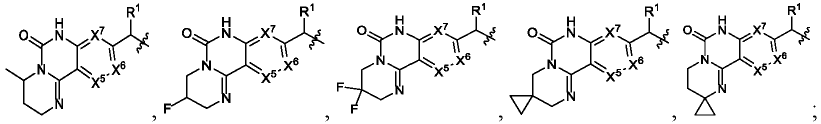 wherein, each substitution defined as said in claim 1.

4. The compound as shown in formula (I), or the stereoisomer, the geometric isomer, the tautomer, the pharmaceutically acceptable salt, the crystal form, the solvate, the hydrate, or the prodrug thereof according to claim 1, wherein 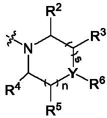 in formula (I) is selected from the following structures: 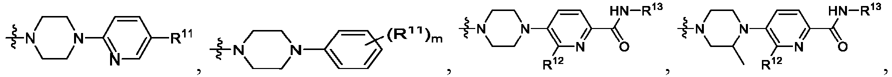 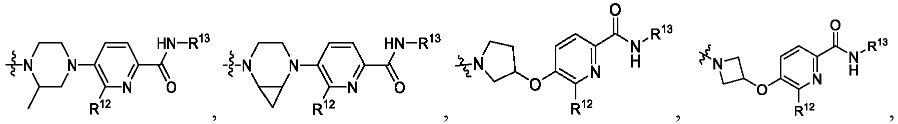 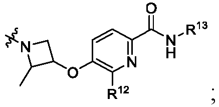 wherein, each substitution defined as said in claim 1.

5. The compound as shown in formula (I), or the stereoisomer, the geometric isomer, the tautomer, the pharmaceutically acceptable salt, the crystal form, the solvate, the hydrate, or the prodrug thereof according to claim 1, wherein 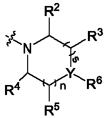 in formula (I) is selected from the following structures: 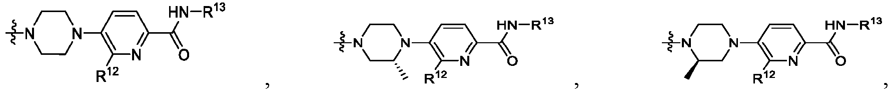 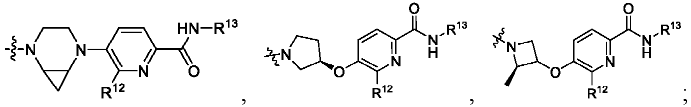 wherein, each substitution defined as said in claim 1.

6. The compound as shown in formula (I), or the stereoisomer, the geometric isomer, the tautomer, the pharmaceutically acceptable salt, the crystal form, the solvate, the hydrate, or the prodrug thereof according to any claim of 1-5, wherein
the compound in formula (I) is selected from the following specific compounds:
| No. | Structure | No. | Structure |
|---|---|---|---|
| 5 | 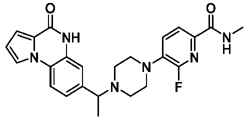 | 4 | 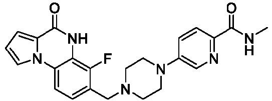 |
| 7 | 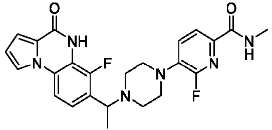 | 6 | 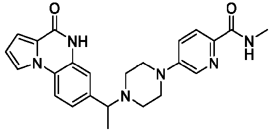 |
| 9 | 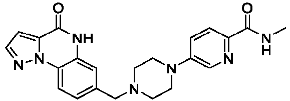 | 8 | 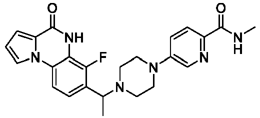 |
| 13 | 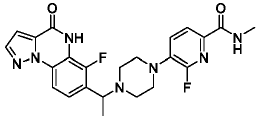 | 12 | 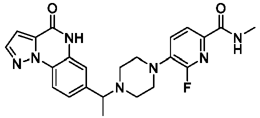 |
| 15 | 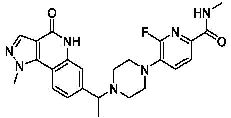 | 16 | 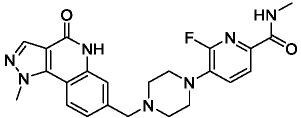 |
| 17 | 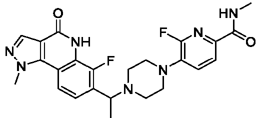 | 19 | 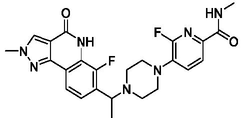 |
| 21 | 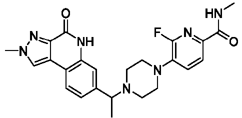 | 22 | 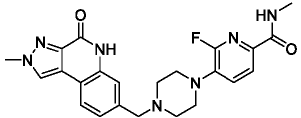 |
| 26 | 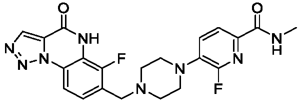 | 34 | 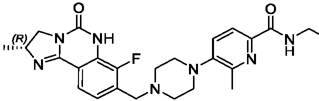 |
| 75 | 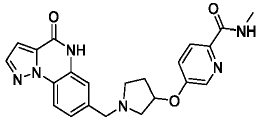 | 77 | 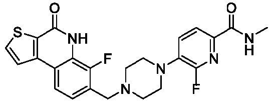 |
| 80 | 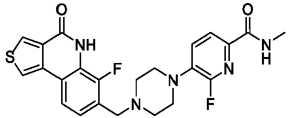 | 98 | 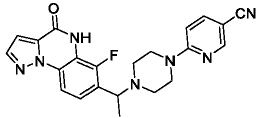 |
| 99 | 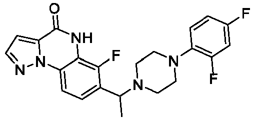 | 102 | 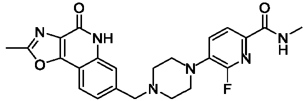 |
| 101 | 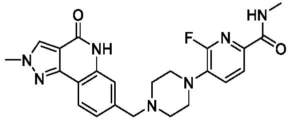 | 106 | 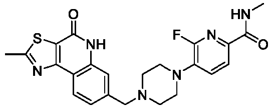 |
| 103 | 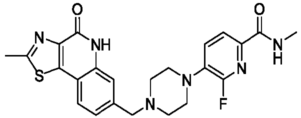 | 112 | 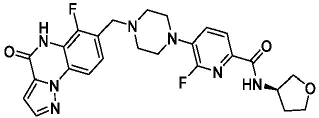 |
| 113 | 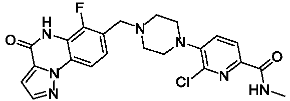 | 117 | 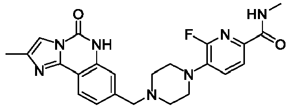 |
| 123 | 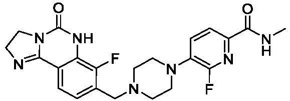 | 124 | 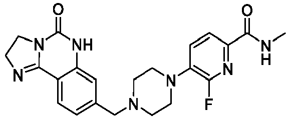 |
| 125 | 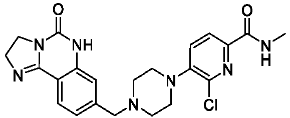 | 126 | 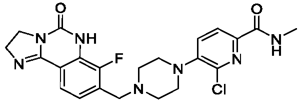 |
| 127 | 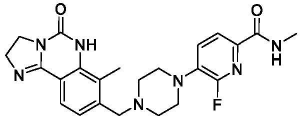 | 128 | 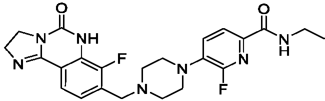 |
| 129 | 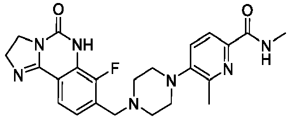 | 130 | 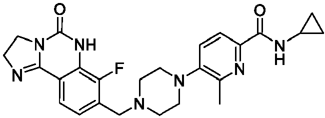 |
| 131 | 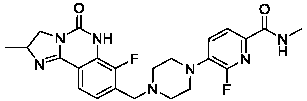 | 132 | 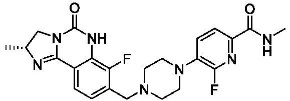 |
| 133 | 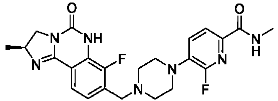 | 134 | 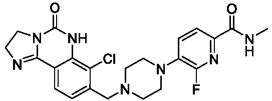 |
| 135 | 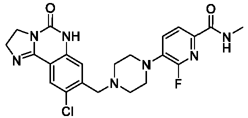 | 136 | 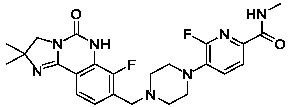 |
| 137 | 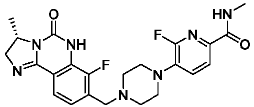 | 138 | 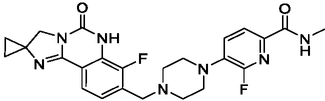 |
| 139 | 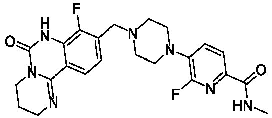 | 140 | 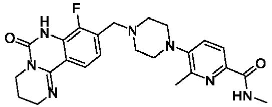 |
| 141 | 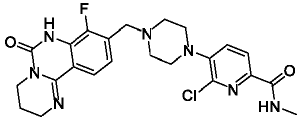 | 142 | 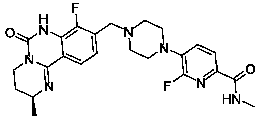 |
| 143 | 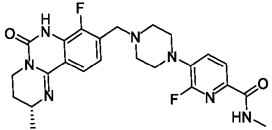 | 144 | 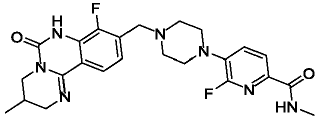 |
| 145 | 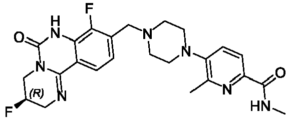 | 146 | 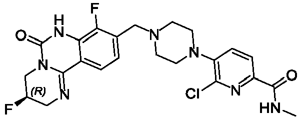 |
| 147 | 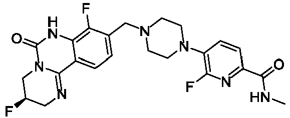 | 148 | 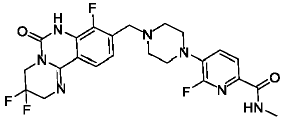 |
| 149 | 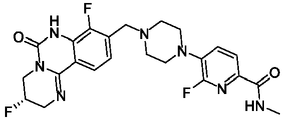 | 150 | 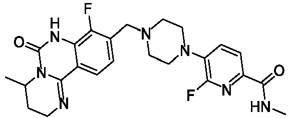 |
| 151 | 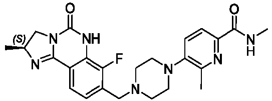 | 152 | 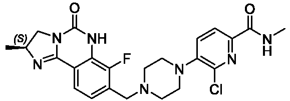 |
| 153 | 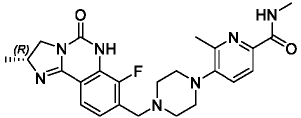 | 154 | 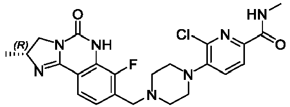 |
| 155 | 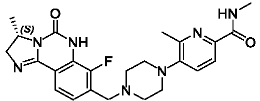 | 156 | 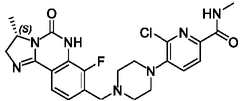 |
| 157 | 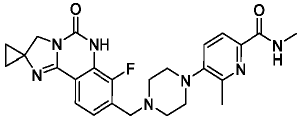 | 158 | 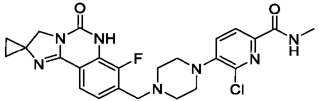 |
| 159 | 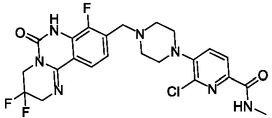 | 160 | 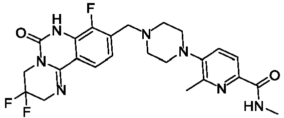 |
| 161 | 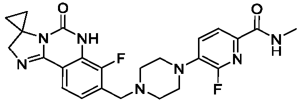 | 162 | 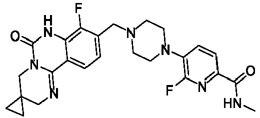 |
| 163 | 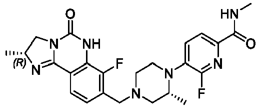 | 164 | 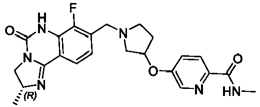 |
| 165 | 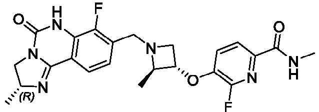 | 166 | 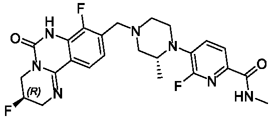 |
| 167 | 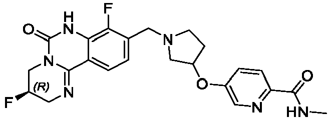 | 168 | 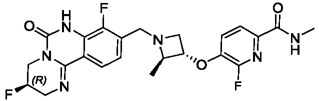 |
| 169 | 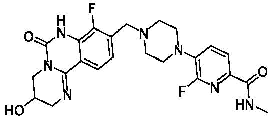 | 170 | 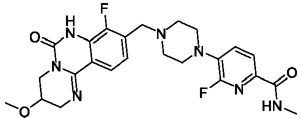 |
| 171 | 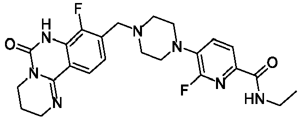 | 172 | 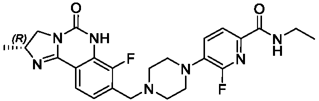 |
| 173 | 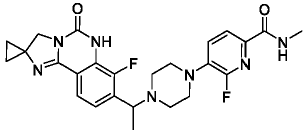 | 174 | 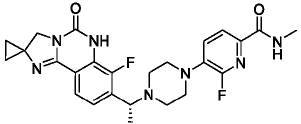 |
| 175 | 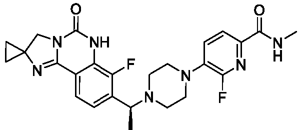 | 176 | 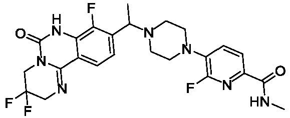 |
| 177 | 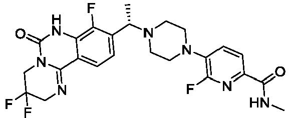 | 178 | 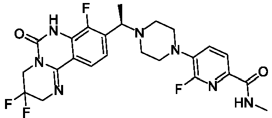 |
| 179 | 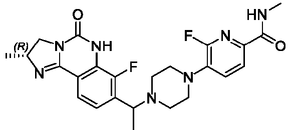 | 180 | 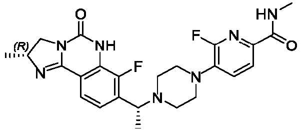 |
| 181 | 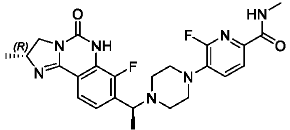 | 182 | 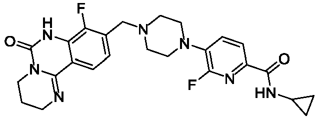 |
| 183 | 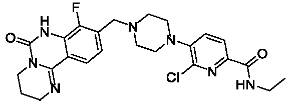 | 184 | 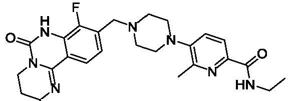 |
| 185 | 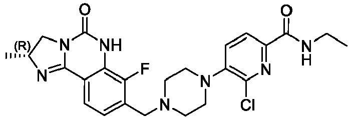 | 186 | 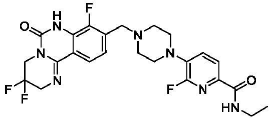 |
| 187 | 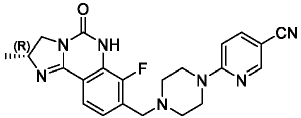 | 188 | 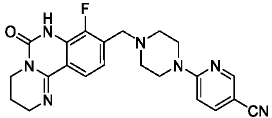 |
| 189 | 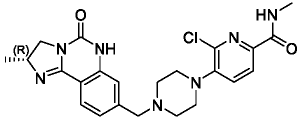 | 190 | 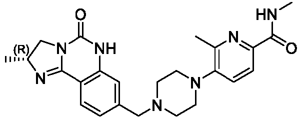 |
| 191 | 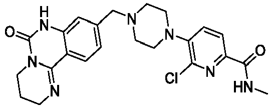 | 192 | 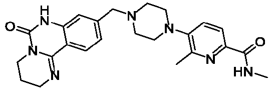 |
| 193 | 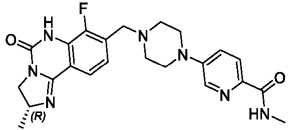 | 194 | 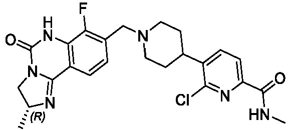 |
| 195 | 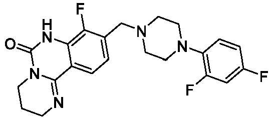 | 196 | 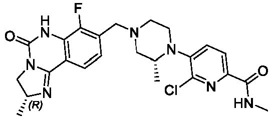 |
| 197 | 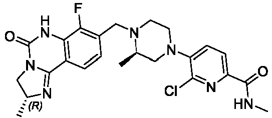 | 198 | 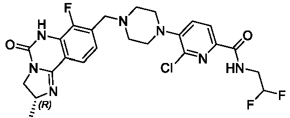 |
| 199 | 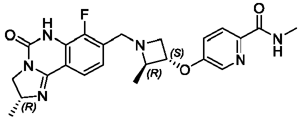 | 200 | 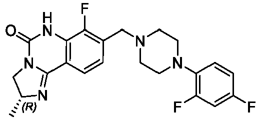 |
| 201 | 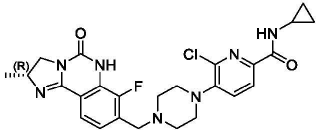 | 202 | 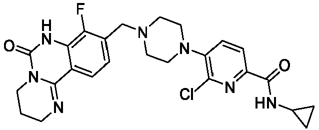 |
| 203 | 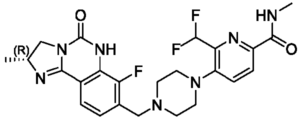 | 204 | 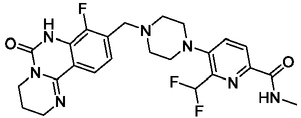 |

7. A pharmaceutical composition comprising a therapeutically effective amount of the compound of formula (I), or the stereoisomer, the geometric isomer, the tautomer, the pharmaceutically acceptable salt, the crystal form, the solvate, the hydrate or the prodrug thereof as said in any claim of 1-6, and a pharmaceutically acceptable carrier.

8. Use of the compound of formula (I), or the stereoisomer, the geometric isomer, the tautomer, the pharmaceutically acceptable salt, the crystal form, the solvate, the hydrate or the prodrug thereof as said in any claim of 1-6, or the pharmaceutical composition as said in claim 7 in the preparation of a drug for preventing, treating or improving a disease by inhibiting PARP1.

9. The use as said in claim 8, wherein
the disease is cancer, the genome of the cancer is a type of homologous recombination repair deficiency,
or, the cancer relies on a pathway where DNA double-strand damage is deficient in homologous recombination repair,
or, the cancer comprises one or more cancer cells that lack the ability to repair DNA double-strand breaks by homologous recombination relative to normal cells,
or, the cancer comprises one or more cancer cells that lack BRCA1 or BRCA2, or have a BRCA1 or BRCA2 mutation.

10. The use as said in claim 9, wherein
the cancer includes but is not limited to malignant tumors, such as anyone of ovarian cancer, breast cancer, fallopian tube cancer, endometrial cancer, peritoneal cancer, gastric cancer, colon cancer, bladder cancer, pancreatic cancer, biliary cancer, osteosarcoma, cervical cancer, head and neck tumors, germ cell and embryonic cancer, esophageal cancer, malignant glioma, Ewing sarcoma, pancreatic cancer, melanoma, bile duct cancer, prostate cancer, small cell lung cancer, non-small cell lung cancer, lymphoma and blood cancer.
